# EUROPEAN PATENT SPECIFICATION

(11) **EP 1 305 026 B1**
(45) Date of publication and mention of the grant of the patent: **13.05.2009**
(21) Application number: 01934202.1
(22) Date of filing: 05.06.2001
(51) Int. Cl.: A61K 31/515

(54) **BARBITURIC ACID ANALOGS AS THERAPEUTIC AGENTS**
ANALOGE DER BARBITURSÄURE ALS THERAPEUTISCHE WIRKSTOFFE
ANALOGUES DE L'ACIDE BARBITURIQUE UTILISES COMME AGENTS THERAPEUTIQUES

(30) Priority: 05.06.2000 GB 0013655
(43) Date of publication of application: 02.05.2003
(73) Proprietor: Novuspharma S.p.A., 20091 Bresso (IT)
(72) Inventor: DE BELIN, Jackie Y., Oxford Oxfordshire OX14 4RY (GB); ROMERO-MARTIN, Maria-Rosario, Oxford Oxfordshire OX14 4RY (GB); FINN, Paul, W., Oxford Oxfordshire OX14 4RY (GB); SAYERS, Lee, G., Oxford Oxfordshire OX14 4RY (GB); LAW, Norman, M., Oxford Oxfordshire OX14 4RY (GB); BILLINGTON, David, Pharmaceutical Sc. Res. Inst., Birmingham, West Midlnds B4 7ET (GB); RYLEY, Stephen Pharmaceutical Sc. Res. Inst., Birmingham, West Midlands B4 7ET (GB); BHATTACHARYA, Shoumo, Oxford OxfordshireOX3 7BN (GB)
(74) Representative: Minoja, Fabrizio
(86) International application number: PCT/GB2001/002468
(87) International publication number: WO 2001/093841

(56) References cited:
- WO-A-00/13708
- WO-A-00/74725
- WO-A-91/16315
- WO-A-94/15605
- DATABASE WPI Section Ch, Derwent Publications Ltd., London, GB; Class B00, AN 1966-29541F XP002185020 & JP 42 022775 B (MIURA K)
- CHEMICAL ABSTRACTS, vol. 56, no. 5, 5 March 1962 (1962-03-05) Columbus, Ohio, US; UKITA ET AL.: "Antitumor activity of.." column 5344h; XP002185008
- CHEMICAL ABSTRACTS, vol. 122, no. 21, 22 May 1995 (1995-05-22) Columbus, Ohio, US; abstract no. 255535, AMES ET AL.: "Electrochemistry of thiobarbiturates..." XP002185009
- PATENT ABSTRACTS OF JAPAN vol. 011, no. 206 (C-433), 3 July 1987 (1987-07-03) & JP 62 029570 A (KANEGAFUCHI CHEM IND CO LTD), 7 February 1987 (1987-02-07)
- CHEMICAL ABSTRACTS, vol. 128, no. 18, 4 May 1998 (1998-05-04) Columbus, Ohio, US; abstract no. 217352, PAN ET AL.: "Synthesis and biological activity..." XP002185010
- CHEMICAL ABSTRACTS, vol. 110, no. 21, 22 May 1989 (1989-05-22) Columbus, Ohio, US; abstract no. 189241, BAUTISTA ET AL.: "Antimicrobial activity of ..." XP002185011
- CHEMICAL ABSTRACTS, vol. 60, no. 10, 11 May 1964 (1964-05-11) Columbus, Ohio, US; BOBAREVIC ET AL.: "Pharmacological investigation of ..." column 12554c; XP002185012
- CHEMICAL ABSTRACTS, vol. 81, no. 11, 16 September 1974 (1974-09-16) Columbus, Ohio, US; abstract no. 63577, SUVOROV ET AL.: "Indole derivatives..." XP002185013
- CHEMICAL ABSTRACTS, vol. 117, no. 13, 28 September 1992 (1992-09-28) Columbus, Ohio, US; abstract no. 131145, POPOV-PERGAL ET AL.: "Base catalyzed condensation..." XP002185014
- CHEMICAL ABSTRACTS, vol. 123, no. 21, 20 November 1995 (1995-11-20) Columbus, Ohio, US; abstract no. 285128, KUANAR ET AL.: "Applicability of Williams-Norrington..." XP002185015
- CHEMICAL ABSTRACTS, vol. 85, no. 11, 13 September 1976 (1976-09-13) Columbus, Ohio, US; abstract no. 78074, D'YACHKOV ET AL.: "Studies of pyrimidines..." XP002185016
- CHEMICAL ABSTRACTS, vol. 72, no. 21, 25 May 1970 (1970-05-25) Columbus, Ohio, US; abstract no. 111402, VVEDENSKII ET AL.: "Intramolecular substitution of barbituric acids..." XP002185017
- DATABASE CHEMCATS [Online] AN: 2000:615355; RN: 202063-67-0, 28 March 2000 (2000-03-28) XP002185018
- DATABASE BIOSIS [Online] BIOSCIENCES INFORMATION SERVICE, PHILADELPHIA, PA, US; FISCHER S ET AL: "Barbiturates decrease the expression of vascular endothelial growth factor in hypoxic cultures of porcine brain derived microvascular endothelial cells." Database accession no. PREV199800484071 XP002185019 & MOLECULAR BRAIN RESEARCH, vol. 60, no. 1, pages 89-97, ISSN: 0169-328X

## Description

### RELATED APPLICATION

This application claims priority to United Kingdom patent application GB 0013655.6 filed 05 June 2000, the contents of which are incorporated herein by reference in their entirety.

### TECHNICAL FIELD

This invention pertains generally to the field of antiproliferative compounds, and more specifically to certain active compounds which inhibit HIF-1 activity (e.g., the interaction between HIF-1α and p300), and thereby inhibit angiogenesis. The present invention also pertains to pharmaceutical compositions comprising such compounds, and the use of such compounds and compositions, both in vitro and in vivo, to inhibit the interaction between HIF-1α and p300, and to inhibit angiogenesis.

### BACKGROUND

Solid tumour growth is dependent upon the supply of nutrients and oxygen from the blood. Typically a tumour mass will not grow beyond 2-3 mm³ unless new blood vessels are formed within the tumour. Such "pre-vascular" tumours and dormant micrometastases maintain their small volume due to a balance of cell proliferation and cell death; they are generally asymptomatic and hence clinically undetected. The formation of new blood vessels (vasculature) within a tumour, by a process known as angiogenesis or neovascularisation, permits further growth, and it is typically vascularised solid tumours which are detected and which require treatment. Thus, angiogenesis is an essential component of tumorigenesis and the pathogenesis of cancer, and is a recognized target for cancer therapy.

The phenomenon of angiogenesis has many features, for instance intra- and intercellular signalling, tissue remodelling and endothelial cell proliferation. In addition, it has the significant feature that angiogenic endothelial cells have not suffered the unpredictable and undefined mutational changes which characterise tumour cells.

The fundamental stimulus for angiogenesis is believed to be localized tissue "hypoxia," in which tumour cells become starved of oxygen. This condition is typically observed within solid tumours, and the hypoxic environment is believed to arise largely as a result of the rapid aberrant proliferation of the cancer cell, and thus inability of the tumor to maintain an adequate and organised vasculature to supply oxygen to cells within the tumor. When the tumour cells become starved of oxygen, they respond by the expression and secretion of proteins important for stimulating angiogenesis. This response, known as hypoxia adaptation, leads to vascularisation, and facilitates further tumour growth. The most powerful and predominant angiogenic factors appear to be VEGF (Vascular Endothelial Growth Factor) and bFGF (basic Fibroblast Growth Factor).

Recent studies on the inhibition of angiogenesis, taking several approaches, have clearly demonstrated that efficient inhibition of this process can block tumour growth in animal models. Probably the most dramatic examples of induced tumour regression under experimental conditions using an anti-angiogenic strategy have recently been provided by the studies using the naturally occurring polypeptides angiostatin and endostatin. These studies, in addition to the apparent efficacy, have shown no toxicity and no acquired drug resistance. In addition, promising anti-angiogenic strategies are in clinical development using small molecules targeting several aspects of blood vessel growth, e.g., VEGF/VEGF receptor, integrin (αvβ3) : vitronectin interaction, or the inhibition of matrix metalloproteinases. The only clear undesirable side effects to an anti-angiogenic strategy that has been determined so far is a reversible loss of female fertility.

Therefore, inhibition of angiogenesis is an attractive aim in pharmaceutical discovery because it should be clinically efficacious and because the genetic homogeneity of the target tissue renders it unlikely to acquire drug resistance. Disruption of signal transduction pathways that mediate adaptation to hypoxia and angiogenesis may represent potentially effective anti-cancer strategies. It is important to realize that the target of an anti-angiogenesis therapy would primarily be the endothelial cell rather than the cancer cell. One advantage that the endothelial cell would offer as a cellular target is that it is not an immortalised cell line, and multi-drug resistance mechanisms operating in cancer cells would presumably be absent.

There are several control points influencing angiogenesis which may be considered as targets for intervention, and one of particular interest is the transcription factor Hypoxia-Inducible Factor 1 (HIF-1). HIF-1 has been shown to play an essential role in cellular responses to hypoxia. Upon hypoxic stimulation, HIF-1 is known to activate genes that contain Hypoxic Response Elements (HREs) in their promoters, and thus up-regulate a series of gene products that promote cell survival under conditions of low oxygen availability.

The list of HIF-responsive genes is constantly expanding, but known gene products include glycolytic enzymes such as lactate dehydrogenase, (LDH-A), enolase-1 (ENO-1), and aldolase A; glucose transporters GLUT 1, & 3; vascular endothelial growth factor (VEGF); inducible nitric oxide synthase (NOS-2); and erythropoietin (EPO). The switch of the cell to anaerobic glycolysis, and the up-regulation of angiogenesis by VEGF is geared at maximizing cell survival under conditions of low oxygen tension by reducing the requirement for oxygen, and increasing vasculature to maximise oxygen delivery to tissues. Induction of NOS-2, and the subsequent increase in NO would effectively promote a state of vasodilation in the hypoxic microenvironment thereby maximizing blood flow and oxygen delivery to cells. Increased EPO production by the tubular interstitial cells of the kidney is geared at promoting erythropoiesis, and increasing red blood cell number to further facilitate oxygen delivery to hypoxic tissues.

The HIF-1 transcription complex has recently been shown to comprise a heterodimer of two basic helix-loop-helix proteins, HIF-1α and HIF-1β (also known as ARNT, Aryl Hydrocarbon Receptor Nuclear Translocator). See, for example, Wood et al., 1996. Oxygen tension regulates the expression levels of both factors.

HIF-1α is a member of the basic-helix-loop-helix PAS domain protein family and is an approximately 120 kDa protein containing 2x transactivation domains (TAD) in its carboxy-terminal half and DNA binding activity located in the N-terminal half of the molecule. HIF-1α is constitutively degraded by the ubiquitin-proteosome pathway under conditions of normoxia, a process that is facilitated by binding of the von Hippel-Lindau (VHL) tumor suppressor protein to HIF-1α. Under conditions of hypoxia, degradation of HIF-1α is blocked and active HIF-1α accumulates. The subsequent dimerization of HIF-1α with ARNT leads to the formation of active HIF transcription complexes in the nucleus, which can bind to and activate HREs on HIF-responsive genes.

Recent evidence suggests that nuclear translocation is a function intrinsic to HIF-1α and does not require ARNT. Indeed, ARNT has recently been postulated to function to lock HIF-1α in the nucleus and protect it from proteolytic degradation, enabling the active complex to bind DNA and activate transcription. Studies in a mouse hepatoma cell line found to be deficient in ARNT showed that HIF-1 activity was not induced by hypoxia. Furthermore, in animal tumour model studies using this cell line, reduced VEGF expression was observed associated with decreased tumour vascularity and growth rate. In a separate approach, a targeted gene disruption of ARNT in the mouse was found to cause embryonic lethality (day 10.5) with angiogenic abnormalities similar to those observed for VEGF deficiency. Associated studies confirmed that these ARNT^{-/-} embryonic stem cells were unable to induce genes such as VEGF in response to hypoxia.

It is known that HIF-1 activity is sustained by the p300/CBP co-activator family of proteins and that recruitment of the transcriptional adapter protein p300 to the HIF-1 complex is an essential step to activate HIF-responsive genes. The protein p300 physically interacts with the activation domain of HIF-1α to facilitate the transcription of target genes, and this interaction has been shown to be mediated by the N-terminal CH1 domain of p300. It is believed that histone acetyl transferase (HAT) activity of p300 is required to allow the HIF-1 complex to access chromatin and bind to sites on DNA. Since there are known to be multiple HIF-1 binding sites on a single promoter, p300 has also been postulated to physically link several HIF-1 complexes to maximally activate transcription. A recent study demonstrating that binding of adenoviral protein E1A to p300 completely abolished HIF-dependent transcriptional activation demonstrates an essential role for p300 in HIF activation. Indeed, a mutant E1A molecule selectively deficient for p300 binding failed to block HIF-dependent transcriptional activation, providing convincing evidence that pharmaceutical intervention at the level of HIF-1α/p300 would completely inactivate the complex.

Several lines of evidence support the importance of HIF-1 as a viable therapeutic target in angiogenesis. HIF-1α^{-/-} mice show an embryonic lethal phenotype, which is characterised by a lack of cephalic vascularisation. Teratocarcinomas generated from HIF-1α^{-/-} mice were 75% smaller than wildtype tumours, the reduced size resulting from increased levels of apoptosis. Furthermore, inactivation of ARNT in a mouse hepatoma cell line resulted in retarded angiogenesis and tumour growth. Other studies have documented the levels of HIF-1α with a highly metastatic and aggressive tumour phenotype, for example in the human prostrate cell line PC3 which has high levels of HIF-1α and is very metastatic. More recently, a transgenic mouse approach has been taken to demonstrate the importance of the HIF-1α/p300 interaction for tumourigenesis.

Additional discussion of hypoxia, HIF-1, and related topics is provided in following recent review articles: Brown et al., 2000; Semenza et al., 1999a; Semenza et al., 1999b; Richard et al., 1999; Taylor et al., 1999; and Wenger et al., 1999.

Several components of the HIF-1 complex offer potential sites where a small molecule drug could cause disruption and inactivate the transcription of HIF-responsive genes. Essential interactions required to activate transcription include the HIF-1α/ARNT interaction, the HIF-1α/p300 interaction, and the HIF-1/DNA interaction. One target of particular interest is the HIF-1α/p300 interaction. This interaction offers a more attractive target than HIF-1α/ARNT since disruption of dimerization would presumably liberate ARNT, which has other functions within the cell.

Methods of identifying compounds which modulate a transcriptional response to hypoxia in a cell are described in Livingston et al., 2000. Similar methods are also described in Arany et al., 1996.

One aim of the present invention is the provision of small drug-like molecules which interfere with the pro-angiogenic response of tumour cells to hypoxic conditions. There is a pressing need for such anticancer compounds, since present drugs are of low efficacy, have many deleterious side-effects, and often give rise to drug-resistance in the tumour.

Such molecules desirably have one or more of the following properties and/or effects:
(a) easily gain access to and act upon endothelial cells of the tumor vasculature;
(b) down-regulate HIF-1 activity;
(c) inhibit the formation of the HIF-1 complex;
(d) inhibit the interactions of the HIF-1 complex;
(e) inhibit the HIF-1α/p300 interaction;
(f) inhibit the transcription of HIF-responsive genes, for example, the VEGF gene;
(g) inhibit the hypoxic response of tumours;
(h) inhibit angiogenesis;
(i) promote tumour cell apoptosis;
(j) inhibit tumour growth; and,
(k) complement the activity of traditional chemotherapeutic agents.

A number of barbituric acid derivatives are known, and, as discussed below, some have been reported to have biological activity.

Pan et al., 1997, describe certain 5-(phenylmethylene) barbituric acid analogs which apparently inhibit tyrosine protein kinase (TPK) of HL-60 leukemia cells and normal rat spleen cells.

Fellahi et al., 1995, describe certain 2-substituted-5-(1,2-diarylethyl)-4,6-dichloropyrimidine derivatives which apparently are active against a wide range of bacterial flora of the axilla and foot, and in particular, against Corynebacterium xerosis and Arcanobacterium haemolyticum of the human axilla.

Naguib et al., 1993, describe certain 5-benzylbarbituric acid derivatives which apparently are potent and specific inhibitors of uridine phosphorylase.

Miyazaki et al., 1987, describe certain barbituric acid derivatives which apparently showed excellent maintenance effect on the survival and function of adult rat hepatocytes in primary culture.

Rehse et al., 1982, describe certain barbituric acid derivatives which were synthesized and tested for anticoagulant activity, but found to be inactive.

Vida et al., 1974, describe certain 5-substituted-5-proprionoxybarbituric acid derivatives which apparently had analgesic activity in mice.

Weinryb et al., 1971, describe certain barbiturates, including 5,5-dibromobarbiturate and 5-bromo-5-phenylbarbiturate, which apparently were potent inhibitors of basal adenylate cylcase activity in particulate fractions from guinea pig heart and lung.

### SUMMARY OF THE INVENTION

One aspect of the invention pertains to the use of active compounds, as described herein, which inhibit HIF-1 activity, and the interaction between HIF-1α and p300 for the manufacture of a medicament for inhibiting angiogenesis.

### DETAILED DESCRIPTION OF THE INVENTION

### Compounds

The well known compound, barbituric acid, has the following formula:

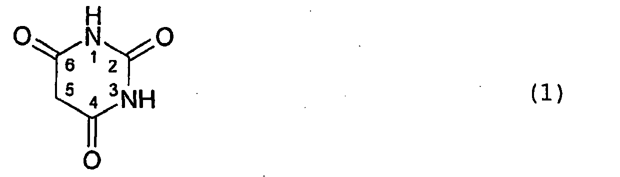

The present invention pertains to the use of certain barbituric acid analogs, specifically, compounds of the formula: 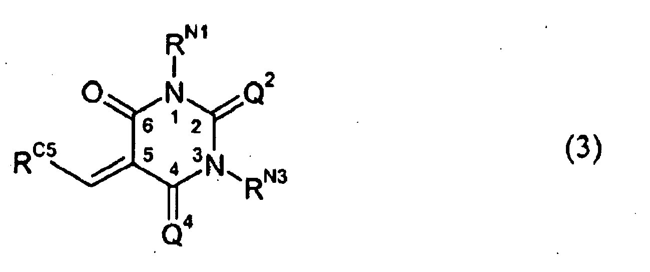 wherein:
Q² is =O, =S, or =NR^{N2};
Q⁴ is =O, =S, or =NR^{N4};
R^{C5} is a carbon substituent, and is independently **optionally substituted** aliphatic or alicyclic C₁₋₇alkyl, **which may be saturated, partially saturated or fully unsaturated;** optionally substituted C₃₋₂₀heterocyclyl, or optionally substituted C₅₋₂₀aryl and may be cis- or trans-;
each one of R^{N1}, R^{N2}, R^{N3}, and R^{N4} is a nitrogen substituent, and is independently hydrogen, optionally substituted C₁₋₇alkyl, optionally substituted C₃₋₂₀heterocyclyl, or optionally substituted C₅₋₂₀aryl;
in which the substituents are selected from hydrogen, halo, hydroxy, C₁₋₇alkoxy, C₅₋₂₀aryloxy, oxo, formyl, acyl, carboxy, carboxylate, acyloxy, amido, acylamido, amino, cyano, nitro, sulfhydryl, thioether, sulfonamino, sulfinamino, sulfamyl, sulfonamido, C₁₋₇alkyl, C₁₋₇haloalkyl, C₁₋₇hydroxyalkyl, C₁₋₇carboxyalkyl, C₁₋₇aminoalkyl, C₅₋₂₀aryl-C₁₋₇alkyl, optionally substituted C₃₋₂₀heterocyclyl, optionally substitutedC₅₋₂₀aryl, C₅₋₂₀heteroaryl, C₁₋₇alkyl-C₅₋₂₀aryl and C₅₋₂₀haloaryl) or a pharmaceutically acceptable salt or solvate thereof
for the manufacture of a medicament **for inhibiting angiogenesis.**

### Q² and Q⁴

In one embodiment, Q² is =O or =S; and Q⁴ is =O or =S.

In one embodiment, Q² is =O and Q⁴ is =O; or Q² is =S and Q⁴ is =O.

In one embodiment, Q² is =O and Q⁴ is =O.

In one embodiment, Q² is =S and Q⁴ is =O.

### Nitrogen Subsituents, R^{N}

Each one of R^{N1}, R^{N2}, R^{N3}, and R^{N4} is a nitrogen substituent, and is independently hydrogen, optionally substituted C₁₋₇alkyl (including, e.g., C₁₋₇haloalkyl, C₁₋₇hydroxyalkyl, C₁₋₇aminoalkyl, C₁₋₇carboxyalkyl, C₅₋₂₀aryl-C₁₋₇alkyl), optionally substituted C₃-₂₀heterocyclyl, or optionally substituted C₅₋₂₀aryl (including, e.g., C₅₋₂₀haloaryl, C₁₋₇alkyl-C₅₋₂₀aryl).

In one embodiment, each one of R^{N1}, R^{N2}, R^{N3}, and R^{N4} is independently hydrogen, optionally substituted C₁₋₇alkyl (including, e.g., C₁₋₇hydroxyalkyl, C₁₋₇haloalkyl, C₁₋₇aminoalkyl), or optionally substituted C₅₋₂₀aryl (including, e.g., C₅₋₂₀haloaryl, C₁₋₇alkyl-C₅₋₂₀aryl).

In one embodiment, each one of R^{N1}, R^{N2}, R^{N3}, and R^{N4} is independently hydrogen, saturated aliphatic C₁₋₇alkyl, saturated aliphatic C₁₋₇haloalkyl, saturated aliphatic C₁₋₇hydroxyalkyl, saturated aliphatic C₁₋₇aminoalkyl, saturated aliphatic C₁₋₇carboxyalkyl, C₅₋₂₀aryl-C₁₋₇alkyl, C₅₋₂₀carboaryl, or C₅₋₂₀haloaryl.

In one embodiment, each one of R^{N1}, R^{N2}, R^{N3}, and R^{N4} is independently -H, -Me, -Et, -CH₂COOH, -Ph, -C₆H₄F, -C₆H₄Cl, -C₆H₄Br, -C₆H₄-OCH₃, or -C₆H₄-CH₃.

In one embodiment, each one of R^{N1}, R^{N2}, R^{N3}, and R^{N4} is independently -H, -Me, -Et, -CH₂COOH, -Ph, or -C₆H₄Cl.

In one embodiment, R^{N1} is -H or other than -H; and each one of R^{N2}, R^{N3}, and R^{N4} is -H.

In one embodiment, R^{N3} is -H or other than -H; and each one of R^{N1}, R^{N2}, and R^{N4} is -H.

In one embodiment, each one of R^{N1}, R^{N2}, R^{N3}, and R^{N4} is -H_{.}

### 5-(Substituted Methylene) Compounds

In one embodiment:
Q² is =O and
Q⁴ is =O;
   and the compounds have the following formula:

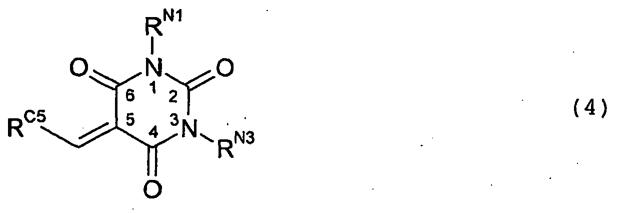

In one embodiment:
Q² is =S; and
Q⁴ is =O;
   and the compounds have the following formula:

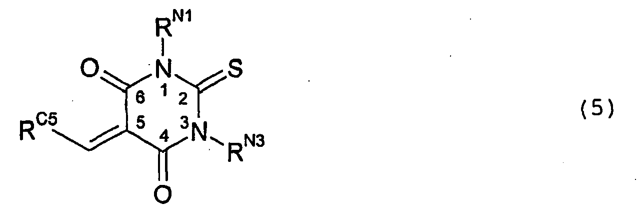

In the above formulae, R^{C5} is, as mentioned above, optionally substituted C₁₋₇alkyl (including, e.g., C₁₋₇haloalkyl, C₁₋₇hydroxyalkyl, C₁₋₇aminoalkyl, C₁₋₇carboxyalkyl, C₅₋₂₀aryl-C₁₋₇alkyl), optionally substituted C₃₋₂₀heterocyclyl, or optionally substituted C₅₋₂₀aryl (including, e.g., C₅₋₂₀haloaryl, C₁₋₇alkyl-C₅₋₂₀aryl).

In one embodiment, R^{C5} is C₁₋₇alkyl, and is optionally substituted. In one embodiment, R^{C5} is C₃₋₆cycloalkyl, and is optionally substituted. In one embodiment, R^{C5} is partially unsaturated C₃₋₆cycloalkyl, for example, cyclohexenyl.

In one embodiment, R^{C5} is C₅₋₂₀aryl-C₁₋₇alkyl, and is optionally substituted, for example, phenyl-ethenyl (styryl), furanyl-ethenyl, and thiophenyl-ethenyl.

In one embodiment, R^{C5} is C₃₋₂₀heterocyclyl, and is optionally substituted. In one embodiment, R^{C5} is C₅₋₇heterocyclyl, and is optionally substituted.

In one embodiment, R^{C5} is C₅₋₂₀aryl, and is optionally substituted.

In one embodiment, R^{C5} is C₅₋₂₀carboaryl, and is optionally substituted. In one embodiment, R^{C5} is phenyl, naphthyl, anthracenyl, or phenanthryl, and is optionally substituted. In one embodiment, R^{C5} is phenyl, and is optionally substituted.

In one embodiment, R^{C5} is C₅₋₂₀heteroaryl, and is optionally substituted. In one embodiment, R^{C5} is furanyl, thiophenyl, pyrrolyl, indolyl, or benzopyronyl (e.g., chromonyl), and is optionally substituted. In one embodiment, R^{C5} is furanyl, thiophenyl, or pyrrolyl, and is optionally substituted, for example, nitrothiophenyl.

In one embodiment, R^{C5} is cyclohexenyl, phenyl, furanyl, thiophenyl, pyrrolyl, indolyl, or benzopyronyl (e.g., chromonyl), and is optionally substituted.

In one embodiment, R^{C5} is phenyl, and is optionally substituted.

Examples of substituents include, but are not limited to, hydrogen, halo, hydroxy, ether (including, e.g., C₁₋₇alkoxy, C₅₋₂₀aryloxy oxo, formyl, acyl, carboxy, carboxylate, acyloxy, amido, acylamido, amino, cyano, nitro, sulfhydryl, thioether, sulfonamino, sulfinamino, sulfamyl, sulfonamido, C₁₋₇alkyl (including, e.g., C₁₋₇haloalkyl, C₁₋₇hydroxyalkyl, C₁₋₇carboxyalkyl, C₁₋₇aminoalkyl, C₅₋₂₀aryl-C₁₋₇alkyl), optionally substiuted C₃₋₂₀heterocyclyl, optionally substituted C₅₋₂₀aryl (including, e.g., C₅₋₂₀heteroaryl, C₁₋₇alkyl-C₅₋₂₀aryl and C₅₋₂₀haloaryl)

### 5-(Phenylmethylene) Compounds

In one embodiment the compounds have the following formula: 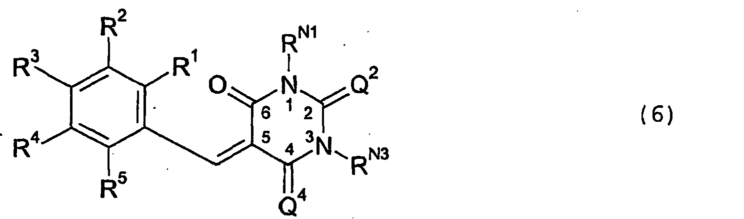 wherein Q₂, Q₄, R^{N1} and R^{N3} are as defined herein before

In one embodiment:
Q² is =O;
Q⁴ is =O;
   and the compounds have the following formula:

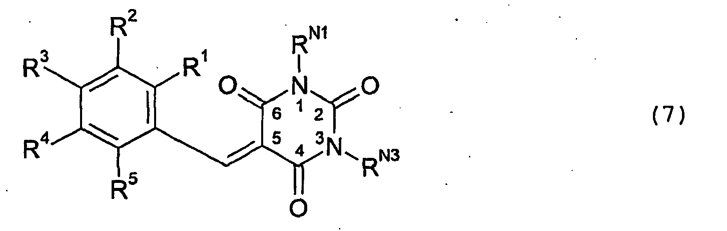

In one embodiment:
Q² is =S;
Q⁴ is =O;
   and the compounds have the following formula:

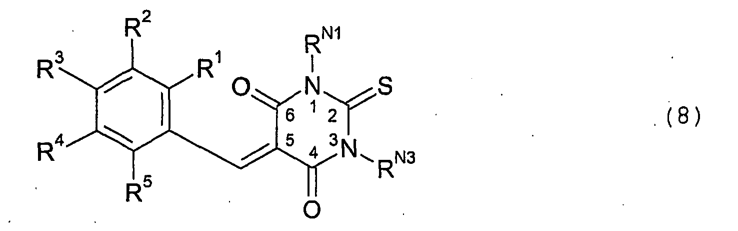

In the above formulae, each one of R¹ through R⁵ is a phenyl substituent, and is independently hydrogen, halo, hydroxy, ether (e.g., C₁₋₇alkoxy, C₅₋₂₀aryloxy), formyl, acyl, carboxy, carboxylate, amido, acylamido, amino, nitro, optionally substituted C₁₋₇alkyl (including, e.g., C₁₋₇haloalkyl), optionally substituted C₃₋₂₀heterocyclyl, or optionally substituted C₅₋₂₀aryl.

Also, two of R¹ through R⁵, preferably adjacent groups, may together form a bidentate structure which, together with the two carbon atoms to which it is attached, forms a cyclic structure with five or six ring atoms, which ring atoms are carbon, nitrogen, or oxygen, and wherein the bonds between said ring atoms of the cyclic structure are single or double bonds, as permitted by the valencies of the ring atoms. Examples of such bidentate structures include, but are not limited to, -(CH₂)₃-, - (CH₂)₄-, -O-CH₂-O-, and -O-CH₂CH₂-O-, and substituted and/or unsaturated forms thereof.

In one embodiment, each one of R¹ through R⁵ is hydrogen, halo, hydroxy, C₁₋₇alkoxy, optionally substituted C₅₋₂₀aryloxy, optionally substituted C₅₋₂₀aryl-C₁₋₇alkoxy, acyl, amino (e.g., with from 0 to 2 optionally substituted C₁₋₇allkyl substituents), or optionally substituted C₁₋₇alkyl (including, e.g., C₁₋₇haloalkyl).

In one embodiment, each one of R¹ through R⁵ is independently selected from:
-H;
-F, -Cl, -Br, and -I;
-OH;
-OCH₃, -OCH₂CH₃, -OC(CH₃)₃, and -OCH₂Ph;
-C(=O)H;
-C(=O)CH₃, -C(=O)CH₂CH₃, -C(=O)C(CH₃)₃, and -C(=O)Ph;
-COOH;
-COOCH₃, -COOCH₂CH₃, and -COOC (CH₃) ₃;
-C(=O)NH₂, -C(=O)NHCH₃, -C(=O)N(CH₃)₂, and -C (=O) NHCH₂CH₃;
-NHC(=O)CH₃, -NHC(=O)CH₂CH₃, -NHC(=O)Ph, succinimidyl, and maleimidyl;
-NH₂, -NHCH₃, -NHCH(CH₃) ₂, -N(CH₃)₂, and -N(CH₂CH₃)₂;
-NO₂ ;
-CH₃, -CH₂CH₃, -CH₂CH₂CH₃, and -CH(CH₃)₂;
-CF₃, -CHF₂, -CH₂F, -CCl₃, -CBr₃, -CH₂CH₂F, -CH₂CHF₂, and -CH₂CF₃;
-OCF₃, -OCHF₂, -OCH₂F, -OCCl₃, -OCBr₃, -OCH₂CH₂F, -OCH₂CHF₂, and -OCH₂CF₃;
-CH₂OH, -CH₂CH₂OH, and -CH(OH)CH₂OH;
-CH₂NH₂,-CH₂CH₂NH₂, and -CH₂CH₂N(CH₃)₂; and,
optionally substituted phenyl.

In one embodiment, each one of R¹ through R⁵ is independently selected from: -H, -F, -Cl, -Br, -I, -NMe₂, -NEt₂, -OH, -OCH₃, -OCH₂CH₃, -OPh, -OCH₂Ph, -C (=O) CH₃, -CONH₂, -CONHCH₃, -NO₂ -CH₃, -CH₂CH₃, -CF₃, -OCF₃, -CH₂OH -Ph, and -CH₂Ph.

In one embodiment, each one of R¹ through R⁵ is independently selected from: -H, -F, -Cl, -Br, -I, -NMe₂, -NEt₂, -OH, -OMe, -OEt, -CONHMe, -NO₂ and -CF₃.

In one embodiment, each one of R¹ through R⁵ is independently selected from: -H, -NMe₂, -OH, -OMe, -OEt, and -NO₂.

In one embodiment, each one of R¹ through R⁵ is independently selected from: -H, -F, -Cl, -Br, and -I.

### Examples of Specific Embodiments

Some individual embodiments of the present invention include the following compounds:

| | | |
|---|---|---|
| (1) | 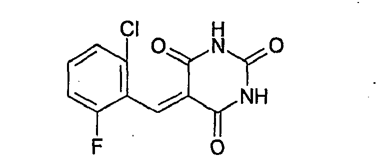 | PX069119 |
| (2) | 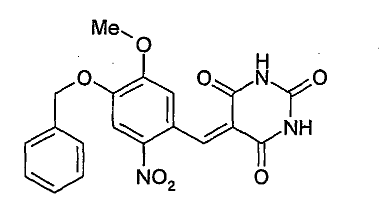 | PX069153 |
| (3) | 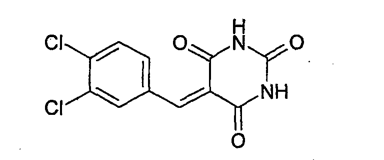 | PX072002 |
| (4) | 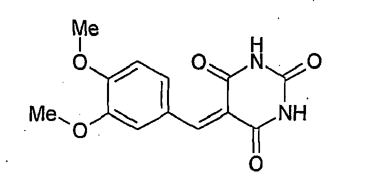 | PX072004 |
| (5) | 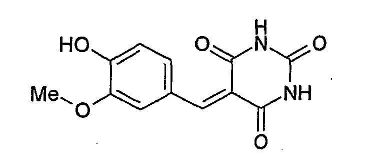 | PX072008 |
| (6) | 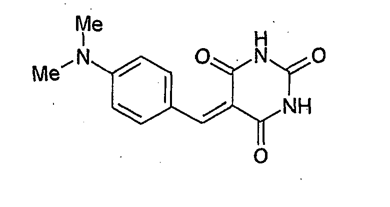 | PX072009 |
| (7) | 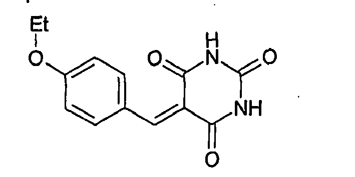 | PX072012 |
| (8) | 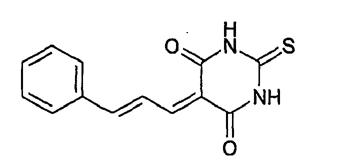 | PX072015 |
| (9) | 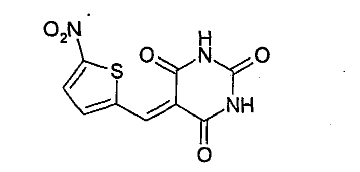 | PX074037 |
| (10) | 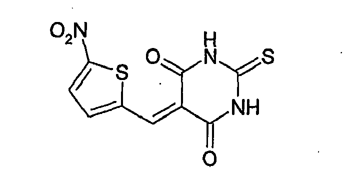 | PX074038 |
| (11) | 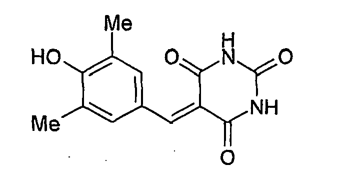 | PX074100 |
| (12) | 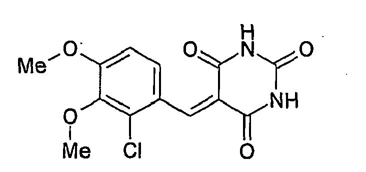 | PX074728 |
| (13) | 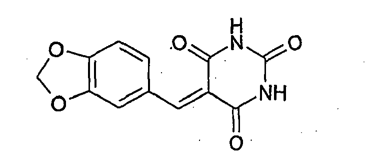 | PX075240 |
| (14) | 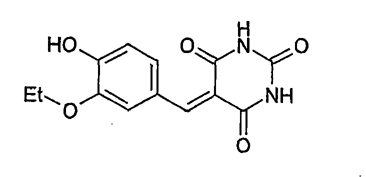 | PX075244 |
| (15) | 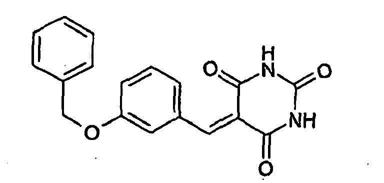 | PX075245 |
| (16) | 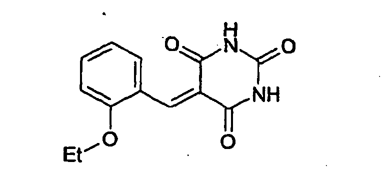 | PX075248 |
| (17) | 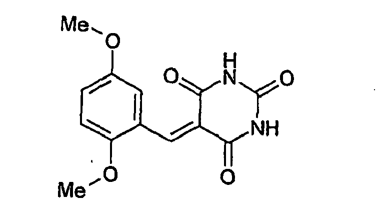 | PX075257 |
| (18) | 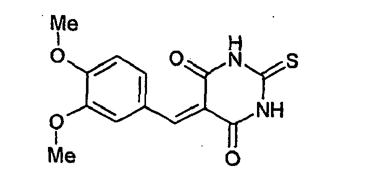 | PX075262 |
| (19) | 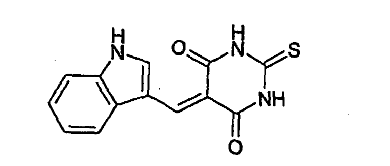 | PX075276 |
| (20) | 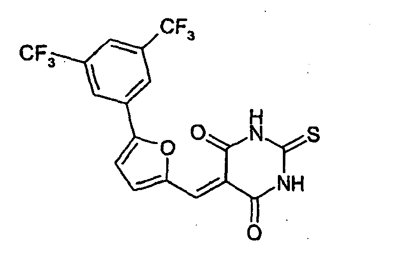 | PX075367 |
| (28) | 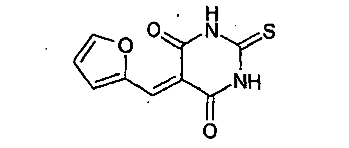 | PX083634 |
| (29) | 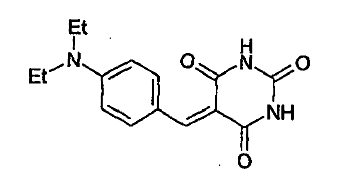 | PX083675 |
| (30) | 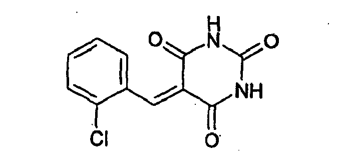 | PX083677 |
| (33) | 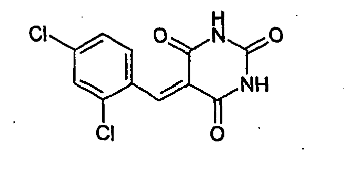 | PX089367 |
| (34) | 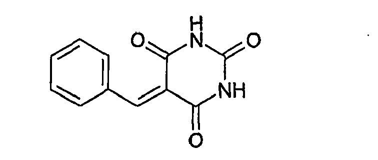 | PX089368 |
| (35) | 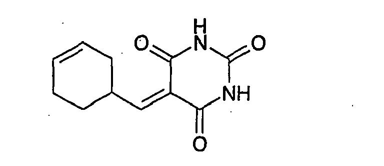 | PX089369 |
| (36) | 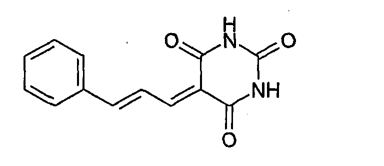 | PX089370 |
| (37) | 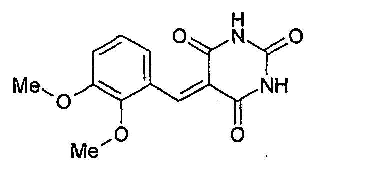 | PX089371 |
| (38) | 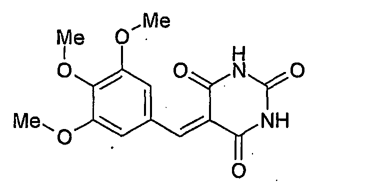 | PX089372 |
| (39) | 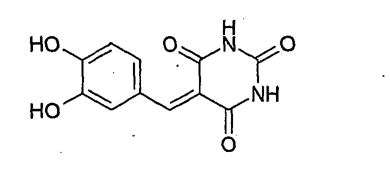 | PX089374 |
| (40) | 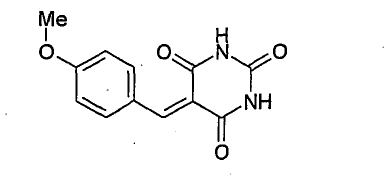 | PX089375 |
| (41) | 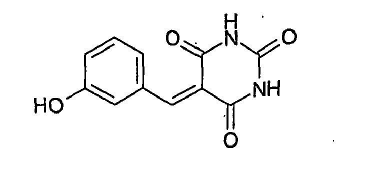 | PX089376 |
| (42) | 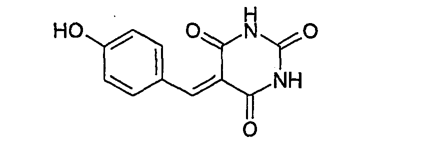 | PX089377 |
| (43) | 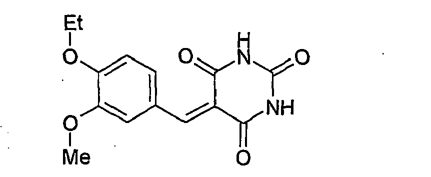 | PX089378 |
| (44) | 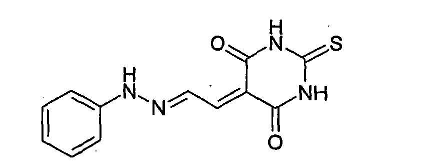 | PX089619 |
| (45) | 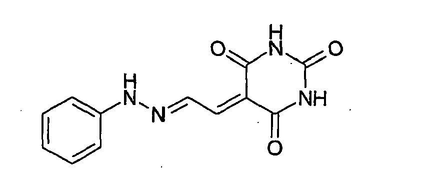 | PX089620 |
| (46) | 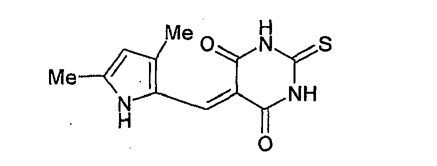 | PX089624 |
| (47) | 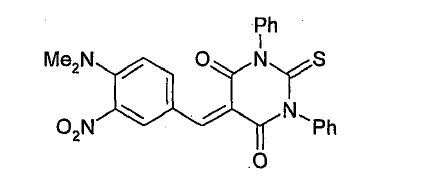 | PX089626 |
| (48) | 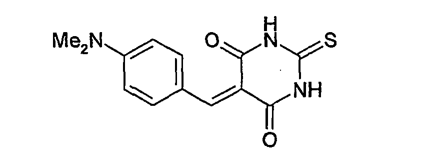 | PX089631 |
| (49) | 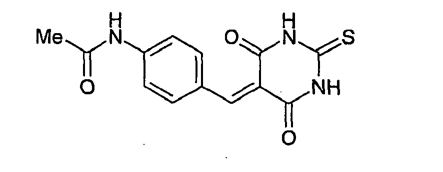 | PX089632 |
| (50) | 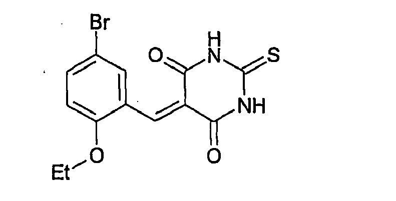 | PX089633 |
| (51) | 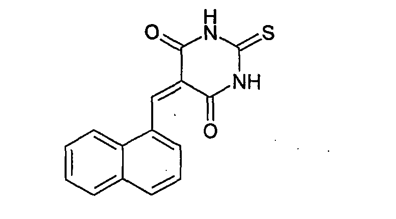 | PX089635 |
| (52) | 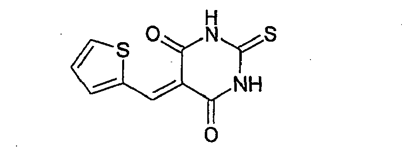 | PX089638 |
| (53) | 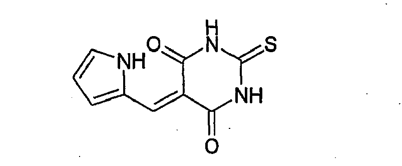 | PX089639 |
| (54) | 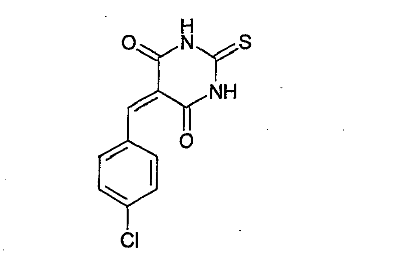 | PX089640 |
| (55) | 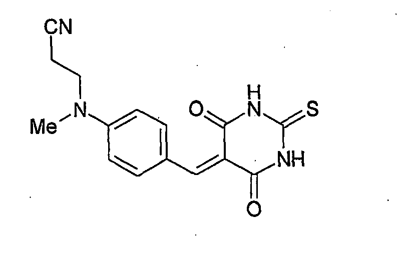 | PX089643 |
| (56) | 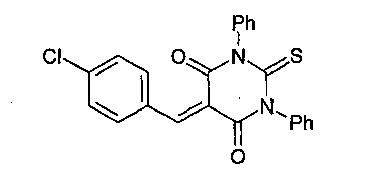 | PX089645 |
| (57) | 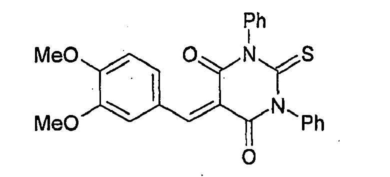 | PX089646 |
| (58) | 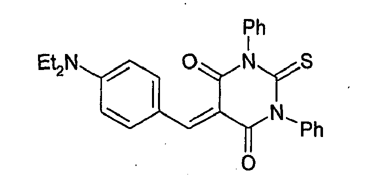 | PX089647 |
| (59) | 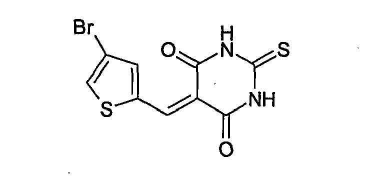 | PX089648 |
| (60) | 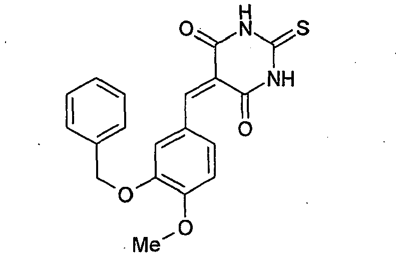 | PX105990 |
| (61) | 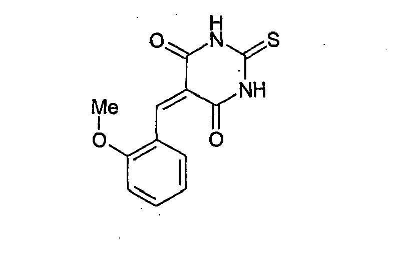 | PX105993 |
| (62) | 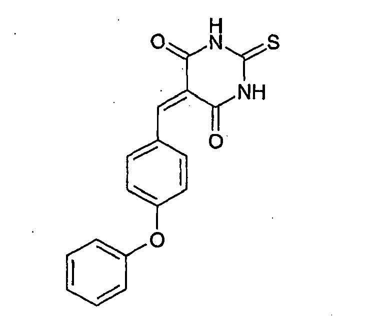 | PX106021 |
| (63) | 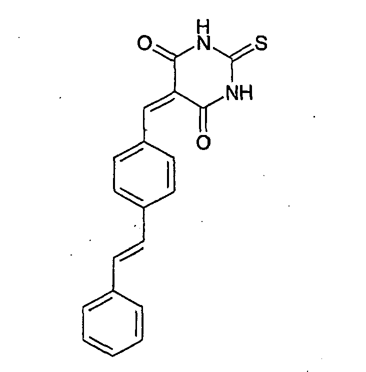 | PX106027 |
| (64) | 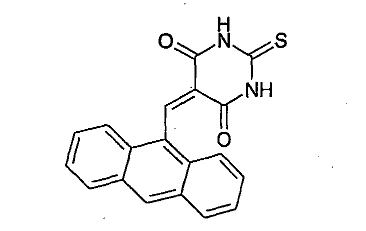 | PX106031 |
| (65) | 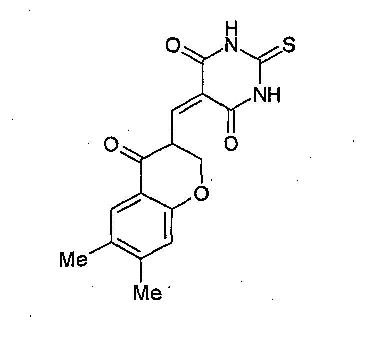 | PX106036 |
| (66) | 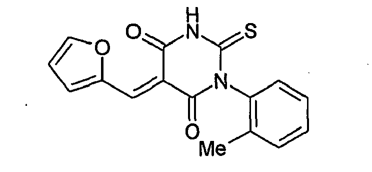 | PX106130 |
| (67) | 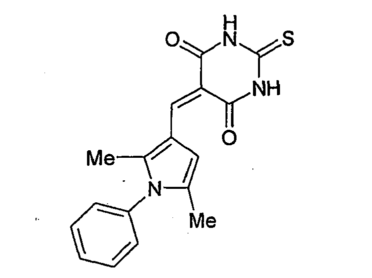 | PX106151 |
| (68) | 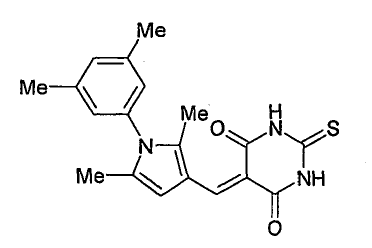 | PX106155 |
| (69) | 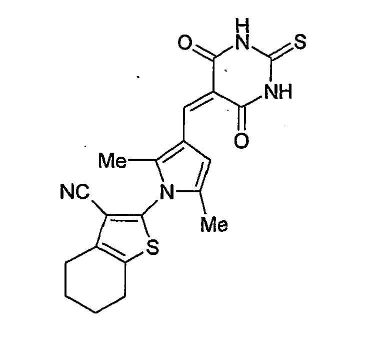 | PX106174 |
| (70) | 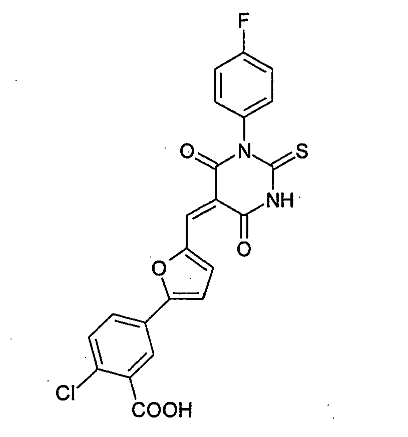 | PX106244 |
| (71) | 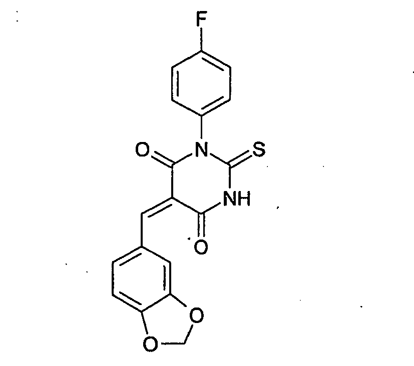 | PX106255 |
| (72) | 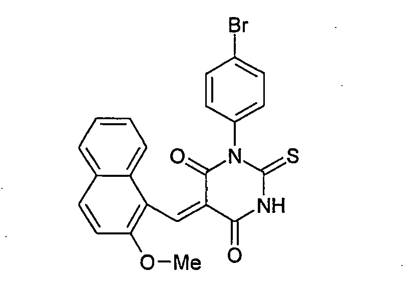 | PX106265 |
| (73) | 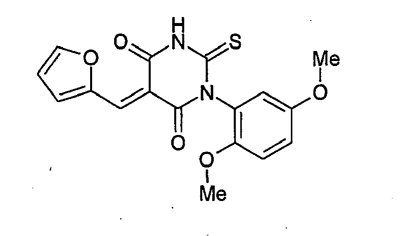 | PX106274 |
| (74) | 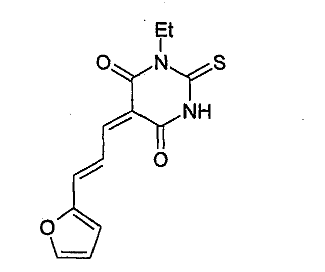 | PX106281 |
| (75) | 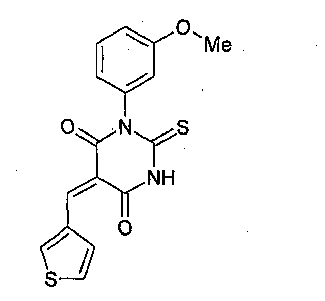 | PX106287 |
| (76) | 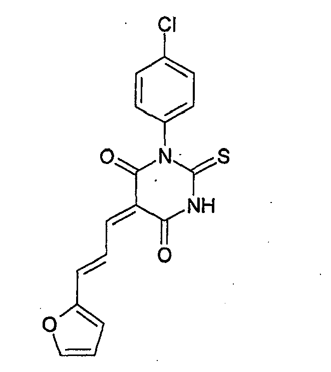 | PX106291 |
| (77) | 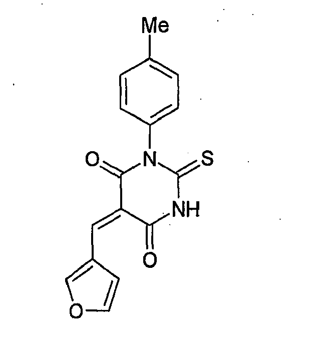 | PX106297 |
| (78) | 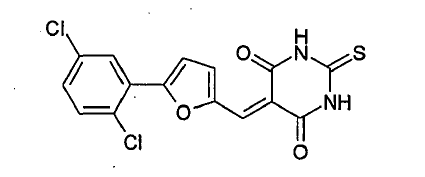 | PX106326 |
| (79) | 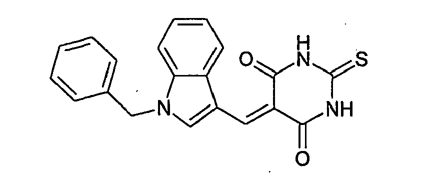 | PX106341 |
| (80) | 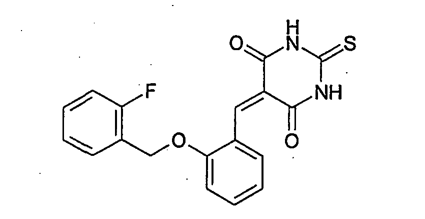 | PX106343 |

### Substituents

The term "substituent" is used herein in the conventional sense and refers to a chemical moiety which is covalently attached to, appended to, or if appropriate, fused to, a parent group. A wide variety of substituents are well known, and methods for their formation and introduction into a variety of parent groups are also well known. Examples of substituents include, but are not limited to, the following:
Hydrogen: -H. Note that if the substituent at a particular position is hydrogen, it may be convenient to refer to the compound as being "unsubstituted" at this position.
Halo: -F, -Cl, -Br, and -I.
Hydroxy: -OH.
Ether: -OR, wherein R is an ether substituent, for example, a C₁₋₇alkyl group (resulting in a C₁₋₇alkoxy group, discussed below), a C₃₋₂₀heterocyclyl group (resulting in a C₃-₂₀hetercyclyloxy group), or a C₅₋₂₀aryl group (resulting in a C₅₋₂₀aryloxy group), preferably a C₁₋₇alkyl group.
C₁₋₇alkoxy: -OR, wherein R is a C₁₋₇alkyl group. Examples of C₁₋₇alkoxy groups include, but are not limited to, -OCH₃ (methoxy), -OCH₂CH₃ (ethoxy) and -OC (CH₃) ₃ (tert-butoxy).
Oxo (keto): =O.
Imino: =NR, wherein R is an imino substituent, for example, for example, hydrogen, a C₁₋₇alkyl group, a C₃₋₂₀heterocyclyl group, or a C₅₋₂₀aryl group, preferably H or a C₁₋₇alkyl group.
Formyl (carbaldehyde): -C(=O)H.
Acyl (keto): -C(=O)R, wherein R is an acyl substituent, for example, a C₁₋₇alkyl group (also referred to as C₁₋₇alkylacyl) , a C₃₋₂₀heterocyclyl group (also referred to as C₃₋₂₀heterocyclylacyl), or a C₅₋₂₀aryl group (also referred to as C₅₋₂₀arylacyl), preferably a C₁₋₇alkyl group. Examples of acyl groups include, but are not limited to, -C(=O)CH₃ (acetyl), -C(=O)CH₂CH₃ (propionyl), -C(=O)C(CH₃)₃ (butyryl), and -C (=O) Ph (benzoyl).
Carboxy (carboxylic acid): -C(=O)OH.
Carboxylate (carboxylic acid ester): -C(=O)OR, wherein R is an ester substituent, for example, a C₁₋₇alkyl group, a C₃-₂₀heterocyclyl group, or a C₅₋₂₀aryl group, preferably a C₁₋₇alkyl group. Examples of carboxylate groups include, but are not limited to, -COOCH₃, -COOCH₂CH₃, and -COOC(CH₃)₃.
Acyloxy (reverse ester): -OC(=O)R, wherein R is an acyloxy substituent, for example, a C₁₋₇alkyl group, a C₃₋₂₀heterocyclyl group, or a C₅₋₂₀aryl group, preferably a C₁₋₇alkyl group. Examples of acyloxy groups include, but are not limited to, -OC(=O)CH₃ (acetoxy), -OC(=O)CH₂CH₃, and -OC(=O)C(CH₃)₃.
Amido (carbamoyl, carbamyl, aminocarbonyl): -C(=O)NR¹R², wherein R¹ and R² are independently amino substituents, as defined for amino groups. Examples of amido groups include, but are not limited to, -C (=O) NH₂, -C(=O)NHCH₃, -C (=O) NH (CH₃) ₂, and -C(=O)NHCH₂CH₃.
Acylamido (acylamino) : -NR¹C(=O)R², wherein R¹ is an amide substituent, for example, a C₁₋₇alkyl group, a C₃-₂₀heterocyclyl group, or a C₅₋₂₀aryl group, preferably a C₁₋₇alkyl group, and R² is an acyl substituent, for example, a C₁₋₇alkyl group, a C₃₋₂₀heterocyclyl group, or a C₅₋₂₀aryl group, preferably a C₁₋₇alkyl group. Examples of acylamide groups include, but are not limited to, -NHC(=O)CH₃ , -NHC(=O)CH₂CH₃, and -NHC (=O) Ph. R¹ and R² may together form a cyclic structure, as in, for example, succinimidyl and maleimidyl: 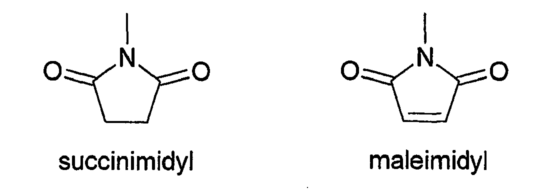
Amino: -NR¹R², wherein R¹ and R² are independently amino substituents, for example, hydrogen, a C₁₋₇alkyl group, a C₃₋₂₀heterocyclyl group, or a C₅₋₂₀aryl group, preferably H or a C₁₋₇alkyl group. R¹ and R², taken together with the nitrogen atom may form a heterocyclic ring having from 4 to 8 ring atoms (for example, aziridinyl, azetidinyl, pyridyl). Examples of amino groups include, but are not limited to, -NH₂, -NHCH₃, -NHCH(CH₃)₂, -N(CH₃)₂, and -N(CH₂CH₃)₂.
Cyano (nitrile, carbonitrile): -CN.
Nitro: -NO₂.
Sulfhydryl (thiol, mercapto): -SH.
Thioether: -SR, wherein R is a thioether substituent, for example, a C₁₋₇alkyl group, a C₃₋₂₀heterocyclyl group, or a C₅₋₂₀aryl group, preferably a C₁₋₇alkyl group (also referred to herein as thioC₁₋₇alkyl). Examples of thioC₁₋₇alkyl groups include, but are not limited to, -SCH₃ and -SCH₂CH₃.
Sulfonamino: -NR¹S(=O)₂R, wherein R¹ is an amino substituent, as defined for amino groups, and R is a sulfonamino substituent, for example, a C₁₋₇alkyl group, a C₃-₂₀heterocyclyl group, or a C₅₋₂₀aryl group, preferably a C₁₋₇alkyl group. Examples of sulfonamino groups include, but are not limited to, -NHS(=O)₂CH₃ and -N(CH₃)S(=O)₂C₆H₅.
Sulfinamino: -NR¹S(=O)R, wherein R¹ is an amino substituent, as defined for amino groups, and R is a sulfinamino substituent, for example, a C₁₋₇alkyl group, a C₃-₂₀heterocyclyl group, or a C₅₋₂₀aryl group, preferably a C₁₋₇alkyl group. Examples of sulfinamino groups include, but are not limited to, -NHS (=O) CH₃ and -N(CH₃)S(=O)C₆H₅.
Sulfamyl: -S(=O)NR¹R², wherein R¹ and R² are independently amino substituents, as defined for amino groups. Examples of sulfamyl groups include, but are not limited to, -S (=O) NH₂ and -S (=O) N (CH₃) ₂.
Sulfonamido: -S(=O)₂NR¹R², wherein R¹ and R² are independently amino substituents, as defined for amino groups. Examples of sulfonamido groups include, but are not limited to, -S(=O)₂NH₂ and -S(=O)₂N(CH₃)₂.
C₁₋₇alkyl: The term "C₁₋₇alkyl," as used herein, pertains to monovalent alkyl groups having from 1 to 7 carbon atoms, which may be aliphatic or alicyclic, or a combination thereof, and which may be saturated, partially unsaturated, or fully unsaturated.

The term "aliphatic," as used herein, pertains to groups which are linear or branched, but not cyclic. The term "alicyclic," as used herein, pertains to groups which have one ring, or two or more rings (e.g., spiro, fused, bridged), but which are not aromatic. The term "saturated," as used herein, pertains to groups which do not have any carbon-carbon double bonds or carbon-carbon triple bonds. The term "unsaturated," as used herein, pertains to groups which have at least one carbon-carbon double bond or carbon-carbon triple bond.

Examples of saturated linear C₁₋₇alkyl groups include, but are not limited to, methyl, ethyl, n-propyl, n-butyl, and n-pentyl (amyl).

Examples of saturated branched C₁₋₇alkyl groups include, but are not limited to, iso-propyl, iso-butyl, sec-butyl, tert-butyl, and neo-pentyl.

Examples of saturated alicylic (carbocyclic) C₁₋₇alkyl groups (also referred to as "C₃₋₇cycloalkyl" groups) include, but are not limited to, cyclopropyl, cyclobutyl, cyclopentyl, and cyclohexyl, as well as groups which comprise such groups, including, but not limited to, cyclopropylmethyl and cyclohexylmethyl.

Examples of unsaturated C₁₋₇alkyl groups which have one or more carbon-carbon double bonds (also referred to as "C₂₋₇alkenyl" groups) include, but are not limited to, ethenyl (vinyl) and 2-propenyl (allyl).

Examples of unsaturated C₁₋₇alkyl groups which have one or more carbon-carbon triple bonds (also referred to as "C₂₋₇alkynyl" groups) include, but are not limited to, ethynyl (ethinyl) and 2-propynyl (propargyl).

Examples of unsaturated alicylic (carbocyclic) C₁₋₇alkyl groups which have one or more carbon-carbon double bonds (also referred to as "C₃₋₇cycloalkenyl" groups) include, but are not limited to, cyclopropenyl and cyclohexenyl, as well as groups which comprise such groups, including but not limited to cyclopropenylmethyl and cyclohexenylmethyl.

C₃₋₂₀heterocyclyl : The term "C₃₋₂₀heterocyclyl, as used herein, pertains to a monovalent moiety obtained by removing a hydrogen atom from a ring atom of an alicyclic (i.e., nonaromatic cyclic) compound, said compound having one ring, or two or more rings (e.g., spiro, fused, bridged), having from 3 to 20 ring atoms, of which from 1 to 10 are ring heteroatoms, including, but not limited to, nitrogen, oxygen, and sulfur. Preferably, each ring has from 3 to 7 ring atoms, of which from 1 to 4 are ring heteroatoms. "C₃₋₂₀" denotes ring atoms, whether carbon atoms or heteroatoms.

Examples of monocyclic C₃₋₂₀heterocyclyl groups include, but are not limited to, those derived from:
N₁: aziridine (C₃), azetidine (C₄) , pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆) , tetrahydropyridine (C₆) , azepine (C₇);
O₁: oxirane (C₃) , oxetane (C₄), oxolane (tetrahydrofuran) (C₅), oxole (dihydrofuran) (C₅) , oxane (tetrahydropyran) (C₆), dihydropyran (C₆), pyran (C₆), oxepin (C₇);
S₁: thiirane (C₃) , thietane (C₄) , thiolane (tetrahydrothiophene) (C₅) , thiane (tetrahydrothiopyran) (C6), thiepane (cm) ;
O₂: dioxolane (C₅), dioxane (C₆), and dioxepane (C₇);
O₃: trioxane (C₆) ;
N₂: imidazolidine (C₅) , pyrazolidine (diazolidine) (C₅) , imidazoline (C₅), pyrazoline (dihydropyrazole) (C₅), piperazine (C₆);
N₁O₁: tetrahydrooxazole (C₅) , dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅) , thiazolidine (C₅), thiomorpholine (C₆);
N₂O₁: oxadiazine (C₆) ;
O₁S₁: oxathiole (C₅) and oxathiane (thioxane) (C₆); and,
N₁O₁S₁: oxathiazine (C₆).
C₅₋₂₀aryl: The term "C₅₋₂₀aryl, as used herein, pertains to a monovalent moiety obtained by removing a hydrogen atom from a ring atom of an aromatic compound, said compound having one ring, or two or more fused rings, and having from 5 to 20 ring atoms. The ring atoms may be all carbon atoms, as in "carboaryl groups," or may include one or more heteroatoms (including but not limited to oxygen, nitrogen, and sulfur), as in "heteroaryl groups." In the latter case, the group may conveniently be referred to as a "C₅₋₂₀heteroaryl" group, wherein "C₅₋₂₀" denotes ring atoms, whether carbon atoms or heteroatoms. Preferably, each ring has from 3 to 7 ring atoms, of which from 0 to 4 are ring heteroatoms.
Examples of carboaryl groups include, but are not limited to, those derived from benzene (i.e., phenyl) (C₆), naphthalene (C₁₀) , azulene (C₁₀) , anthracene (C₁₄) , phenanthrene (C₁₄) , naphthacene (C₁₈), and pyrene (C₁₆).

Examples of aryl groups which comprise fused rings, at least one of which is an aromatic ring, include, but are not limited to, groups derived from indene (C₉), isoindene (C₉), and fluorene (C₁₃).

Examples of monocyclic heteroaryl groups include, but are not limited to, those derived from:
N₁: pyrrole (azole) (C₅), pyridine (azine) (C₆);
O₁: furan (oxole) (C₅) ;
S₁: thiophene (thiole) (C₅) ;
N₁O₁: oxazole (C₅) , isoxazole (C₅) , isoxazine (C₆) ;
N₂O₁: oxadiazole (furazan) (C₅);
N₃O₁: oxatriazole (C₅) ;
N₁S₁: thiazole (C₅), isothiazole (C₅) ;
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅) , triazine (C₆) ; and,
N₄: tetrazole (C₅) .

Examples of heterocyclic groups (some of which are also heteroaryl groups) which comprise fused rings, include, but are not limited to:
C₉heterocyclic groups (with 2 fused rings) derived from benzofuran (O₁) , isobenzofuran (O₁), indole (N₁) , isoindole (N₁), purine (N₄) (e.g., adenine, guanine), benzimidazole (N₂), benzoxazole (N₁O₁), benzisoxazole (N₁O₁) , benzodioxole (O₂), benzofurazan (N₂O₁) , benzotriazole (N₃), benzothiofuran (S₁) , benzothiazole (N₁S₁) , benzothiadiazole (N₂S) ;
C₁₀heterocyclic groups (with 2 fused rings) derived from benzodioxan (O₂), quinoline (N₁), isoquinoline (N₁), benzoxazine (N₁O₁), benzodiazine (N₂), pyridopyridine (N₂), quinoxaline (N₂), quinazoline (N₂) ;
C₁₃heterocyclic groups (with 3 fused rings) derived from carbazole (N₁), dibenzofuran (O₁), dibenzothiophene (S₁) ; and,
C₁₄heterocyclic groups (with 3 fused rings) derived from acridine (N₁) , xanthene (O₁) , phenoxathiin (O₁S₁) , phenazine (N₂), phenoxazine (N₁O₁) , phenothiazine (N₁S₁) , thianthrene (S₂) , phenanthridine (N₁) , phenanthroline (N₂), phenazine (N₂).

Heterocyclic groups (including heteroaryl groups) which have a nitrogen ring atom in the form of an -NH- group may be N-substituted, that is, as -NR-. For example, pyrrole may be N-methyl substituted, to give N-methypyrrole. Examples of N-substitutents include, but are not limited to C₁₋₇alkyl, C₃₋₂₀heterocyclyl, C₅₋₂₀aryl, and acyl groups .

Heterocyclic groups (including heteroaryl groups) which have a nitrogen ring atom in the form of an -N= group may be substituted in the form of an N-oxide, that is, as -N(→O)= (also denoted -N⁺(→O⁻)=). For example, quinoline may be substituted to give quinoline N-oxide; pyridine to give pyridine N-oxide; benzofurazan to give benzofurazan N-oxide (also known as benzofuroxan).

Cyclic groups may additionally bear one or more oxo (=O) groups on ring carbon atoms. Monocyclic examples of such groups include, but are not limited to, those derived from:
C₅: cyclopentanone, cyclopentenone, cyclopentadienone;
C₆: cyclohexanone, cyclohexenone, cyclohexadienone;
O₁: furanone (C₅), pyrone (C₆);
N₁: pyrrolidone (pyrrolidinone) (C₅), piperidinone (piperidone) (C₆), piperidinedione (C₆);
N₂: imidazolidone (imidazolidinone) (C₅), pyrazolone (pyrazolinone) (C₅), piperazinone (C₆), piperazinedione (C₆), pyridazinone (C₆), pyrimidinone (C₆) (e.g., cytosine), pyrimidinedione (C₆) (e.g., thymine, uracil), barbituric acid (C₆) ;
N₁S₁: thiazolone (C₅), isothiazolone (C₅) ;
N₁O₁: oxazolinone (C₅).

Polycyclic examples of such groups include, but are not limited to, those derived from:
C₉: indenedione;
N₁: , oxindole (C₉) ;
O₁: benzopyrone (e.g., coumarin, isocoumarin, chromone) (C₁₀) ;
N₁O₁: benzoxazolinone (C₉), benzoxazolinone (C₁₀);
N₂: quinazolinedione (C₁₀);
N₄: purinone (C₉) (e.g., guanine).

Still more examples of cyclic groups which bear one or more oxo (=O) groups on ring carbon atoms include, but are not limited to, those derived from:
cyclic anhydrides (-C(=O)-O-C(=O)- in a ring), including but not limited to maleic anhydride (C₅), succinic anhydride (C₅), and glutaric anhydride (C₆);
cyclic carbonates (-O-C(=O)-O- in a ring), such as ethylene carbonate (C₅) and 1,2-propylene carbonate (C₅);
imides (-C(=O)-NR-C(=O)- in a ring), including but not limited to, succinimide (C₅), maleimide (C₅), phthalimide, and glutarimide (C₆);
lactones (cyclic esters, -O-C(=O)- in a ring), including, but not limited to, β-propiolactone, γ-butyrolactone, δ-valerolactone (2-piperidone), and ε-caprolactone;
lactams (cyclic amides, -NR-C(=O)- in a ring), including, but not limited to, β-propiolactam (C₄), γ-butyrolactam (2-pyrrolidone) (C₅), δ-valerolactam (C₆), and ε-caprolactam (C₇);
cyclic carbamates (-O-C(=O)-NR- in a ring), such as 2-oxazolidone (C₅) ; cyclic ureas (-NR-C(=O)-NR- in a ring), such as 2-imidazolidone (C₅) and pyrimidine-2,4-dione (e.g., thymine, uracil) (C₆).

The above C₁₋₇alkyl, C₃₋₂₀heterocyclyl, and C₅₋₂₀aryl groups, whether alone or part of another substituent, may themselves optionally be substituted with one or more groups selected from themselves and the preceding substituents (e.g., halo, hydroxy, carboxylic acid) to give substituted C₁₋₇alkyl groups, substituted C₃₋₂₀heterocyclyl groups, and substituted C₅₋₂₀aryl groups, respectively. Unless otherwise specified, a reference to a such a group is also a reference to the corresponding substituted group. Specific examples of such substituted groups are discussed below.

C₁₋₇haloalkyl group: The term "C₁₋₇haloalkyl group, as used herein, pertains to a C₁₋₇alkyl group in which at least one hydrogen atom has been replaced with a halogen atom (e.g., F, Cl, Br, I). If more than one hydrogen atom has been replaced with a halogen atom, the halogen atoms may independently be the same or different. Every hydrogen atom may be replaced with a halogen atom, in which case the group may conveniently be referred to as a C₁₋₇perhaloalkyl group." Examples of C₁₋₇haloalkyl groups include, but are not limited to, -CF₃, -CHF₂, -CH₂F, -CCl₃, -CBr₃, -CH₂CH₂F, -CH₂CHF₂, and -CH₂CF₃.

C₁₋₇haloalkoxy: -OR, wherein R is a C₁₋₇haloalkyl group. Examples of C₁₋₇haloalkoxy groups include, but are not limited to, -OCF₃, -OCHF₂, -OCH₂F, -OCCl₃, -OCBr₃, -OCH₂CH₂F, -OCH₂CHF₂, and -OCH₂CF₃.

C₁₋₇hydroxyalkyl: The term "C₁₋₇hydroxyalkyl group, as used herein, pertains to a C₁₋₇alkyl group in which at least one hydrogen atom has been replaced with a hydroxy group. Examples of C₁₋₇hydroxyalkyl groups include, but are not limited to, -CH₂OH, -CH₂CH₂OH, and -CH(OH)CH₂OH.

C₁₋₇carboxyalkyl: The term "C₁₋₇carboxyalkyl group," as used herein, pertains to a C₁₋₇alkyl group in which at least one hydrogen atom has been replaced with a carboxy group. Examples of C₁₋₇carboxyalkyl groups include, but are not limited to, -CH₂COOH and -CH₂CH₂COOH.

C₁₋₇aminoalkyl: The term "C₁₋₇aminoalkyl group," as used herein, pertains to a C₁₋₇alkyl group in which at least one hydrogen atom has been replaced with an amino group. Examples of C₁₋₇aminoalkyl groups include, but are not limited to, -CH₂NH₂, -CH₂CH₂NH₂, and -CH₂CH₂N(CH₃)₂.

C₁₋₇alkyl-C₅₋₂₀aryl: The term "C₁₋₇alkyl-C₅₋₂₀aryl," as used herein, describes certain C₅₋₂₀aryl groups which have been substituted with a C₁₋₇alkyl group. Examples of such groups include, but are not limited to, tolyl, xylyl, mesityl, and cumenyl.

C₁₋₇alkyl-C₅₋₂₀aryloxy: The term "C₁₋₇alkyl-C₅₋₂₀aryloxy," as used herein, describes certain C₅₋₂₀aryloxy groups which have been substituted with a C₁₋₇alkyl group. Examples of such groups include, but are not limited to, tolyloxy, xylyloxy, mesityloxy, and cumenyloxy.

C₅₋₂₀aryl-C₁₋₇alkyl: The term "C₅₋₂₀aryl-C₁₋₇alkyl, as used herein, describers certain C₁₋₇alkyl groups which have been substituted with a C₅₋₂₀aryl group. Examples of such groups include, but are not limited to, benzyl, tolylmethyl, phenylethyl, and triphenylmethyl (trityl).

C₅₋₂₀aryl-C₁₋₇alkoxy: The term "C₅₋₂₀aryl-C₁₋₇alkoxy," as used herein, describes certain C₁₋₇alkoxy groups which have been substituted with a C₅₋₂₀aryl group. Examples of such groups include, but are not limited to, benzyloxy, tolylmethoxy, and phenylethoxy.

C₅₋₂₀haloaryl : The term "C₅₋₂₀-haloaryl," as used herein, describes certain C₅₋₂₀aryl groups which have been substituted with one or more halo groups. Examples of such groups include, but are not limited to, halophenyl (e.g., fluorophenyl, chlorophenyl, bromophenyl, or iodophenyl, whether ortho-, meta-, or para-substituted), dihalophenyl, trihalophenyl, tetrahalophenyl, and pentahalophenyl. Included in the above are the well known ionic, salt, solvate, and protected forms of these substituents. For example, a reference to carboxylic acid (-COOH) also includes the anionic (carboxylate) form (-COO⁻), a salt or solvate thereof, as well as conventional protected forms. Similarly, a reference to an amino group includes the protonated form (-N⁺HR¹R²) , a salt or solvate of the amino group, for example, a hydrochloride salt, as well as conventional protected forms of an amino group. Similarly, a reference to a hydroxyl group also includes the anionic form (-O⁻), a salt or solvate thereof, as well as conventional protected forms of a hydroxyl group.

### Acronyms

For convenience, many chemical moieties are represented herein using well known abbreviations, including but not limited to, methyl (Me), ethyl (Et), n-propyl (nPr), iso-propyl (iPr), n-butyl (nBu), tert-butyl (tBu), n-hexyl (nHex), cyclohexyl (cHex), phenyl (Ph), biphenyl (biPh), benzyl (Bn), naphthyl (naph), methoxy (MeO), ethoxy (EtO), benzoyl (Bz), and acetyl (Ac) .

For convenience, many chemical compounds are represented herein using well known abbreviations, including but not limited to, methanol (MeOH), ethanol (EtOH), iso-propanol (i-PrOH), methyl ethyl ketone (MEK), acetic acid (AcOH), dichloromethane (methylene chloride, DCM), trifluoroacetic acid (TFA), dimethylformamide (DMF), and tetrahydrofuran (THF) .

### Isomers, Salts, Solvates, Protected Forms, and Prodrugs

A certain compound may exist in one or more particular geometric, optical, enantiomeric, diasteriomeric, epimeric, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

Note that, except as discussed below for tautomeric forms, specifically excluded from the term "isomers," as used herein, are structural (or constitutional) isomers (i.e., isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g., C₁₋₇alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro. 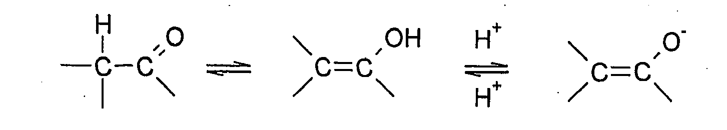 Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including racemic and other mixtures thereof. Methods for the preparation (e.g., asymmetric synthesis) and separation (e.g., fractional crystallisation and chromatographic means) of such isomeric forms are either known in the art or are readily obtained by adapting the methods taught herein in a known manner.

Unless otherwise specified, a reference to a particular compound also includes ionic, salt, hydrate, and protected forms of thereof, for example, as discussed below.

It may be convenient or desirable to prepare, purify, and/or handle a corresponding salt of the active compound, for example, a pharmaceutically-acceptable salt. Examples of pharmaceutically acceptable salts are discussed in Berge et al.; 1977, "Pharmaceutically Acceptable Salts," J. Pharm. Sci., Vol. 66, pp. 1-19.

For example, if the compound is anionic, or has a functional group which may be anionic (e.g., -COOH may be -COO⁻) , then a salt may be formed with a suitable cation. Examples of suitable inorganic cations include, but are not limited to, alkali metal ions such as Na+ and K+, alkaline earth cations such as Ca²⁺ and Mg²⁺, and other cations such as Al⁺³. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺) . Examples of some suitable substituted ammonium ions are those derived from: ethylamine, diethylamine, ethylenediamine, ethanolamine, diethanolamine, piperazine. An example of a common quaternary ammonium ion is N(CH₃)₄⁺.

If the compound is cationic, or has a functional group which may be cationic (e.g., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, anions from the following organic acids: acetic, propionic, succinic, gycolic, stearic, lactic, malic, tartaric, citric, ascorbic, maleic, hydroxymaleic, phenylacetic, glutamic, benzoic, salicylic, sulfanilic, 2-acetyoxybenzoic, fumaric, toluenesulfonic, methanesulfonic, ethane disulfonic, oxalic, isethionic, and valeric.

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a di-hydrate, a tri-hydrate, etc. It may be convenient or desirable to prepare, purify, and/or handle the active compound in a chemically protected form. The term "chemically protected form," as used herein, pertains to a compound in which one or more reactive functional groups are protected from undesirable chemical reactions, that is, are in the form of a protected or protecting group (also known as a masked or masking group). By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Green and P. Wuts, Wiley, 1991).

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)CH₃, -OAc).

For example, an aldehyde or ketone group may be protected as an acetal or ketal, respectively, in which the carbonyl group (>C=O) is converted to a diether (>C(OR)₂), by reaction with, for example, a primary alcohol. The aldehyde or ketone group is readily regenerated by hydrolysis using a large excess of water in the presence of acid.

For example, an amine group may be protected, for example, as an amide or a urethane, for example, as: a methyl amide (-NHCO-CH₃); a benzyloxy amide (-NHCO-OCH₂C₆H₅, -NH-Cbz); as a t-butoxy amide (-NHCO-OC(CH₃)₃, -NH-Boc); a 2-biphenyl-2-propoxy amide (-NHCO-OC(CH₃)₂C₆H₄C₆H₅, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulphonyl)ethyloxy amide (-NH-Psec); or, in suitable cases, as an N-oxide (>NO•).

For example, a carboxylic acid group may be protected as an ester or an amide, for example, as: a benzyl ester; a t-butyl ester; a methyl ester; or a methyl amide.

For example, a thiol group may be protected as a thioether (-SR), for example, as: a benzyl thioether; an acetamidomethyl ether (-S-CH₂NHC(=O)CH₃).

It may be convenient or desirable to prepare, purify, and/or handle the active compound in the form of a prodrug. The term "prodrug," as used herein, pertains to a compound which, when metabolised, yields the desired active compound. Typically, the prodrug is inactive, or less active than the active compound, but may provide advantageous handling, administration, or metabolic properties. For example, some prodrugs are esters of the active compound; during metabolysis, the ester group is cleaved to yield the active drug. Also, some prodrugs are activated enzymatically to yield the active compound, or a compound which, upon further chemical reaction, yields the active compound. For example, the prodrug may be a sugar derivative or other glycoside conjugate, or may be an amino acid ester derivative.

### Synthesis

The compounds of the present invention may be prepared using well known methods, or by adapting well known methods in well known ways.

For example, compounds of the type: 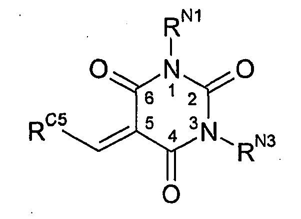 may be readily prepared by the acylation reaction of a parent ring system (e.g., barbituric acid) with an aldehyde, ketone, dinitrile, or other reactive species.

Many examples of such reactions have been reported in the chemical literature, including, but not limited to, the following:

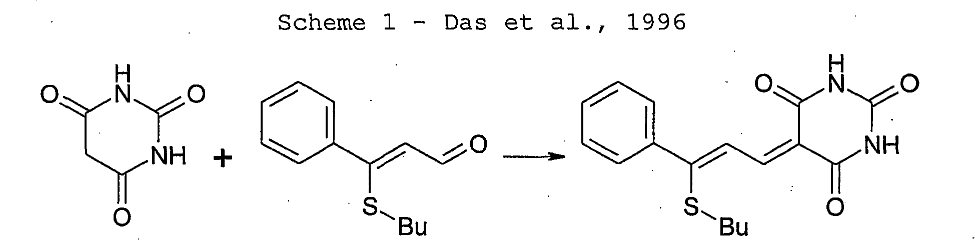

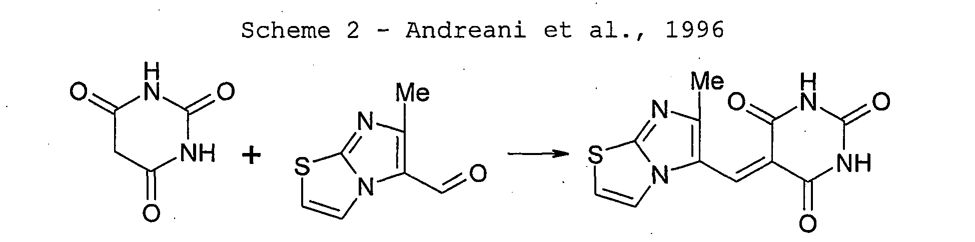

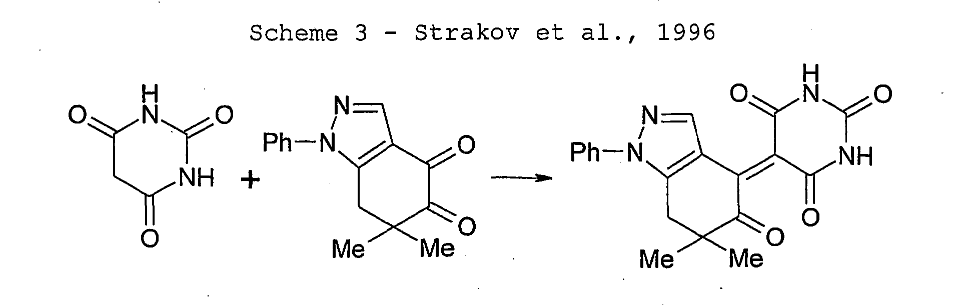

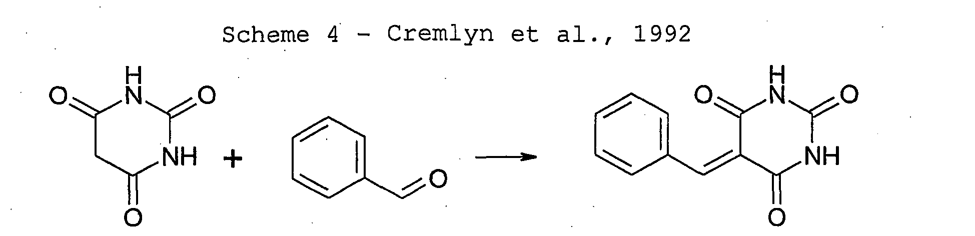

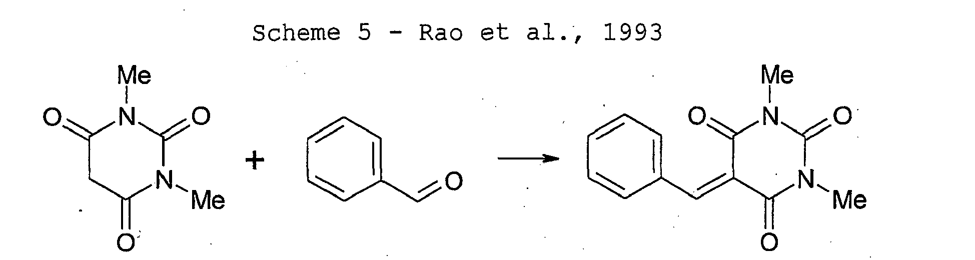

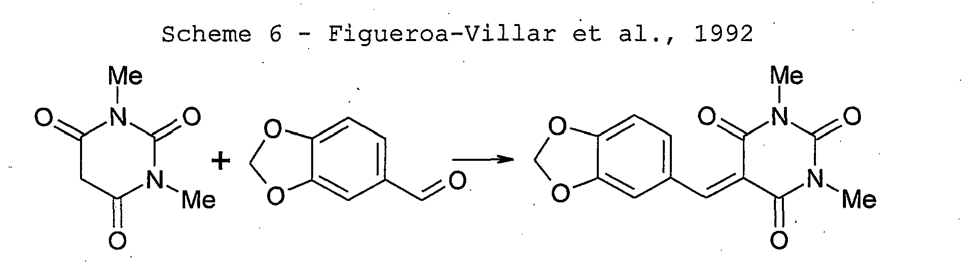

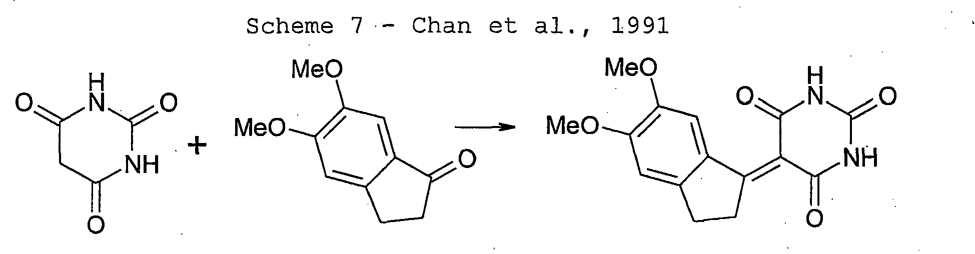

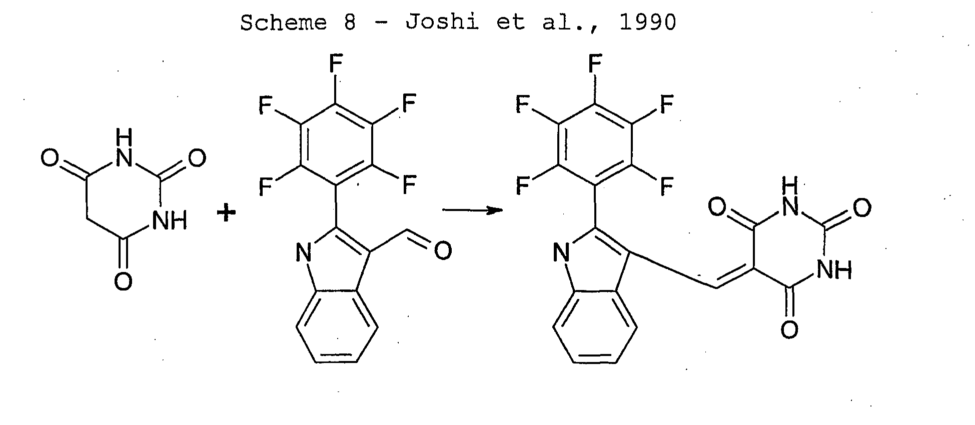

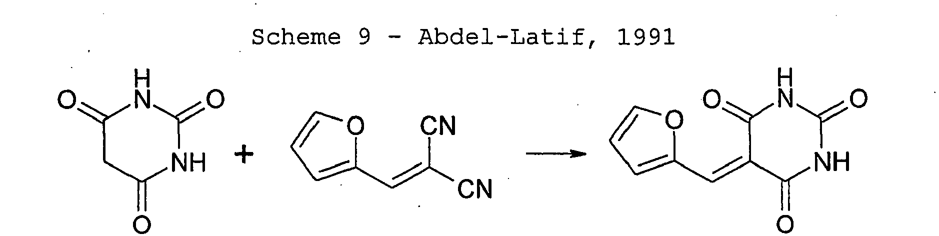

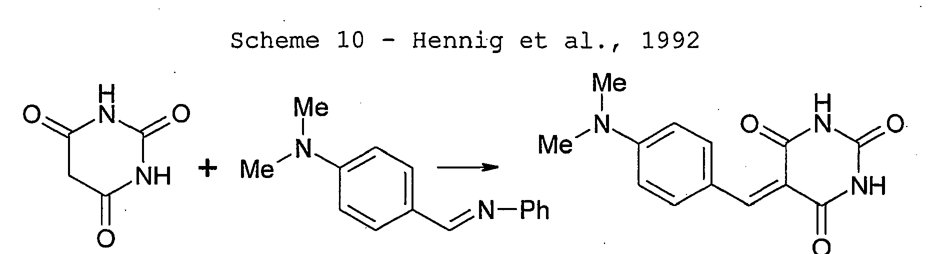

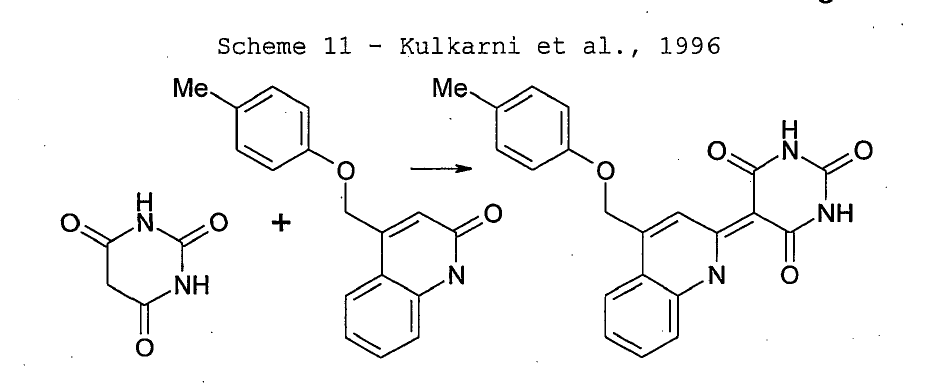

Compounds of the type: 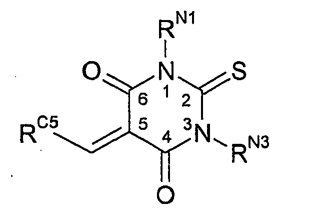 may be readily prepared using methods similar to those described above.

For example, such compounds may be prepared by reaction of a suitable aldehyde or dinitrile with a suitable thio-barbituric acid derivative. Examples of such reactions have been reported in the chemical literature, including, but not limited to, the following:

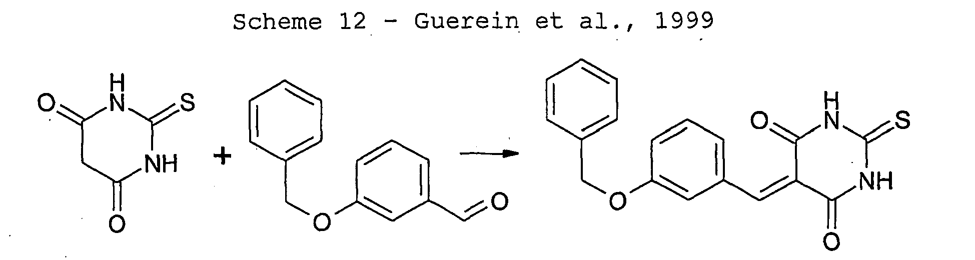

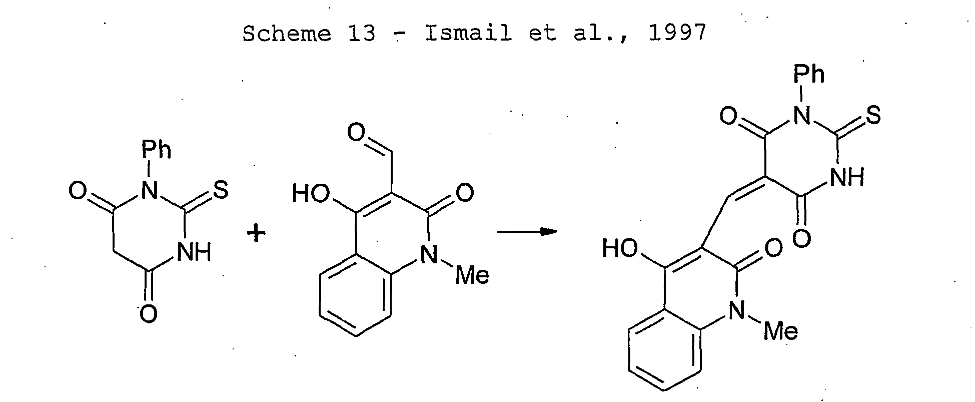

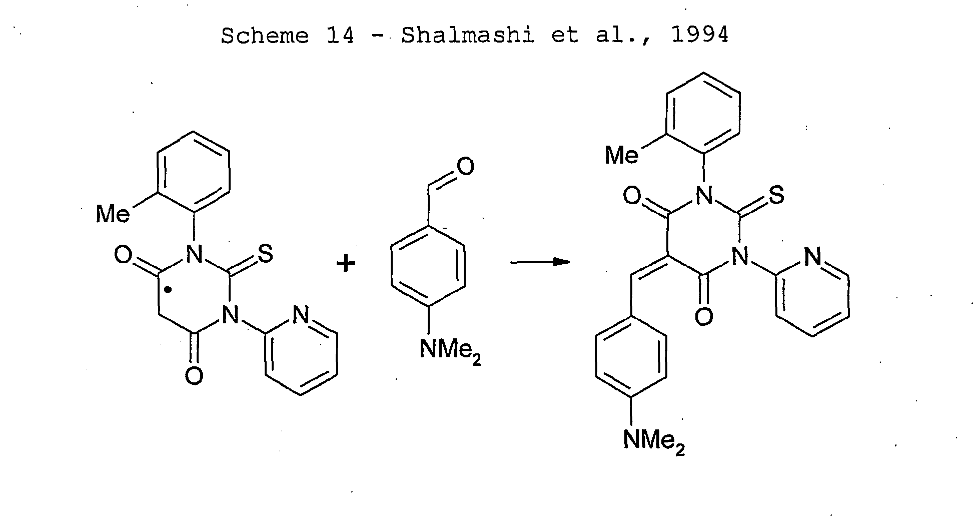

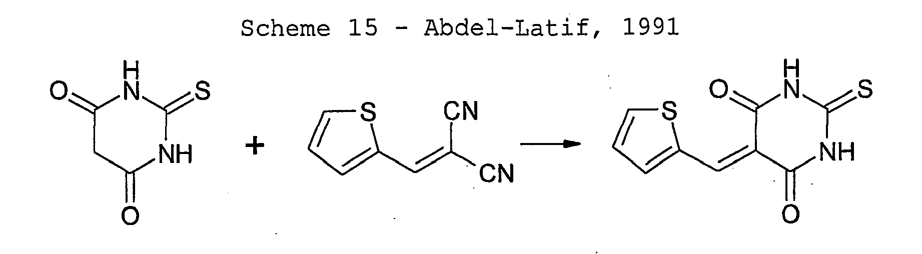

In one method (see also the Examples below), thiobarbituric acid and aldehyde are dissolved in ethanol. A catalytic amount of pyridine is added, and the reaction mixture is heated, e.g., to 60°C for 5 hours or, where R^{N1} and/or R^{N3} is phenyl, for 24 hours. In some cases, a precipitate is formed while in other cases, a coloured solution results. Ethanol is evaporated off and the residue washed, e.g., with petroleum ether (bp 40-60°C) .

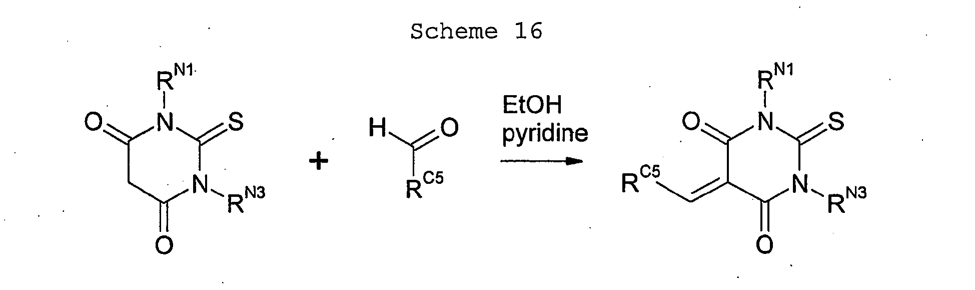

Also, such compounds may be prepared by reaction of a suitable thiourea with a suitable malonic acid. Examples of such reactions have been reported in the chemical literature, including, but not limited to, the following:

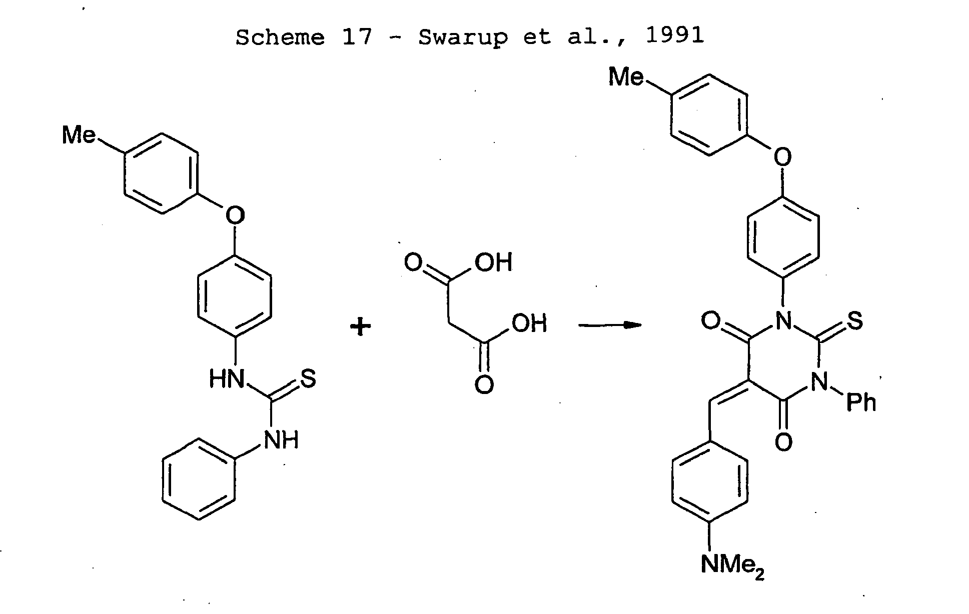

Also, such compounds may be prepared by addition of a suitable thio-barbituric acid to a suitable acetylenic compound. Examples of such reactions have been reported in the chemical literature, including, but not limited to, the following:

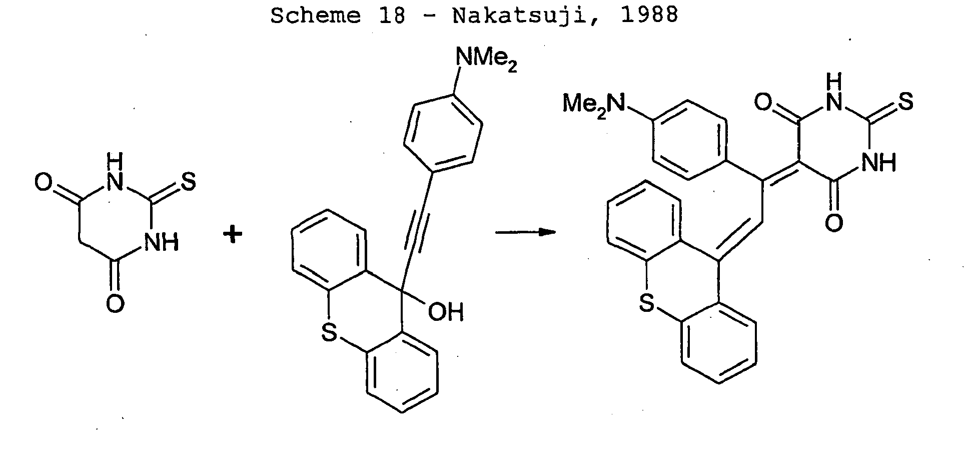

### Uses

The present invention provides active compounds which are capable of inhibiting HIF-1 activity (for example, capable of inhibiting the interaction between HIF-1α and p300), as well as methods of inhibiting HIF-1 activity, comprising contacting a cell with an effective amount of an active compound, whether *in vitro* or *in vivo*.

The term "active," as used herein, pertains to compounds which are capable of inhibiting HIF-1 activity.

One of ordinary skill in the art is readily able to determine whether or not a candidate compound is active, that is, capable of inhibiting HIF-1 activity, for example, capable of inhibiting the interaction between HIF-1α and p300. For example, assays which may conveniently be used to assess the inhibition offered by a particular compound are described in the examples below.

For example, a sample of cells (e.g., from a tumour) may be grown in vitro and a candidate compound brought into contact with the cells, and the effect of the compound on those cells observed. As examples of "effect," the morphological status of the cells may be determined (e.g., alive or dead), or the expression levels of genes regulated by the HIF-1 transcription factor. Where the candidate compound is found to exert an influence on the cells, this may be used as a prognostic or diagnostic marker of the efficacy of the compound in methods of treating a patient carrying the tumour or a tumour of the same cellular type.

Thus, in one aspect, the present invention provides angiogenesis inhibitors, as well as methods of inhibiting angiogenesis, comprising contacting a cell (e.g., a tumour cell, an endothelial cell, etc.) with an effective amount of an active compound, whether *in vitro* or *in vivo.* The term "angiogenesis inhibitor" as used herein, pertains to an active compound which inhibits angiogenesis, that is, which inhibits the progress of angiogenesis, and includes both a reduction in the rate of progress and a halt in the rate of progress.

Active compounds may also be used, as described above, in combination therapies, that is, in conjunction with other agents, for example, cytotoxic agents.

Active compounds may also be used as part of an in vitro assay, for example, in order to determine whether a candidate host is likely to benefit from treatment with the compound in question.

Active compounds may also be used as a standard, for example, in an assay, in order to identify other active compounds, other antiproliferative agents, etc.

### Administration

The active compound or pharmaceutical composition comprising the active compound may be administered to a subject by any convenient route of administration, whether systemically/peripherally or at the site of desired action, including but not limited to, oral (e.g, by ingestion); topical (including transdermal, intranasal, ocular, buccal, and sublingual); pulmonary (e.g., by inhalation therapy using, for example, an aerosol); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal.

The subject may be a eukaryote, an animal, a vertebrate animal, a mammal, a rodent (e.g., a guinea pig, a hamster, a rat, a mouse), murine (e.g., a mouse), a simian (e.g., a chimpanzee), or a human.

### Formulations

While it is possible for the active ingredient to be administered alone, it is preferable to present it as a pharmaceutical composition (e.g., formulation) comprising at least one active ingredient, as defined above, together with one or more pharmaceutically acceptable carriers, excipients, buffers, adjuvants, stabilisers, or other materials well known to those skilled in the art and optionally other therapeutic agents.

Thus, the present invention further provides pharmaceutical compositions, as defined above, and methods of making a pharmaceutical composition comprising admixing at least one active ingredient, as defined above, together with one or more pharmaceutically acceptable carriers, excipients, buffers, adjuvants, stabilisers, or other materials, as described herein.

The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

The formulations may conveniently be presented in unit dosage form and may be prepared by any methods well known in the art of pharmacy Such methods include the step of bringing into association the active ingredient with the carrier which constitutes one or more accessory ingredients.

In general, the formulations are prepared by uniformly and intimately bringing into association the active ingredient with liquid carriers or finely divided solid carriers or both, and then if necessary shaping the product.

Formulations may be in the form of liquids, solutions, suspensions, emulsions, tablets, losenges, granules, powders, capsules, cachets, pills, ampoules, suppositories, pessaries, ointments, gels, pastes, creams, sprays, foams, lotions, oils, boluses, electuaries, or aerosols.

Formulations suitable for oral administration (e.g., by ingestion) may be presented as discrete units such as capsules, cachets or tablets, each containing a predetermined amount of the active ingredient; as a powder or granules; as a solution or suspension in an aqueous or non-aqueous liquid; or as an oil-in-water liquid emulsion or a water-in-oil liquid emulsion; as a bolus; as an electuary; or as a paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (*e.g*., povidone, gelatin, hydroxypropylmethyl cellulose), lubricant, inert diluent, preservative, disintegrant (*e.g*., sodium starch glycolate, cross-linked povidone, cross-linked sodium carboxymethyl cellulose), surface-active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Formulations suitable for topical administration (e.g., transdermal, intranasal, ocular, buccal, and sublingual) may be formulated as an ointment, cream, suspension, lotion, powder, solution, past, gel, spray, aerosol, or oil. Alternatively, a formulation may comprise a patch or a dressing such as a bandage or adhesive plaster impregnated with active ingredients and optionally one or more excipients or diluents.

Formulations suitable for topical administration in the mouth include losenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations suitable for topical administration to the eye also include eye drops wherein the active ingredient is dissolved or suspended in a suitable carrier, especially an aqueous solvent for the active ingredient.

Formulations suitable for nasal administration, wherein the carrier is a solid, include a coarse powder having a particle size, for example, in the range of about 20 to about 500 microns which is administered in the manner in which snuff is taken, i.e., by rapid inhalation through the nasal passage from a container of the powder held close up to the nose. Suitable formulations wherein the carrier is a liquid for administration as, for example, nasal spray, nasal drops, or by aerosol administration by nebuliser, include aqueous or oily solutions of the active ingredient.

Formulations suitable for topical administration via the skin include ointments, creams, and emulsions. When formulated in an ointment, the active ingredient may optionally be employed with either a paraffinic or a water-miscible ointment base. Alternatively, the active ingredients may be formulated in a cream with an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulfoxide and related analogues.

When formulated as a topical emulsion, the oily phase may optionally comprise merely an emulsifier (otherwise known as an emulgent), or it may comprises a mixture of at lease one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulphate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient, such carriers as are known in the art to be appropriate.

Formulations suitable for parenteral administration (e.g., by injection, including cutaneous, subcutaneous, intramuscular, intravenous and intradermal), include aqueous and non-aqueous isotonic, pyrogen-free, sterile injection solutions which may contain anti-oxidants, buffers, preservatives, stabilisers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection. Typically, the concentration of the active ingredient in the solution is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freese-dried (lyophilised), condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

### Dosage

It will be appreciated that appropriate dosages of the active compounds, and compositions comprising the active compounds, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect.

Administration *in vivo* can be effected in one dose, continuously or intermittently throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 0.1 to about 250 mg per kilogram body weight of the subject per day. Where the active ingredient is a salt, an ester, prodrug, or the like, the amount administered is calculated on the basis the parent compound and so the actual weight to be used is increased proportionately.

### EXAMPLES

### Chemical Synthesis

Several compounds of the present invention were synthesized according to the general method illustrated in Scheme 16 above.

Thiobarbituric acid (250 mmol/dm³, 1.25 eq.) and aldehyde (200 mmol/dm³, 1 eq.) were dissolved in ethanol. A catalytic amount of pyridine (0.2 mmol/dm³, 0.001 eq.) was added, and the reaction mixture was heated, e.g., to 60°C for 5 hours or, where R^{N1} and/or R^{N3} is phenyl, for 24 hours. In some cases, a precipitate was formed while in other cases, a coloured solution resulted. Ethanol was evaporated off and the residue washed with petroleum ether (bp 40-60°C). Structures were confirmed using APCI mass spectrometry (Hewlett Packard MS 59893B) and ¹H NMR (Bruker 250 MHz).

### Example 1

### 5-(3-Phenyl-allylidene)-2-thioxo-dihydro-pyrimidine-4,6-dione (PX072015) (8)

Using the general method and 3-Phenyl-propenal gave a 63% yield of the desired product, 5-(3-Phenyl-allylidene)-2-thioxo-dihydro-pyrimidine-4,6-dione; MS: 258 (M⁻).

### Example 2

### 5-(5-Nitro-thiophen-2-ylmethylene)-2-thioxo-dihydro-pyrimidine-4,6-dione (PX074038) (10)

Using the general method and 5-Nitro-thiophene-2-carbaldehyde gave a 97% yield of the desired product, 5-(5-Nitro-thiophen-2-ylmethylene)-2-thioxo-dihydro-pyrimidine-4,6-dione, MS: 283 (M⁻), Mp = 285°C (decomposes).

### Example 3

### 5-(3,4-Dimethoxy-benzylidene)-2-thioxo-dihydro-pyrimidine-4,6-dione (PX075262) (18)

Using the general method and 3,4-Dimethoxy-benzaldehyde gave a 86% yield of the desired product, 5-(3,4-Dimethoxybenzylidene)-2-thioxo-dihydro-pyrimidine-4,6-dione, MS: 277, 291 (M⁻).

### Example 4

### 5-(1H-Indol-3-ylmethylene)-2-thioxo-dihydro-pyrimidine-4,6-dione (PX075276) (19)

Using the general method and 1H-Indole-3-carbaldehyde gave a 96% yield of the desired product, 5-(1H-Indol-3-ylmethylene)-2-thioxo-dihydro-pyrimidine-4,6-dione, MS: 270 (M⁻), ¹H NMR δ: 12.2(2H,bs,NH), 9.5(1H,s,CH), 8.7(1H,s,CH), 7.9(1H,m,CH), 7.6(1H,m,CH), 7.3(2H,m,CH), Mp = 320°C (decomposes).

### Example 5

### 5-(Furan-2-ylmethylene)-2-thioxo-dihydro-pyrimidine-4,6-dione (PX083634) (28)

Using the general method and Furan-2-carbaldehyde gave a 77% yield of the desired product, 5-Furan-2-ylmethylene-2-thioxo-dihydro-pyrimidine-4,6-dione, MS: 194, 222 (M⁻).

### Example 6

### 5-(4-dimethylamino-benzylidene)-2-thioxo-dihydro-pyrimidine-4,6-dione (PX089631) (48)

Using the general method and 4-dimethylamino benzaldehyde gave a 98% yield of the desired product, 5-(4-dimethylaminobenzylidene)-2-thioxo-dihydro-pyrimidine-4,6-dione, MS: 275 (M⁻), ¹H NMR δ: 12.1(2H,bs,NH), 8.5(2H,d,CH), 8.1(1H,s,CH), 6.9 (2H,d,CH), 3.2(6H,s,CH₃), Mp = 272°C (decomposes).

### Example 7

### N-[4-(4,6-Dioxo-2-thioxo-tetrahydro-pyrimidin-5-ylidenemethyl)-phenyl]-acetamide (PX089632) (49)

Using the general method and N-(4-Formyl-phenyl)-acetamide gave a 82% yield of the desired product, N-[4-(4,6-Dioxo-2-thioxo-tetrahydro-pyrimidin-5-ylidenemethyl)-phenyl]-acetamide, MS: 289 (M⁻).

### Example 8

### 5-(Naphthalen-1-ylmethylene)-2-thioxo-dihydro-pyrimidine-4,6-dione (PX089635) (51)

Using the general method and Naphthalene-1-carbaldehyde gave a 96% yield of the desired product, 5-Naphthalen-1-ylmethylene-2-thioxo-dihydro-pyrimidine-4,6-dione, MS: 281 (M⁻), Mp = 280°C (decomposes).

### Example 9

### 5-(1H-pyrrol-2-ylmethylene)-2-thioxo-dihydro-pyrimidine-4,6-dione (PX089639) (53)

Using the general method and 1H-pyrrole-2-carbaldehyde gave a 90% yield of the desired product, 5-(1H-pyrrol-2-ylmethylene)-2-thioxo-dihydro-pyrimidine-4,6-dione, MS: 221 (M⁻), ¹H NMR δ: 13.0(1H,s,NH 12.3(1H,s,NH), 8.1(1H,s,CH), 7.7(1H,s,CH), 7.4(1H,s,NH), 6.5(1H,s,CH), Mp = 280°C (decomposes).

### Example 10

### 5-(4-Chloro-benzylidene)-2-thioxo-dihydro-pyrimidine-4,6-dione (PX089640) (54)

Using the general method and 4-Chloro-benzaldehyde gave a 18% yield of the desired product, 5-(4-Chloro-benzylidene)-2-thioxo-dihydro-pyrimidine-4,6-dione, MS: 266, 268 (M⁻).

### Example 11

### 5-(4-Chloro-benzylidene)-1,3-diphenyl-2-thioxo-dihydro-pyrimidine-4,6-dione (PX089645) (56)

Using 1,3-Diphenyl-2-thioxo-dihydro-pyrimidine-4,6-dione and 4-Chloro-benzaldehyde gave a 95% yield of the desired product, 5-(4-Chloro-benzylidene)-1,3-diphenyl-2-thioxo-dihydro-pyrimidine-4,6-dione, MS: 418 (M⁻).

### Example 12

### 5-(4-Bromo-thiophen-2-ylmethylene)-2-thioxo-dihydro-pyrimidine-4,6-dione (PX089648) (59)

Using the general method and 4-Bromo-thiophene-2-carbaldehyde gave a 92% yield of the desired product, 5-(4-Bromo-thiophen-2-ylmethylene)-2-thioxo-dihydro-pyrimidine-4,6-dione, MS: 316, 318 (M⁻) , ¹H NMR δ: 12.4 (2H,s,NH), 8.5.(1H,S,CH), 8.4(1H,S,CH), 8.3(1H,S,CH), Mp = 230°C (decomposes).

### Example 13

### 5-(3-Benzyloxy-4-methoxy-benzylidene)-2-thioxo-dihydro-pyrimidine-4,6-dione (PX105990) (60)

Using the general method and 3-Benzyloxy-4-methoxy-benzaldehyde gave a 11% yield of the desired product, 5-(3-Benzyloxy-4-methoxy-benzylidene)-2-thioxo-dihydro-pyrimidine-4,6-dione, MS: 253, 367 (M⁻).

### Example 14

### 5-(2-Methoxy-benzylidene)-2-thioxo-dihydro-pyrimidine-4,6-dione (PX105993) (61)

Using the general method and 2-Methoxy-benzaldehyde gave a 91% yield of the desired product, 5-(2-Methoxy-benzylidene)-2-thioxo-dihydro-pyrimidine-4,6-dione, MS: 247, 261 (M⁻).

### Example 15

### 5-(4-Phenoxy-benzylidene)-2-thioxo-dihydro-pyrimidine-4,6-dione (PX106021) (62)

Using the general method and 4-Phenoxy-benzaldehyde gave a 78% yield of the desired product, 5-(4-Phenoxy-benzylidene)-2-thioxo-dihydro-pyrimidine-4,6-dione, MS: 247, 324 (M⁻).

### Example 16

5-(4-Styryl-benzylidene)-2-thioxo-dihydro-pyrimidine-4,6-dione (PX106027) (63)

Using the general method and 4-Styryl-benzaldehyde gave a 78% yield of the desired product, 5-(4-Styryl-benzylidene)-2-thioxo-dihydro-pyrimidine-4,6-dione, MS: 334 (M⁻).

### Example 17

### 5-(Anthracen-9-ylmethylene)-2-thioxo-dihydro-pyrimidine-4,6-dione (PX106031) (64)

Using the general method and Anthracene-9-carbaldehyde gave a 86% yield of the desired product, 5-Anthracen-9-ylmethylene-2-thioxo-dihydro-pyrimidine-4,6-dione, MS: 332 (M⁻).

### Example 18

### 5-(6,7-Dimethyl-4-oxo-4H-chromen-3-ylmethylene)-2-thioxo-dihydro-pyrimidine-4,6-dione (PX106036) (65)

Using the general method and 6,7-Dimethyl-4-oxo-4H-chromene-3-carbaldehyde gave a 29% yield of the desired product, 5-(6,7-Dimethyl-4-oxo-4H-chromen-3-ylmethylene)-2-thioxo-dihydro-pyrimidine-4,6-dione, MS: 418 (M⁻).

### Primary Assay

Candidate compounds were assessed for their ability to inhibit the interaction between HIF-1α and p300 using a high throughput fluorescence-based screening assay (DELFIA) as follows.

Some of the compounds were obtained from commercial sources (e.g., Sigma Aldrich, Fancy Road, Poole, Dorset, BH12 4QH, United Kingdom; Maybridge Chemical Company Ltd., Trevillet, Tintagel, Cornwall, PL34 0HW, United Kingdom; Menai Organics Ltd., Unit 5, Menai Technology Centre, Deiniol Road, Bangor, Gwynedd, North Wales, LL57 2UP, United Kingdom; Contact Services, P.O. Box 32, Strakhovoi Uchastock, Dolgoprudny, Moscow Region, 131700, Russia) and were used without further purification.

Some of the compounds were synthesized, as described herein.

A plasmid expressing His-HIF-1α fusion protein was constructed by PCR, by amplifying and subcloning a fragment of the human HIF-1α cDNA (NCBI GenBank, accession number AH006957) corresponding to the C-terminal 390 amino acids into vector pET28a (Novagen®, Madison, WI, USA). A plasmid containing the N-terminal 595 amino acids of human p300 (NCBI GenBank, accession number U01877) inserted into vector pGEX2T (Pharmacia®, Little Chalfont, Bucks, UK) was used for the production of GST-p300 fusion protein. The recombinant proteins were produced in E. coli. His-HIF-1α was purified using Ni-NTA agarose beads, according to manufacture's method (Qiagen®, Crawley, West Sussex, UK). GST-p300 was purified using Glutathione-sepharose beads (Amersham Pharmacia®, Little Chalfont, Bucks, UK) according to manufacturer's instructions. A titration of every batch of p300 was carried out in order to determine the optimum dilution of the protein to obtain at least a 10:1 ratio, signal to noise in the binding assay.

The assay was performed in 96-well Polysorb plates (Nalge. Nunc International®, Rochester, NY, USA) as follows. Plates were coated with His-HIF-1α at 50 ng/well in 100 mL PBS and incubated overnight at 4°C. The plates were then washed 3 times with deionized water and blocked with 100 µL/well 3% BSA in PBS for 3 hours at 4°C. After washing 3 times as before, GST-p300 was added at the appropriate dilution (1:800 in this screening) in binding buffer (50 mM HEPES pH 7.5, 50 mM NaCl, 0.1% BSA, 0.5 mM DTT). The reaction was incubated at room temperature for 1 hour. Plates were washed 3 times and anti-GST Europium-conjugated antibody (from Wallac®, Turku, Finland) was added at 50 ng/well in 100 mL of binding buffer. After 45 minutes incubation, plates were washed 3 times as before. Then, 100 µL/well enhancement solution (from Wallac®, Catalog No. 1244-105) was added and allowed to react for 15 minutes at room temperature. Plates were read on a Victor 2 plate reader (from Wallac®).

IC50 data (concentration of compound required to cause a 50% inhibition of the signal; or a different % inhibition, as indicated) for several compounds of the present invention, as determined using this assay, are shown in Table 1.

### Secondary cell-based assays

Compounds with inhibition activity, as determined using the primary assay, were subsequently evaluated using one or more secondary assays.

### VEGF-Luciferase

This cell-based reporter assay involves the use of a luciferase reporter gene under the direct control of the VEGF promoter. Induction of HIF using desferoxamine leads to the transcription of luciferase through activation of the VEGF (Vascular Endothelial Growth Factor) promoter, which in turn leads to an increase in luciferase activity, which can be measured using most commercially available luciferase assay kits. Molecules that disrupt the HIF complex cause inhibition of HIF-dependent luciferase activation and lead to a reduction in luciferase activity. This assay allows the activity of the compounds to be assessed against the VEGF promoter, which is essential for VEGF production and subsequent angiogenesis.

Hepatoma 3B (hep3B) cells (ATCC Ref. No. HB-8064) were plated in 24-well plates at 2 x 10⁴/well in 500 µL DMEM/10% FCS, and were transfected the following day using Fugene 6 (Roche Biochemicals®, Lewes, E. Sussex, UK). Transfection mixtures per well contained 6 µL 10% Fugene, 200 ng VEGF-luciferase reporter (rat VEGF promoter, NCBI GenBank, accession number U22373, Levy et al., 1995) and 2 ng TK-renilla (Promega®, Madison, WI, USA) (for transfection efficiency control). Transfection was performed as recommended by manufacturer. Compounds were added the following day. After 1 hour incubation at 37°C, desferoxamine (Sigma®, Dorset, UK) was added at 100 µM to induce HIF activity. Duplicate wells without desferoxamine were run in parallel. Cells were harvested 15 hours later, and luciferase activity was measured using Dual Luciferase Assay System (Promega®, see also Technical Manual, Part #TM040, Instructions for Use of Products E1910 and E1960, revised 5/99).

IC50 data (concentration of compound required to cause a 50% inhibition of the luciferase signal; or a different % inhibition, if indicated), for several compounds of the present invention, as determined using this assay, are shown in Table 1.

### VEGF-ELISA

This assay employs the quantitative sandwich enzyme immunoassay technique. A monoclonal antibody (R&D Systems®, Abingdon, Oxon, UK) specific for VEGF was pre-coated onto a microplate. To this was added a sample containing VEGF. After washing, a second anti-VEGF antibody coupled to horseradish peroxidase was added. After incubation and washing, the amount of bound antibody, and hence VEGF, was measured using a colorigenic substrate for horseradish peroxidase. Typically, cells were plated at a concentration of 2.5 x 10⁴ cells/well, and incubated with either 100 µM desferroxamine or at 0.1% O₂ for 17 hours at 37°C. 200 µL of supernatant were removed and the VEGF quantitated using the Quantikine® ELISA kit from R&D Systems® (catalog # DVE00) exactly according to the manufacturer's instructions. The assay is calibrated each time using recombinant human VEGF.

IC50 data (concentration of compound required to cause a 50% inhibition of the absorbance signal; or a different % inhibition, if indicated), for several compounds of the present invention, as determined using this assay, are shown in Table 1.

| Table 1 | | | | |
|---|---|---|---|---|
| No. | Ref. No. | Primary Assay | Secondary Assay | |
| | | Inhibition IC50 (µM) | VEGF-luciferase IC50 (µM) | VEGF-ELISA IC50 (µM) |
| 1 | PX069119 | 250 | - | - |
| 2 | PX069153 | 200 | - | - |
| 3 | PX072002 | 225 | - | - |
| 4 | PX072004 | 250 | - | - |
| 5 | PX072008 | 300 | - | - |
| 6 | PX072009 | 500 | - | - |
| 7 | PX072012 | 175 | - | - |
| 8 | PX072015 | 20(a) | 18 | - |
| 9 | PX074037 | 100 | 35 | 30 |
| 10 | PX074038 | 20 | 50 | - |
| 11 | PX074100 | 500 | - | - |
| 12 | PX074728 | 125 | - | - |
| 13 | PX075240 | 100 | - | - |
| 14 | PX075244 | 500 | - | - |
| 15 | PX075245 | 125 | - | - |
| 16 | PX075248 | 450 | - | - |
| 17 | PX075257 | 350 | - | - |
| 18 | PX075262 | 65 | - | - |
| 19 | PX075276 | 30 | 10.8 | 18.8 |
| 20 | PX075367 | 45 | 50 | 75 |
| 28 | PX083634 | 65 | 50 | - |
| 29 | PX083675 | 35 | 50 | 75 |
| 30 | PX083677 | 400 | - | - |
| 33 | PX089367 | 200 | - | - |
| 34 | PX089368 | 350 | - | - |
| 35 | PX089369 | 225 | - | - |
| 36 | PX089370 | 225 | - | - |
| 37 | PX089371 | 400 | - | - |
| 38 | PX089372 | 500 | - | - |
| 39 | PX089374 | 230 | - | - |
| 40 | PX089375 | 200 | - | - |
| 41 | PX089376 | 200 | - | - |
| 42 | PX089377 | 400 | - | - |
| 43 | PX089378 | 500 | - | - |
| 44 | PX089619 | 180 | - | - |
| 45 | PX089620 | 80 | - | - |
| 46 | PX089624 | 45 | 12.5 | 6.2 |
| 47 | PX089626 | 40 | 75 | - |
| 48 | PX089631 | 25 | 16.3 | 27.5 |
| 49 | PX089632 | 15 | - | - |
| 50 | PX089633 | 70 | 50 | - |
| 51 | PX089635 | 80 | - | - |
| 52 | PX089638 | 130 | - | - |
| 53 | PX089639 | 20 | 25 | 50 |
| 54 | PX089640 | 100 | - | - |
| 55 | PX089643 | 55 | 87.5 | 100 |
| 56 | PX089645 | 80 | - | 200 |
| 57 | PX089646 | 100 | - | - |
| 58 | PX089647 | 100 | - | - |
| 59 | PX089648 | 45 | 30 | 32.5 |
| 60 | PX105990 | 20 | 100(f) | - |
| 61 | PX105993 | 80 | - | - |
| 62 | PX106021 | 40(b) | 100 | 80 |
| 63 | px106027 | 20(c) | 75 | - |
| 64 | PX106031 | 80(d) | 50 | - |
| 65 | PX106036 | 20 | 50 | - |
| 66 | PX106130 | 29 | - | - |
| 67 | PX106151 | 160 | 40 | 75 |
| 68 | PX106155 | 10 | 27.5 | 45 |
| 69 | PX106174 | 43 | - | - |
| 70 | PX106244 | 40 | - | - |
| 71 | PX106255 | 3 | - | - |
| 72 | PX106265 | 50 | 32 | - |
| 73 | PX106274 | 29 | - | - |
| 74 | PX106281 | 35 | 28 | 30 |
| 75 | PX106287 | 80(e) | 100 | - |
| 76 | PX106291 | 80 | - | - |
| 77 | PX106297 | 32 | 100 | - |
| 78 | PX106326 | 40 | 50 | - |
| 79 | PX106341 | 49 | 57.5 | 20 |
| 80 | PX106343 | 49 | 100 | - |

| | | | | |
|---|---|---|---|---|
| (a) 58%; (b) 53%; (c) 55%; (d) 78%; (e) 35%; (f) 30%. | | | | |

### REFERENCES

A number of patents and publications are cited above in order to more fully describe and disclose the invention and the state of the art to which the invention pertains. Full citations for these references are provided below. Each of these references is incorporated herein by reference in its entirety into the present disclosure.
Abdel-Latif, F.F., 1991, Indian J. Chem., Sect. B., Vol. 30, No. 3, pp. 363-365.
Andreani, A., et al., 1996, Eur. J. Med. Chem., Vol. 31, No. 5, p. 383.
Arany et al., 1996, Proc. Natl. Acad. Sci. USA, Vol. 93, pp. 12969-12973.
Brown, J.M. , et al., 2000, Mol. Med. Today, Vol. 6, No. 4, pp. 157-162.
Chan, J.H., et al., 1991, J. Med. Chem., Vol. 34, No. 2, p. 550.
Cremlyn, R., et al., 1992, Phosphorus Sulphur Silicon Relat. Elem., Vol. 73, p. 161.
Das, S., et al., 1996, J. Chem: Soc., Perkin Trans. II, Vol. 4, p. 731.
Fellahi, Y., et al., 1995, Eur. J. Med. Chem., Vol. 30, No. 7-8, pp. 633-639.
Figueroa-Villar, J.D., et al., 1992, Heterocycles, Vol. 34, No. 5, p. 891.
Guerin, D.J., et al., 1999, Bioorg. Med. Chem. Lett., Vol. 9, No. 11, pp. 1477-1480.
Hennig, L., et al., 1992, Monatsh. Chem., Vol. 123, p. 571. Ismail, M.M., et al., 1997, Chem. Pap., Vol. 51, No. 1, pp. 43-47.
Joshi, K.C., et al., 1990, J. Indian Chem. Soc., Vol 67, No. 5, p. 434.
Kulkarni, G.M., et al., 1996, J. Indian Chem. Soc., Vol. 73, No. 9, p. 495.
Levy et al., 1995, "Transcriptional regulation of the rat Vascular Endothelial Growth Factor gene by hypoxia," J.B.C., Vol. 270, No. 2, pp. 13333-13340.
Livingston et al., 2000, published international (PCT) patent application, publication no. WO 00/74725, published 14 December 2000.
Miyazaki, M., et al., Res. Exp. Med., Vol. 187, No. 2, pp. 105-117.
Naguib, F.N., et al., 1993, Biochem. Pharmacol., Vol. 46, No. 7, pp. 1273-1283.
Nakatsuji, S., et al., 1988, Bull. Chem. Soc. Jap., Vol. 61, p. 2253.
Pan, S., et al., 1997, Yaoxue Xuebao, Vol. 32, No. 7, pp. 515-523.
Rao, P.S. et al., 1993, Indian J. Chem., Sect. B, Vol. 32, p. 484.
Rehse, K., et al., 1982, Arch. Pharm. (Weinheim, Ger.), Vol. 315, No. 6, pp. 502-509.
Richard, D.E., et al., 1999, Biochem. Biophys. Res. Commun., Vol. 266, No. 3, pp. 718-722.
Semenza, G.L., et al., 2000a, Annu. Rev. Cell Dev. Biol., Vol. 15, pp. 551-578.
Semenza, G.L., et al., 2000b, Biochem. Pharmacol., Vol. 59, No. 1, pp. 47-53.
Shalmashi, A., et al., 1994, Indian J. Chem., Sect. B., Vol. 33, No. 6, pp. 597-599.
Strakov, A. Ya., et al., 1996, Khim. Geterotsiki Soedin, Vol. 4, p. 501.
Swarup, S., et al., 1991, J. Indian Chem. Soc., Vol. 68, No. 5, pp. 302-304.
Taylor, E.C., et al., 1974, J. Org. Chem., p. 39.
Taylor, C.T., et al., 1999, Pharm. Res., Vol. 16, No. 10, pp. 1498-1505.
Vida, J.A., et al., 1974, J. Med. Chem., Vol. 17, No. 7, pp. 732-736.
Weinryb, I., et al., 1971, Arch. Biochem. Biophys., Vol. 146, No. 2, pp. 591-596.
Wenger, R.H., et al., 1999, Environ. Stress and Gene Regul., pp. 25-45.
Wood et al., 1996, J. Biol. Chem., Vol. 271, No. 25, pp. 15117-15123.

## Claims

1. Use of a compound of formula 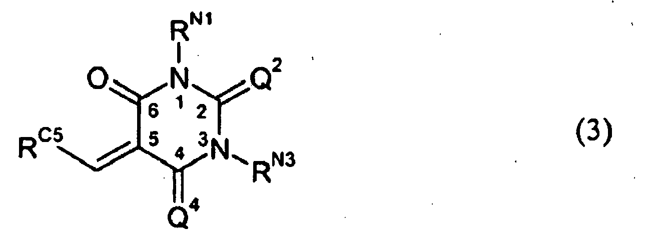 wherein:
Q² is =O, =S, or =NR^{N2};
Q⁴ is =O, =S, or =NR^{N4};
R^{C5} is a carbon substituent, and is independently **optionally substituted** aliphatic or alicyclic C₁₋₇alkyl, **which may be saturated, partially saturated or fully unsaturated;** optionally substituted C₃₋₂₀heterocyclyl, or optionally substituted C₅₋₂₀aryl and may be cis- or trans-;
each one of R^{N1}, R^{N2} , R^{N3}, and R^{N4} is a nitrogen substituent, and is independently hydrogen, optionally substituted C₁₋₇alkyl, optionally substituted C₃₋₂₀heterocyclyl, or optionally substituted C₅₋₂₀aryl;
in which the substituents are selected from hydrogen, halo, hydroxy, C₁₋₇alkoxy, C₅₋₂₀aryloxy, oxo, formyl, acyl, carboxy, carboxylate, acyloxy, amido, acylamido, amino, cyano, nitro, sulfhydryl, thioether, sulfonamino, sulfinamino, sulfamyl, sulfonamido, C₁₋₇alkyl, C₁₋₇haloalkyl, C₁₋₇hydroxyalkyl, C₁₋₇carboxyalkyl, C₁₋₇aminoalkyl, C₅₋₂₀aryl-C₁₋₇alkyl), optionally substituted C₃₋₂₀heterocyclyl, optionally substitutedC₅₋₂₀aryl, C₅
or a pharmaceutically acceptable salt or solvate thereof
for the manufacture of a medicament **for inhibiting angiogenesis.**

2. The use according to claim 1, wherein in compound (3) Q² is =O or =S; and Q⁴ is =O or =S.

3. The use according to claim 1, wherein in compound (3) Q² is =O and Q⁴ is =O; or Q² is =S and Q⁴ is =O.

4. The use according to claim 1, wherein in compound (3) Q² is =O and Q⁴ is =O and the compound has the following formula:

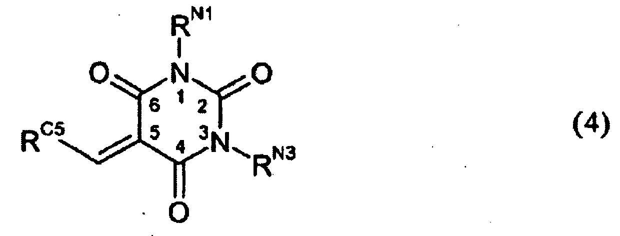

5. The use according to claim 1, wherein in compound (3) Q² is =S and Q⁴ is =O and the compound has the following formula:

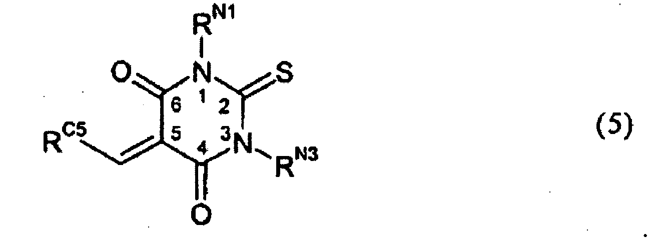

6. The use according to any one of claims 1-5, wherein in the compounds of formula (3)-(5) each one of R^{N1}, R^{N2}, R^{N3}, and R^{N4} is independently hydrogen, saturated aliphatic C₁₋₇alkyl, saturated aliphatic C₁₋₇haloalkyl, saturated aliphatic C₁₋₇hydroxyalkyl, saturated aliphatic C₁₋₇aminoalkyl, saturated aliphatic C₁₋₇carboxyalkyl, C₅₋₂₀aryl-C₁₋₇alkyl, C₅₋₂₀carboaryl, or C₅₋₂₀haloaryl.

7. The use according to any one of claims 1-5, wherein in the compounds of formula (3)-(5) each one of R^{N1}, R^{N2}, R^{N3}, and R^{N4} is independently -H, - Me, -Et, -CH₂COOH, -Ph, -C₆H₄F, -C₆H₄Cl, -C₆H₄Br, -C₆H₄-0CH₃, or -C₆H₄-CH₃.

8. The use according to any one of claims 1-7, wherein in the compounds of formula (3)-(5) R^{N1} is -H or other than -H; and, each one of R^{N2}, R^{N3}, and R^{N4} is -H.

9. The use according to any one of claims 1-7, wherein in the compounds of formula (3)-(5) R^{N3} is -H or other than -H; and each one of R^{N1}, R^{N2}, and R^{N4} is -H.

10. The use according to any one of claims 1-7, wherein in the compounds of formula (3)-(5) each one of R^{N1} R^{N2}, R^{N3}, and R^{N4} is -H.

11. The use according to any one of claims 1-10, wherein in the compounds of formula (3)-(5) R^{C5} is C₁₋₇alkyl, optionally substituted as indicated in claim 1.

12. The use according to any one of claims 1-10, wherein in the compounds of formula (3)-(5) R^{C5} is C₃₋₆cycloalkyl, optionally substituted as indicated in claim 1.

13. The use according to any one of claims 1-10, wherein in the compounds of formula (3)-(5) R^{C5} is partially unsaturated C₃₋₆cyloalkyl, optionally substituted as indicated in claim 1.

14. The use according to any one of claims 1-10, wherein in the compounds of formula (3)-(5) R^{C5} is C₅₋₂₀aryl-C₁₋₇alkyl, optionally substituted as indicated in claim 1.

15. The use according to any one of claims 1-10, wherein in the compounds of formula (3)-(5) R^{C5} is C₃₋₂₀heterocyclyl, optionally substituted as indicated in claim 1.

16. The use according to any one of claims 1-10, wherein in the compounds of formula (3)-(5) R^{C5} is C₅₋₂₀aryl, optionally substituted as indicated in claim 1.

17. The use according to any one of claims 1-10, wherein in the compounds of formula (3)-(5) R^{C5} is C₅₋₂₀carboaryl, optionally substituted as indicated in claim 1.

18. The use according to any one of claims 1-10, wherein R^{C5} is C₅₋₂₀heteroaryl, optionally substituted as indicated in claim 1.

19. The use according to any one of claims 1-10, wherein in the compounds of formula (3)-(5) R^{C5} is cyclohexenyl, phenyl, furanyl, thiophenyl, pyrrolyl, indolyl, or benzopyronyl, optionally substituted as indicated in claim. 1.

20. The use according to any one of claims 1-10, wherein in the compounds of formula (3)-(5) R^{C5} is a phenyl group, optionally substituted as indicated in claim 1.

21. The use according to any one of claims 1-10, wherein the compound has the following formula: 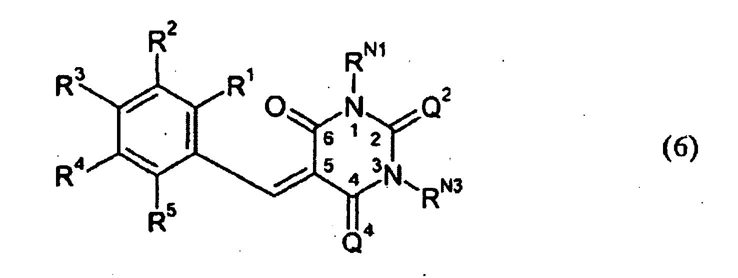 wherein:
each one of R¹ through R⁵ is a phenyl substituent, and is independently hydrogen, halo, hydroxy, ether, formyl, acyl, carboxy, carboxylate, amido, acylamido, amino, nitro, or C₁₋₇alkyl, C₃₋₂₀heterocyclyl, C₅₋₂₀aryl optionally substituted as indicated in claim 1; and,
two of R¹ through R⁵ may together form a bidentate structure which, together with the two carbon atoms to which it is attached, forms a cyclic structure with five or six ring atoms.

22. The use according to claim 21, wherein in compound (6):
each one of R¹ through R⁵ is independently hydrogen, halo, hydroxy, C₁₋₇alkoxy, C₅₋₂₀aryloxy, C₅₋₂₀aryl-C₁₋₇alkoxy, acyl, amino, or C₁₋₇alkyl, the C₅₋₂₀aryloxy, C₅₋₂₀aryl-C₁₋₇alkoxy and C₁₋₇alkyl groups being optionally substituted as indicated in claim 1
and
two of R¹ through R⁵ may form a bidentate structure selected from -(CH₂)₃-, -(CH₂)₄-, -0-CH₂-O-, and -O-CH2CH₂-O-.

23. The use according to claim 21, wherein in compound (6) each one of R¹ through R⁵ is independently selected from: -H, -F, -Cl, -Br, -I, -NMe₂, -Net₂, -OH, -OCH₃, -OCH₂CH₃, -OPh, -OCH₂Ph, -C(=O)CH₃, -CONH₂, -CONHCH₃, -NO₂, -CH₃, -CH₂CH₃, -CF₃, -OCF₃, -CH₂OH, -Ph, and -CH₂Ph.

24. The use according to claim 1, wherein the compound is selected from:
| | | |
|---|---|---|
| **(1)** | 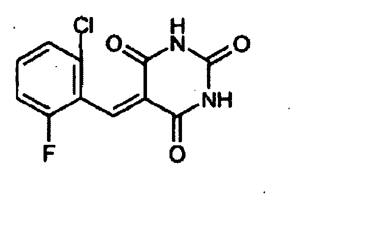 | **PX069119** |
| **(2)** | 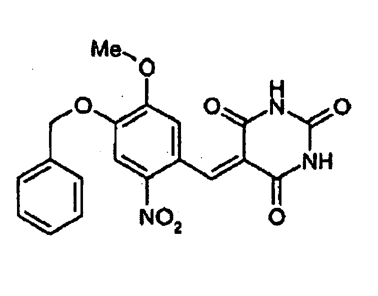 | **PX069153** |
| **(3)** | 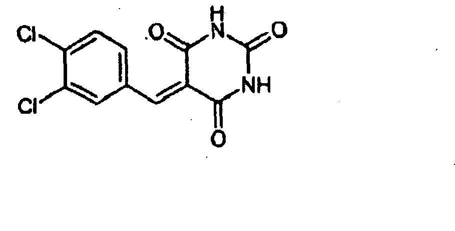 | **PX072002** |
| **(4)** | 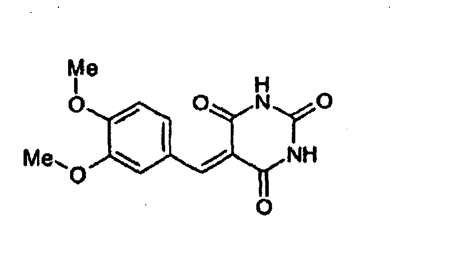 | **PX072004** |
| **(5)** | 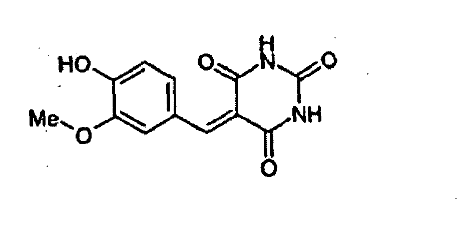 | **PX072008** |
| **(6)** | 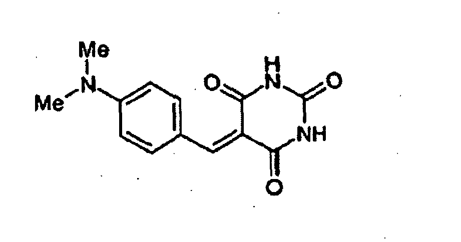 | **PX072009** |
| **(7)** | 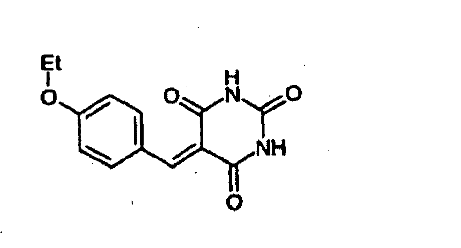 | **PX072012** |
| **(8)** | 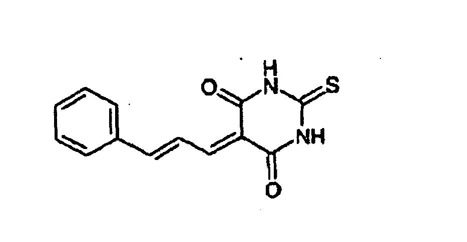 | **PX072015** |
| **(9)** | 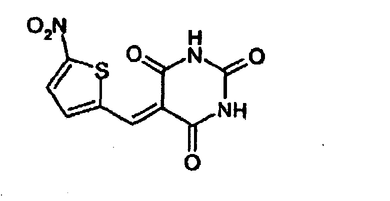 | **PX074037** |
| **(10)** | 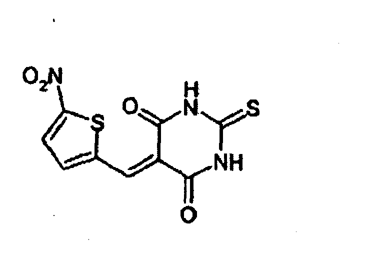 | **PX074038** |
| **(11)** | 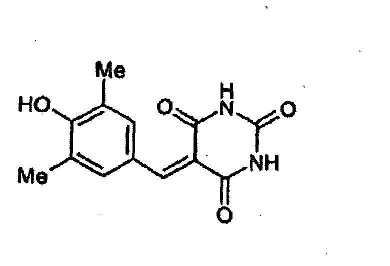 | **PX074100** |
| **(12)** | 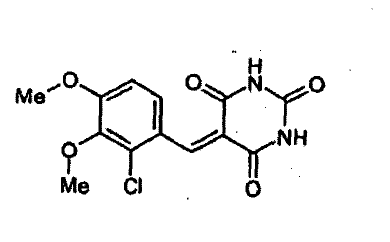 | **PX074728** |
| **(13)** | 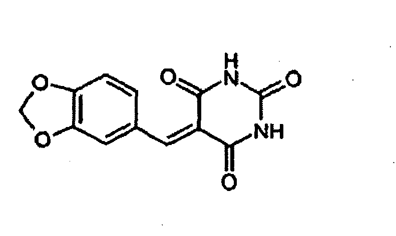 | **PX075240** |
| **(14)** | 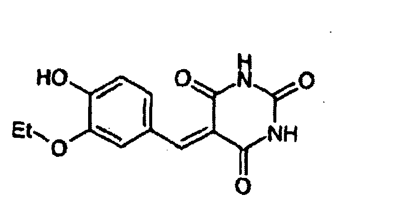 | **PX075244** |
| **(15)** | 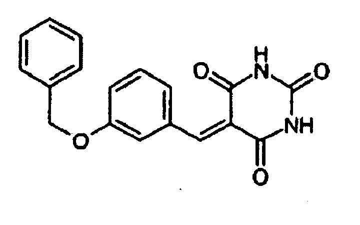 | **PX075245** |
| **(16)** | 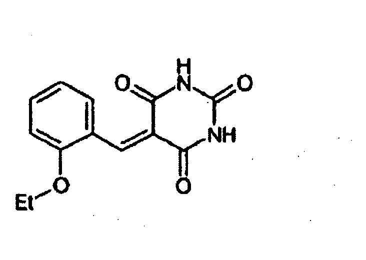 | **PX075248** |
| **(17)** | 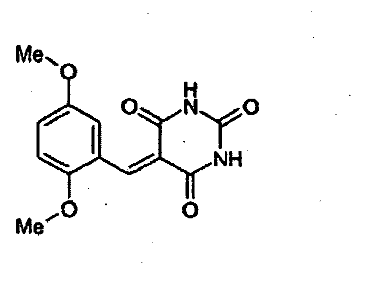 | **PX075257** |
| **(18)** | 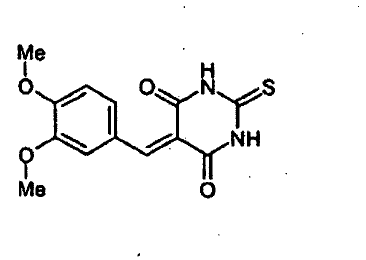 | **PX075262** |
| **(19)** | 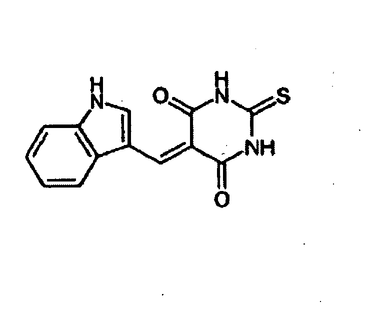 | **PX075276** |
| **(20)** | 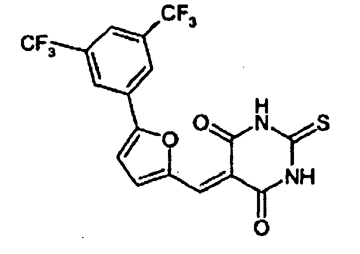 | **PX075367** |
| **(28)** | 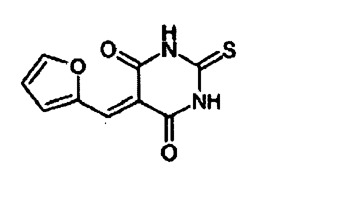 | **PX083634** |
| **(29)** | 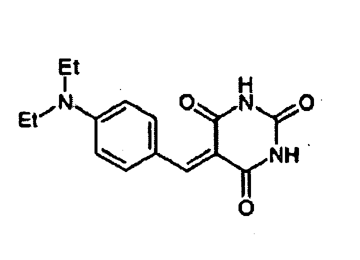 | **PX083675** |
| **(30)** | 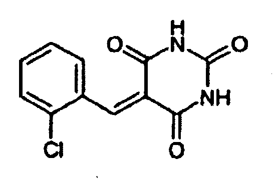 | **PX083677** |
| **(33)** | 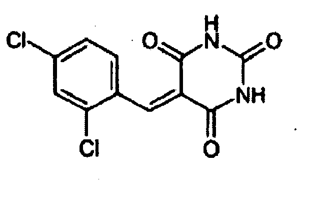 | **PX089367** |
| **(34)** | 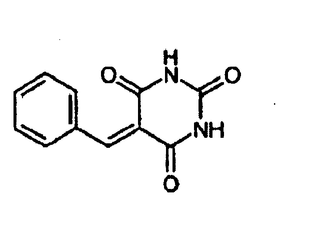 | **PX089368** |
| **(35)** | 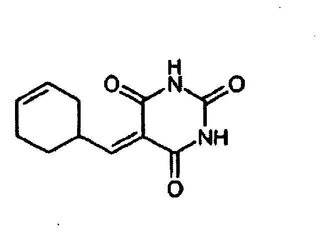 | **PX089369** |
| **(36)** | 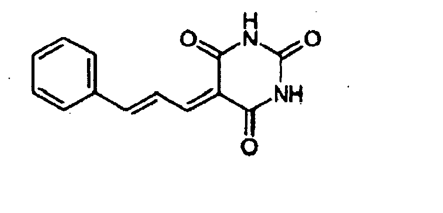 | **PX089370** |
| **(37)** | 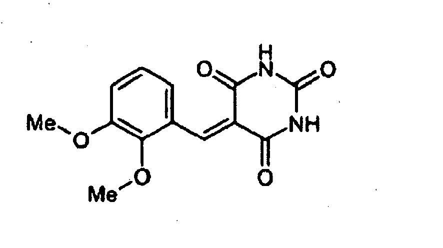 | **PX089371** |
| **(38)** | 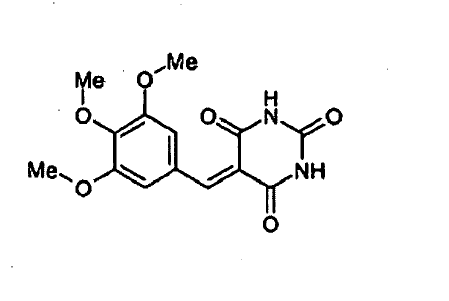 | **PX089372** |
| **(39)** | 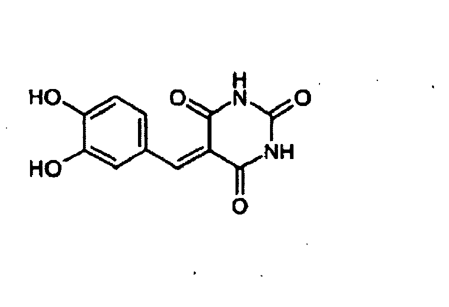 | **PX089374** |
| **(40)** | 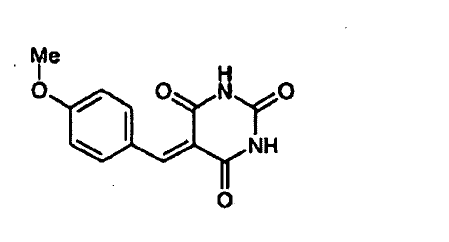 | **PX089375** |
| **(41)** | 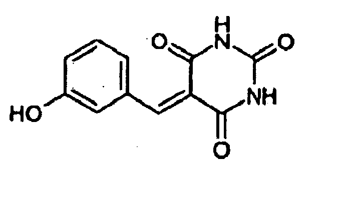 | **PX089376** |
| **(42)** | 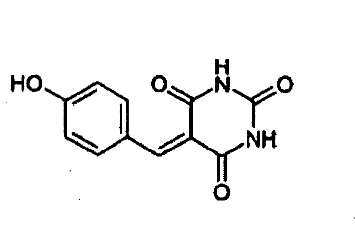 | **PX089377** |
| **(43)** | 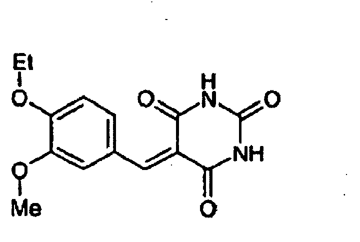 | **PX089378** |
| **(46)** | 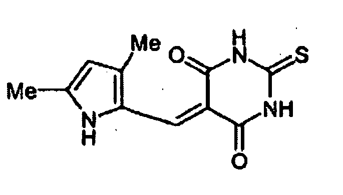 | **PX089624** |
| **(47)** | 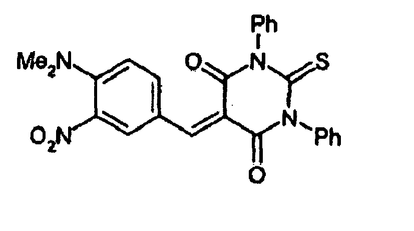 | **PX089626** |
| **(48)** | 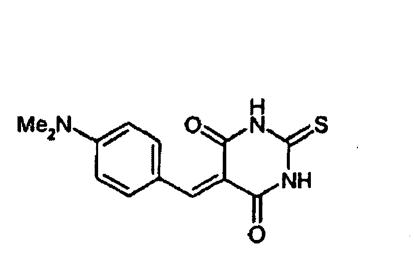 | **PX089631** |
| **(49)** | 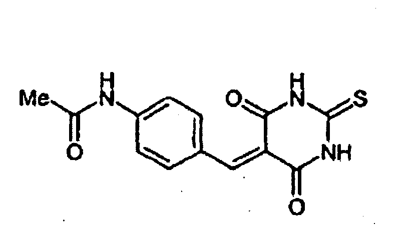 | **PX089632** |
| **(50)** | 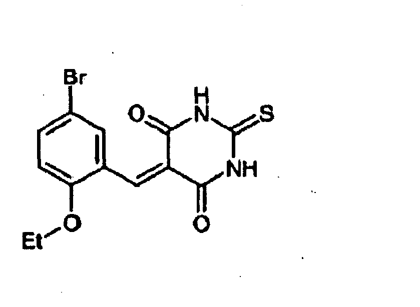 | **PX089633** |
| **(51)** | 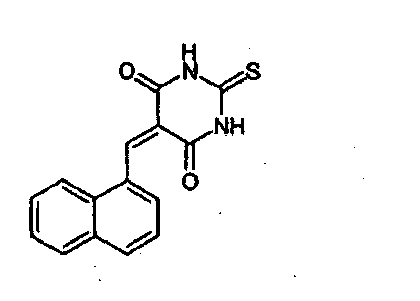 | **PX089635** |
| **(52)** | 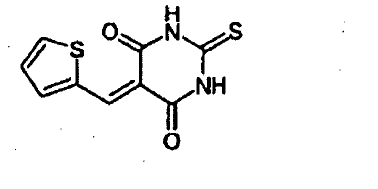 | **PX089638** |
| **(53)** | 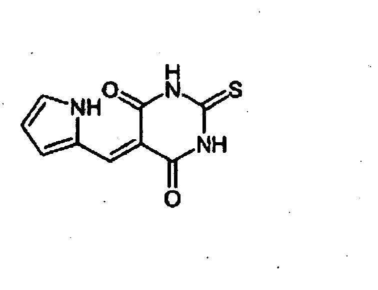 | **PX089639** |
| **(54)** | 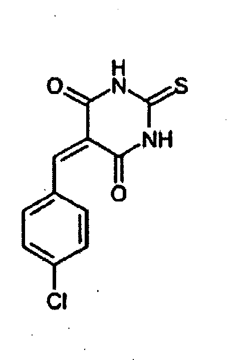 | **PX089640** |
| **(55)** | 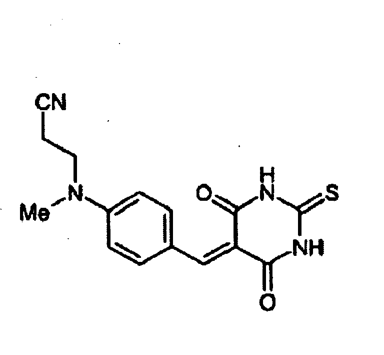 | **PX089643** |
| **(56)** | 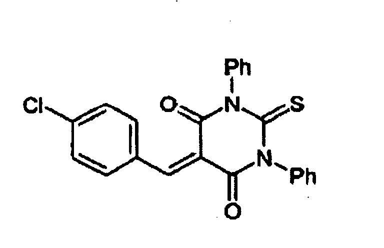 | **PX089645** |
| **(57)** | 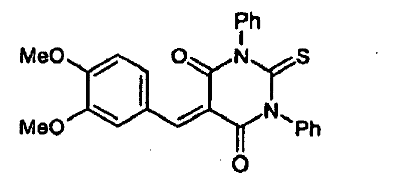 | **PX089646** |
| **(58)** | 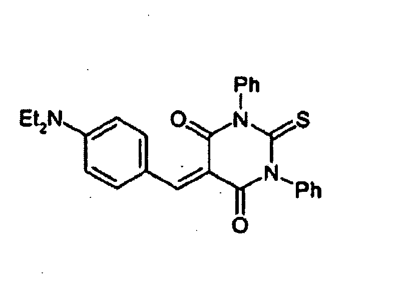 | **PX089647** |
| **(59)** | 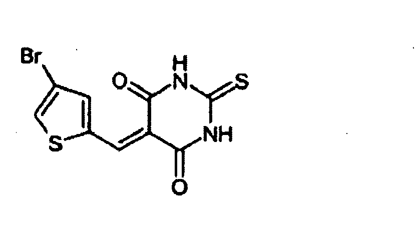 | **PX089648** |
| **(60)** | 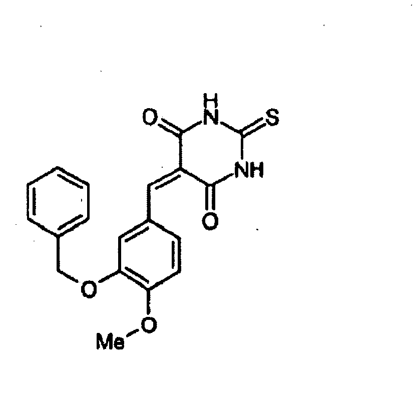 | **PX105990** |
| **(61)** | 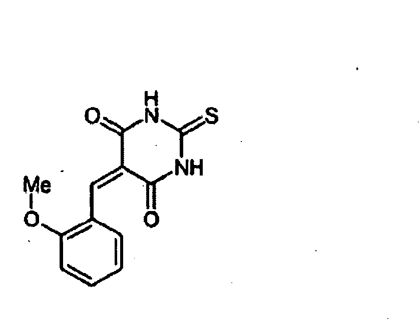 | **PX105993** |
| **(62)** | 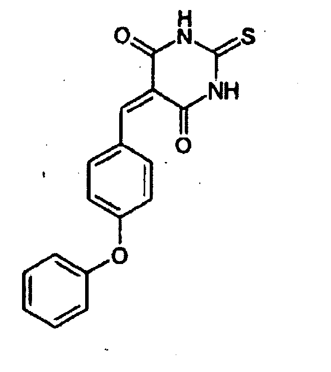 | **PX106021** |
| **(63)** | 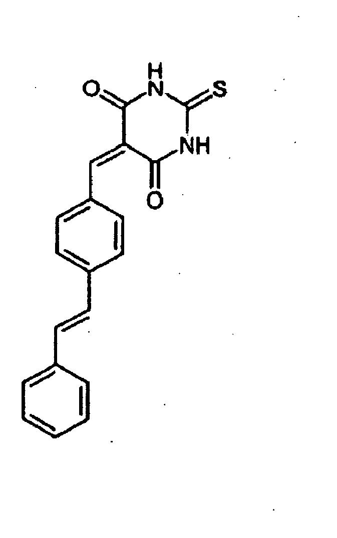 | **PX106027** |
| **(64)** | 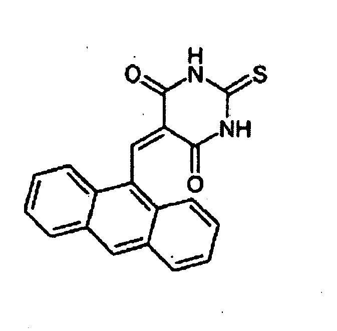 | **PX106031** |
| **(65)** | 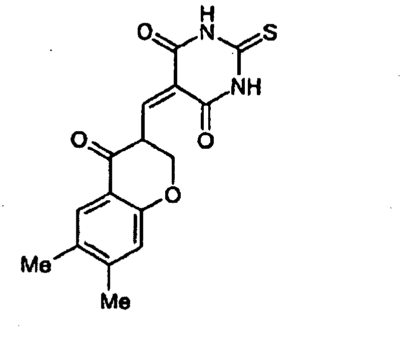 | **PX106036** |
| **(66)** | 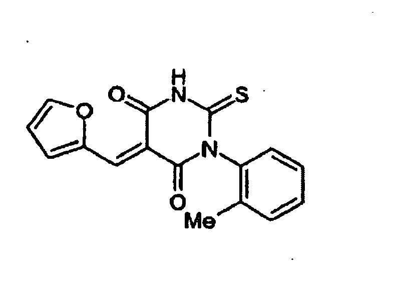 | **PX106130** |
| **(67)** | 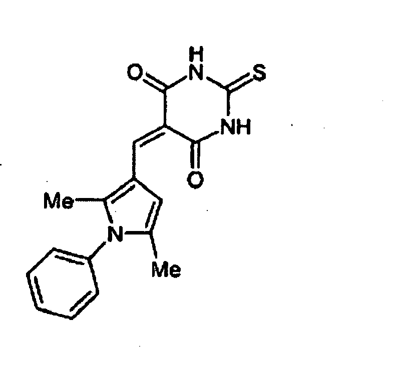 | **PX106151** |
| **(68)** | 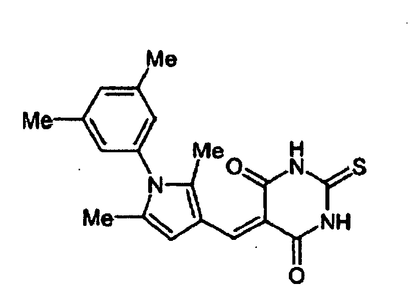 | **PX106155** |
| **(69)** | 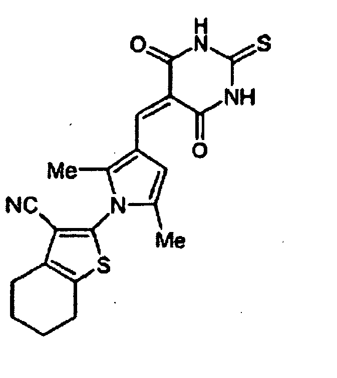 | **PX106174** |
| **(70)** | 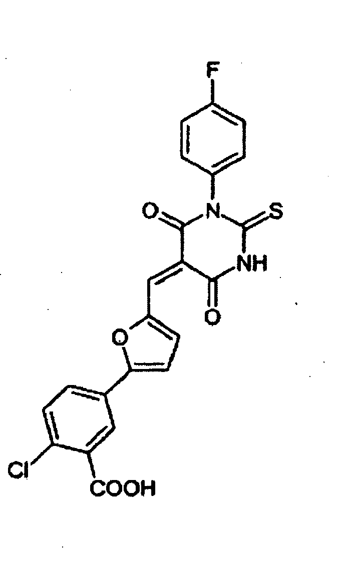 | **PX106244** |
| **(71)** | 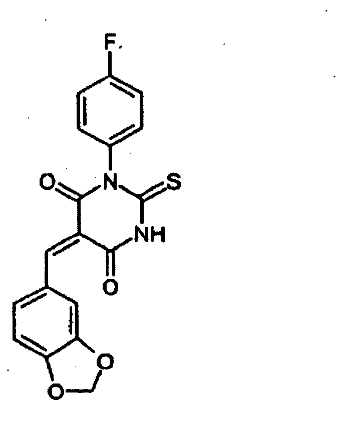 | **PX106255** |
| **(72)** | 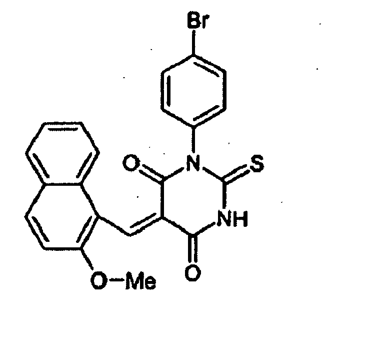 | **PX106265** |
| **(73)** | 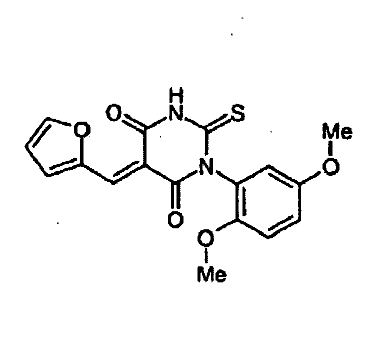 | **PX106274** |
| **(74)** | 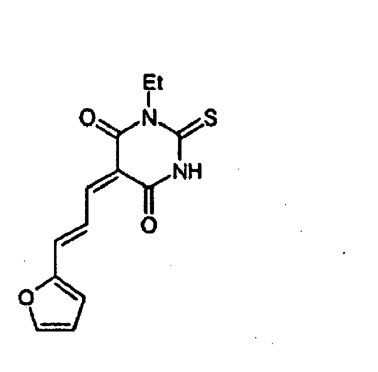 | **PX106281** |
| **(75)** | 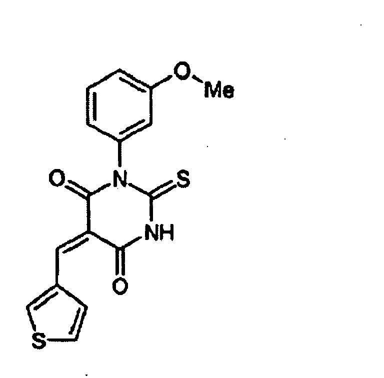 | **PX106287** |
| **(76)** | 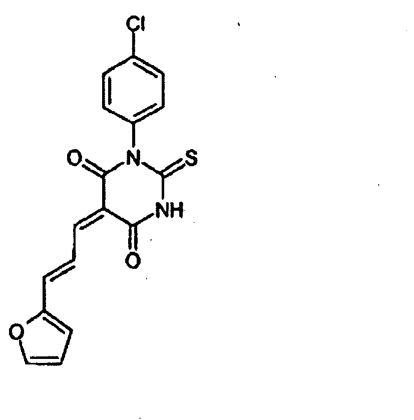 | **PX106291** |
| **(77)** | 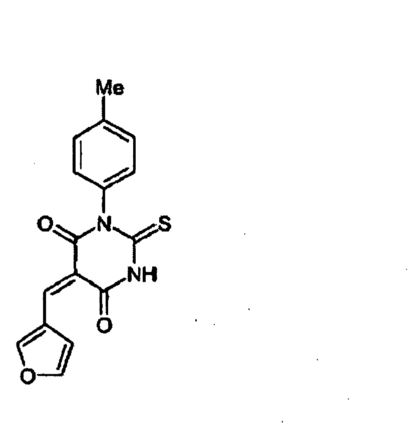 | **PX106297** |
| **(78)** | 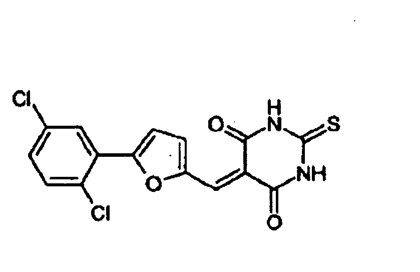 | **PX106326** |
| **(79)** | 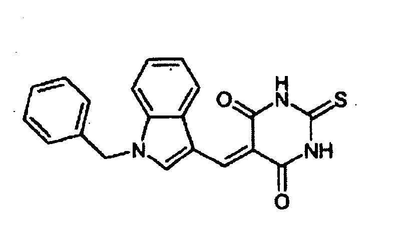 | **PX106341** |
| **(80)** | 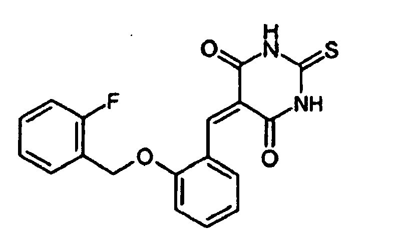 | **PX106343** |

25. Use of a compound selected from
| | | |
|---|---|---|
| (44) | 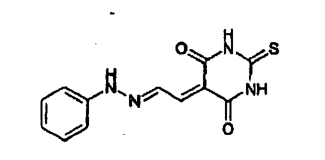 | PX089619 |
and
| | | |
|---|---|---|
| (45) | 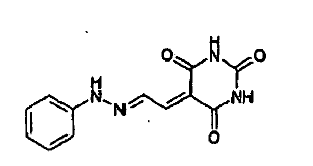 | PX089620 |
for the manufacture of a medicament for the inhibition of angiogenesis.

## Patentansprüche

1. Verwendung einer Verbindung der Formel 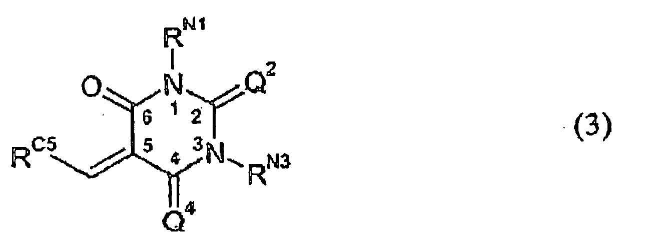 worin:
Q² =O, =S oder =NR^{N2} ist;
Q⁴ =O, =S oder =NR^{N4} ist;
R^{C5} ein Kohlenstoffsubstituent ist und unabhängig gegebenenfalls substituiertes aliphatisches oder alicyclisches C₁₋₇Alkyl, das gesättigt, teilweise gesättigt oder vollständig ungesättigt sein kann, gegebenenfalls substituiertes C₃₋₂₀Heterocyclyl oder gegebenenfalls substituiertes C₅₋₂₀Aryl ist und cis oder trans sein kann;
jedes von R^{N1}, R^{N2}, R^{N3} und R^{N4} ein Stickstoffsubstituent ist und unabhängig Wasserstoff, gegebenenfalls substituiertes C₁₋₇Alkyl, gegebenenfalls substituiertes C₃₋₂₀Heterocyclyl oder gegebenenfalls substituiertes C₅₋₂₀Aryl ist;
worin die Substituenten ausgewählt sind aus Wasserstoff, Halogen, Hydroxy, C₁₋₇Alkoxy, C₅₋₂₀Aryloxy, Oxo, Formyl, Acyl, Carboxy, Carboxylat, Acyloxy, Amido, Acylamido, Amino, Cyano, Nitro, Sulfhydryl, Thioether, Sulfonamino, Sulfinamino, Sulfamyl, Sulfonamido, C₁₋₇Alkyl, C₁₋₇Halogenalkyl, C₁₋₇Hydroxyalkyl, C₁₋₇Carboxyalkyl, C₁₋₇Aminoalkyl, C₅₋₂₀Aryl-C₁₋₇alkyl), gegebenenfalls substituiertem C₃₋₂₀Heterocyclyl, gegebenenfalls substituiertem C₅₋₂₀Aryl, C₅- oder eines pharmazeutisch verträglichen Salzes oder Solvates davon zur Herstellung eines Medikaments zum Inhibieren von Angiogenese.

2. Verwendung gemäß Anspruch 1, wobei in Verbindung (3) Q² =O oder =S ist und Q⁴ =O oder =S ist.

3. Verwendung gemäß Anspruch 1, wobei in Verbindung (3) Q² =O und Q⁴ =O ist oder Q² =S ist und Q⁴ =O ist.

4. Verwendung gemäß Anspruch 1, wobei in Verbindung (3) Q² =O und Q⁴ =O ist und die Verbindung die folgende Formel hat:

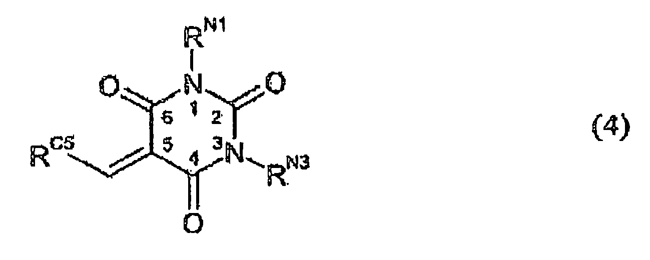

5. Verwendung gemäß Anspruch 1, wobei in Verbindung (3) Q² =S und Q⁴ =O ist und die Verbindung die folgende Formel aufweist:

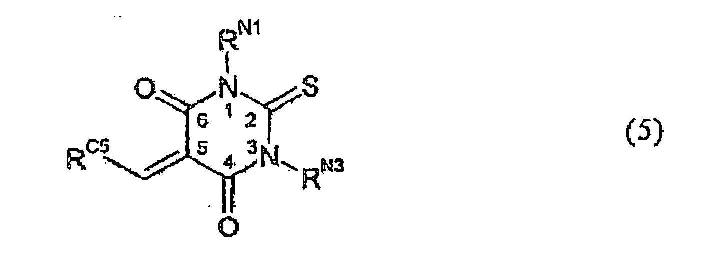

6. Verwendung gemäß einem der Ansprüche 1-5, wobei in den Verbindungen der Formeln (3)-(5) jedes von R^{N1}, R^{N2}, R^{N3} und R^{N4} unabhängig Wasserstoff, gesättigtes aliphatisches C₁₋₇Alkyl, gesättigtes aliphatisches C₁₋₇Halogenalkyl, gesättigtes aliphatisches C₁₋₇Hydroxyalkyl, gesättigtes aliphatisches C₁₋₇Aminoalkyl, gesättigtes aliphatisches C₁₋₇Carboxyalkyl, C₅₋₂₀Aryl-C₁₋₇alkyl, C₅₋₂₀Carboaryl oder C₅₋₂₀Halogenaryl ist.

7. Verwendung gemäß einem der Ansprüche 1-5, wobei in den Verbindungen der Formeln (3)-(5) jedes von R^{N1}, R^{N2}, R^{N3} und R^{N4} unabhängig -H, -Me, -Et, -CH₂CHOOH, -Ph, -C₆H₄F, -C₆H₄Cl, -C₆H₄Br, -C₆H₄-OCH₃ oder -C₆H₄-CH₃ ist.

8. Verwendung gemäß einem der Ansprüche 1-7, wobei in den Verbindungen der Formeln (3)-(5) R^{N1} -H oder etwas anderes als -H ist und jedes von R^{N2}, R^{N3} und R^{N4} -H ist.

9. Verwendung gemäß einem der Ansprüche 1-7, wobei in den Verbindungen der Formeln (3)-(5) R^{N3} -H oder etwas anderes als -H ist und jedes von R^{N1}, R^{N2} und R^{N4} -H ist.

10. Verwendung gemäß einem der Ansprüche 1-7, wobei in den Verbindungen der Formeln (3)-(5) jedes von R^{N1}, R^{N2}, R^{N3} und R^{N4} -H ist.

11. Verwendung gemäß einem der Ansprüche 1-10, wobei in den Verbindungen der Formeln (3) - (5) R^{C5} C₁₋₇Alkyl, gegebenenfalls substituiert, wie in Anspruch 1 angegeben, ist.

12. Verwendung gemäß einem der Ansprüche 1-10, wobei in den Verbindungen der Formeln (3)-(5) R^{C5} C₃₋₆Cycloalkyl, gegebenenfalls substituiert, wie in Anspruch 1 angegeben, ist.

13. Verwendung gemäß einem der Ansprüche 1-10, wobei in den Verbindungen der Formeln (3)-(5) R^{C5} teilweise ungesättigtes C₃₋₆Cycloalkyl, gegebenenfalls substituiert, wie in Anspruch 1 angegeben, ist.

14. Verwendung gemäß einem der Ansprüche 1-10, wobei in den Verbindungen der Formeln (3)-(5) R^{C5} C₅₋₂₀Aryl-C₁₋₇alkyl, gegebenenfalls substituiert, wie in Anspruch 1 angegeben, ist.

15. Verwendung gemäß einem der Ansprüche 1-10, wobei in den Verbindungen der Formeln (3)-(5) R^{C5} C₃₋₂₀Heterocylyl, gegebenenfalls substituiert, wie in Anspruch 1 angegeben, ist.

16. Verwendung gemäß einem der Ansprüche 1-10, wobei in den Verbindungen der Formeln (3)-(5) R^{C5} C₅₋₂₀Aryl, gegebenenfalls substituiert, wie in Anspruch 1 angegeben, ist.

17. Verwendung gemäß einem der Ansprüche 1-10, wobei in den Verbindungen der Formeln (3)-(5) R^{C5} C₅₋₂₀Carboaryl, gegebenenfalls substituiert, wie in Anspruch 1 angegeben, ist.

18. Verwendung gemäß einem der Ansprüche 1-10, wobei R^{C5} C₅₋₂₀Heteroaryl, gegebenenfalls substituiert, wie in Anspruch 1 angegeben, ist.

19. Verwendung gemäß einem der Ansprüche 1-10, wobei in den Verbindungen der Formeln (3)-(5) R^{C5} Cyclohexenyl, Phenyl, Furanyl, Thiophenyl, Pyrrolyl, Indolyl oder Benzopyronyl, gegebenenfalls substituiert, wie in Anspruch 1 angegeben, ist.

20. Verwendung gemäß einem der Ansprüche 1-10, wobei in den Verbindungen der Formeln (3)-(5) R^{C5} eine Phenylgruppe, gegebenenfalls substituiert, wie in Anspruch 1 angegeben, ist.

21. Verwendung gemäß einem der Ansprüche 1-10, wobei die Verbindung die folgende Formel aufweist: 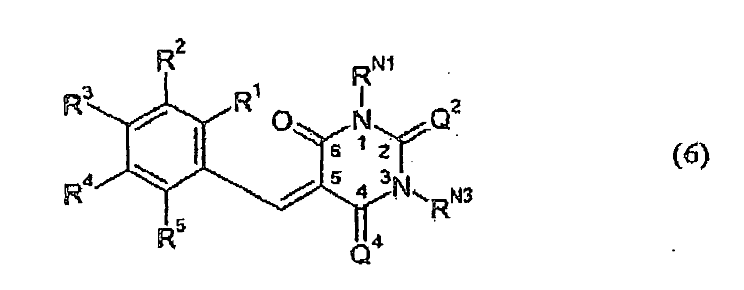 worin:
jedes von R¹ bis R⁵ ein Phenylsubstituent ist und unabhängig Wasserstoff, Halogen, Hydroxy, Ether, Formyl, Acyl, Carboxy, Carboxylat, Amido, Acylamido, Amino, Nitro oder C₁₋₇Alkyl, C₃₋₂₀Heterocyclyl, C₅₋₂₀Aryl, gegebenenfalls substituiert, wie in Anspruch 1 angegeben, ist; und
zwei von R¹ bis R⁵ zusammen eine zweizähnige Struktur bilden können, die zusammen mit den zwei Kohlenstoffatomen an die sie gebunden ist, eine cyclische Struktur mit fünf oder sechs Ringatomen bildet.

22. Verwendung gemäß Anspruch 21, wobei in Verbindung (6):
jedes von R¹ bis R⁵ unabhängig Wasserstoff, Halogen, Hydroxy, C₁₋₇Alkoxy, C₅₋₂₀Aryloxy, C₅₋₂₀Aryl-C₁₋₇alkoxy, Acyl, Amino oder C₁₋₇Alkyl ist, wobei die C₅₋₂₀Aryloxy-, C₅₋₂₀Aryl-C₁₋₇alkoxy- und C₁₋₇Alkylgruppen gegebenenfalls substituiert sind, wie in Anspruch 1 angegeben,
und
zwei von R¹ bis R⁵ eine zweizähnige Struktur bilden können, ausgewählt aus - (CH₂)₃-, - (CH₂)₄-, -O-CH₂-O- und -O-CH₂CH₂-O- .

23. Verwendung gemäß Anspruch 21, wobei in Verbindung (6) jedes von R¹ bis R⁵ unabhängig ausgewählt ist aus: -H, -F, -Cl, -Br, -I, -NMe₂, -NEt₂, -OH, -OCH₃, -OCH₂CH₃, -OPh, -OCH₂Ph, -C (=O) CH₃, -CONH₂, -CONHCH₃, -NO₂, -CH₃, -CH₂CH₃, -CF₃, -OCF₃, -CH₂OH, -Ph und -CH₂Ph.

24. Verwendung gemäß Anspruch 1, wobei die Verbindung ausgewählt ist aus:
| | | |
|---|---|---|
| **(1)** | 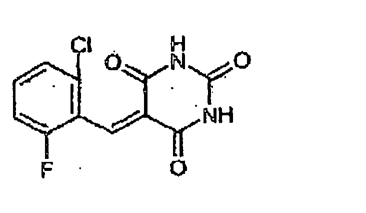 | **PX069119** |
| **(2)** | 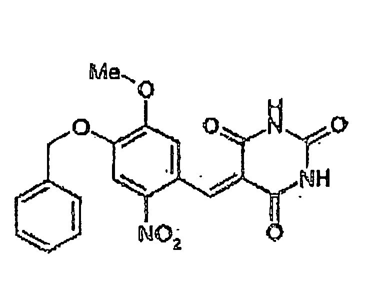 | **PX069153** |
| **(3)** | 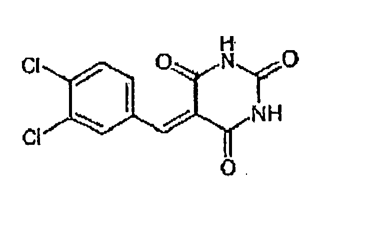 | **PX072002** |
| **(4)** | 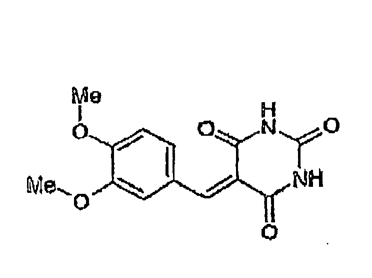 | **PX072004** |
| **(5)** | 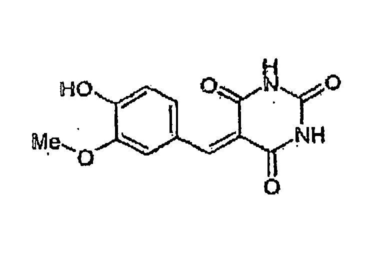 | **PX072008** |
| **(6)** | 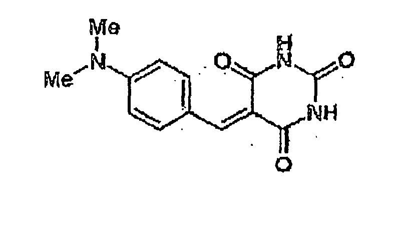 | **PX072009** |
| **(7)** | 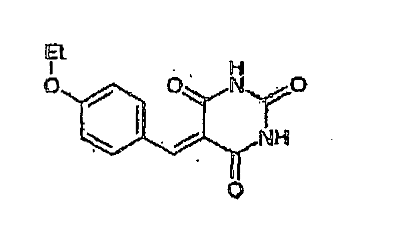 | **PX072012** |
| **(8)** | 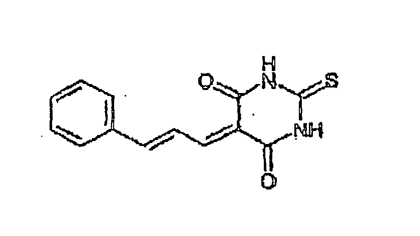 | **PX072015** |
| **(9)** | 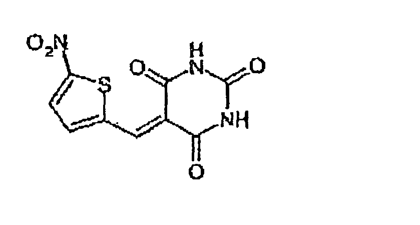 | **PX074037** |
| **(10)** | 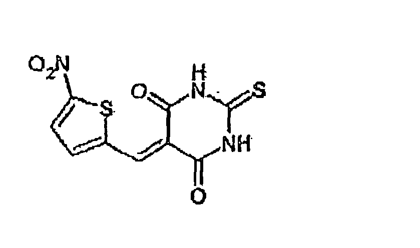 | **PX074038** |
| **(11)** | 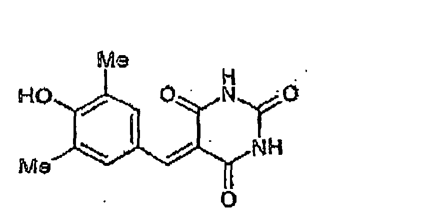 | **PX074100** |
| **(12)** | 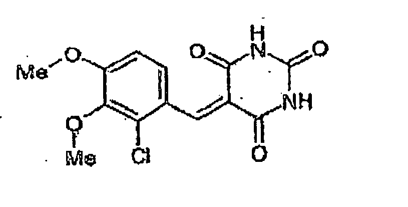 | **PX074728** |
| **(13)** | 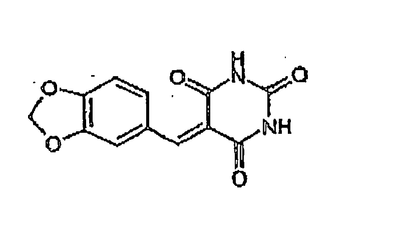 | **PX075240** |
| **(14)** | 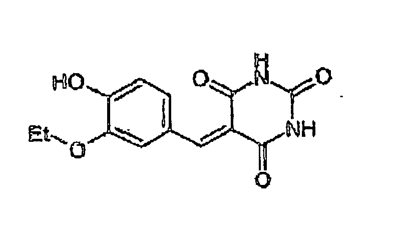 | **PX075244** |
| **(15)** | 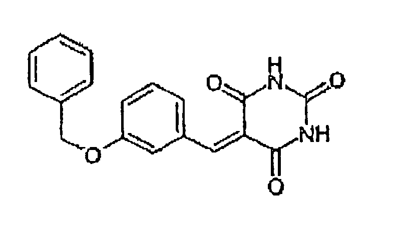 | **PX075245** |
| **(16)** | 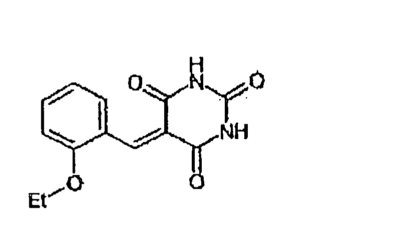 | **PX075248** |
| **(17)** | 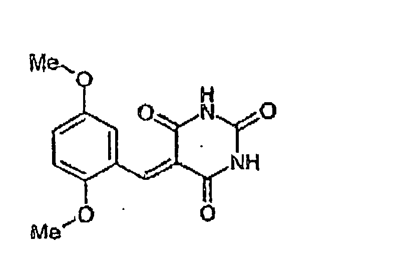 | **PX075257** |
| **(18)** | 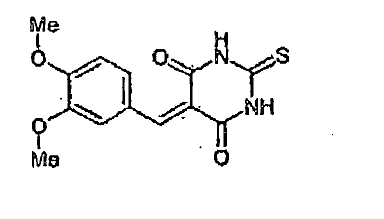 | **PX075262** |
| **(19)** | 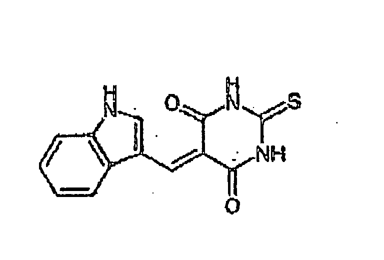 | **PX075276** |
| **(20)** | 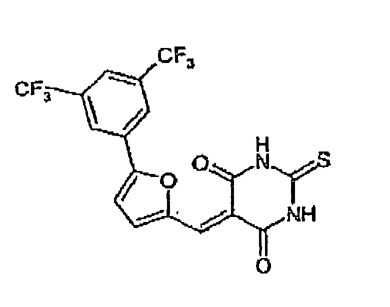 | **PX075367** |
| **(28)** | 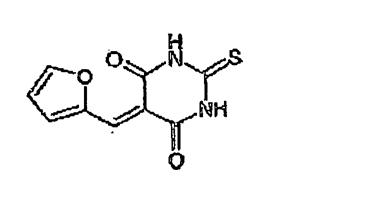 | **PX083634** |
| **(29)** | 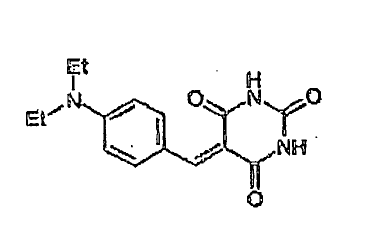 | **PX083675** |
| **(30)** | 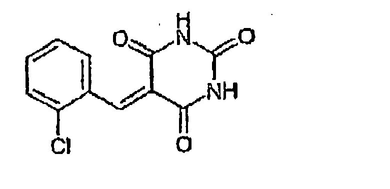 | **PX083677** |
| **(33)** | 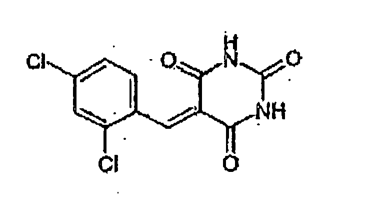 | **PX089367** |
| **(34)** | 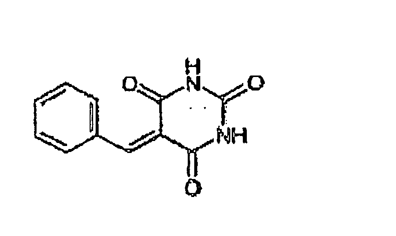 | **PX089368** |
| **(35)** | 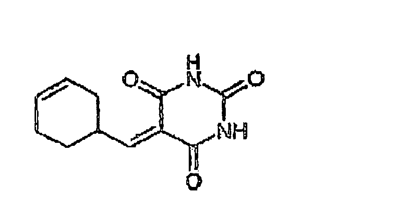 | **PX089369** |
| **(36)** | 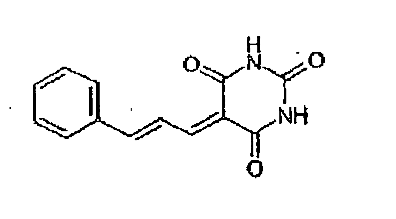 | **PX089370** |
| **(37)** | 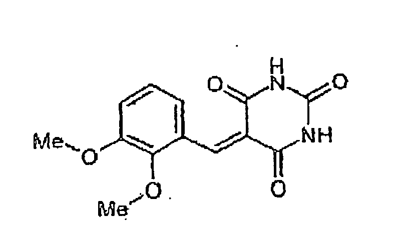 | **PX089371** |
| **(38)** | 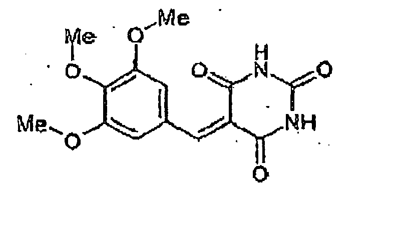 | **PX089372** |
| **(39)** | 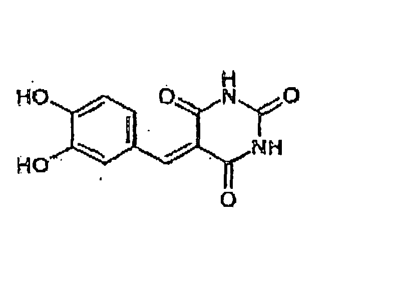 | **PX089374** |
| **(40)** | 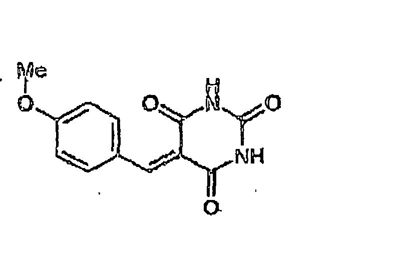 | **PX089375** |
| **(41)** | 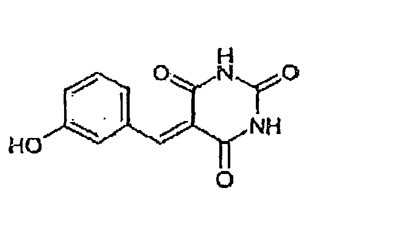 | **PX089376** |
| **(42)** | 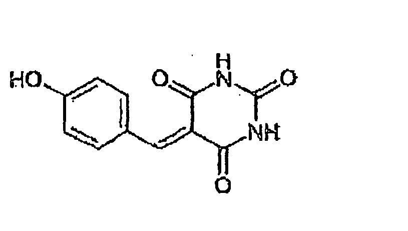 | **PX089377** |
| **(43)** | 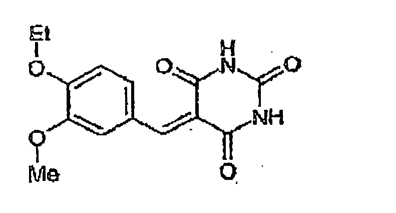 | **PX089378** |
| **(46)** | 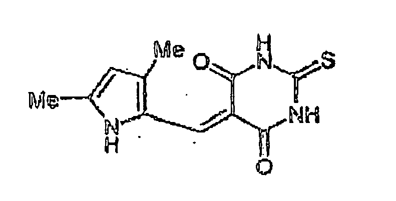 | **PX089624** |
| **(47)** | 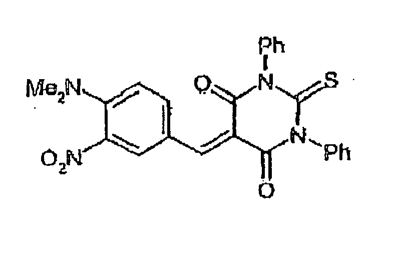 | **PX089626** |
| **(48)** | 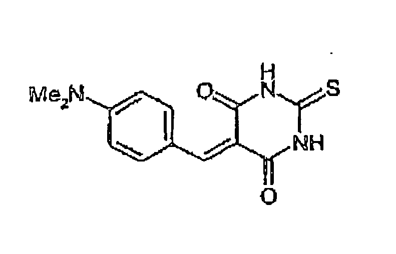 | **PX089631** |
| **(49)** | 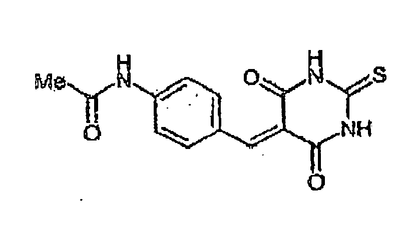 | **PX089632** |
| **(50)** | 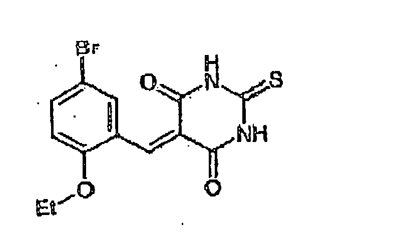 | **PX089633** |
| **(51)** | 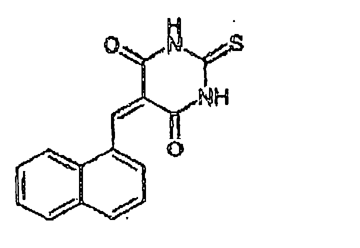 | **PX089635** |
| **(52)** | 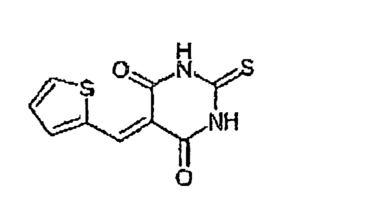 | **PX089638** |
| **(53)** | 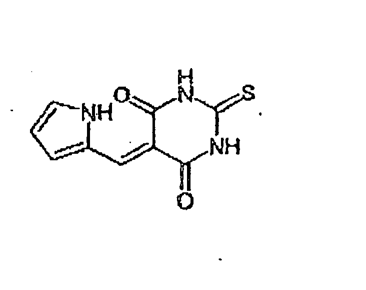 | **PX089639** |
| **(54)** | 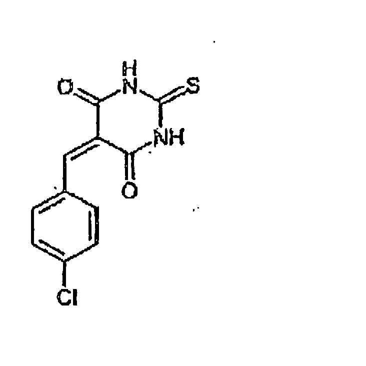 | **PX089640** |
| **(55)** | 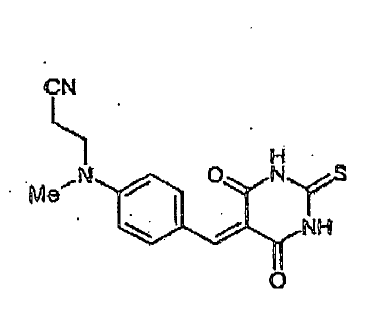 | **PX089643** |
| **(56)** | 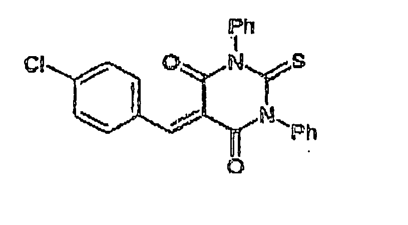 | **PX089645** |
| **(57)** | 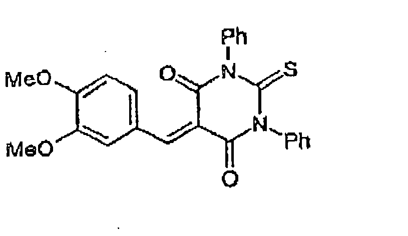 | **PX089646** |
| **(58)** | 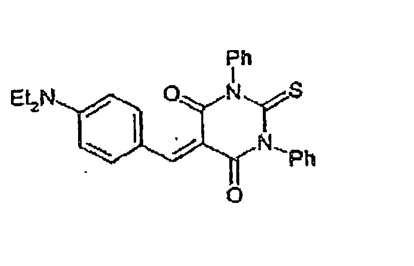 | **PX089647** |
| **(59)** | 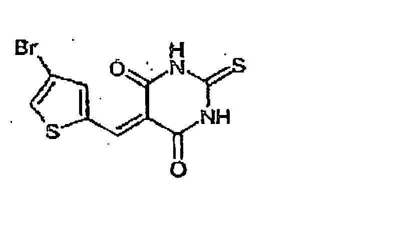 | **PX089648** |
| **(60)** | 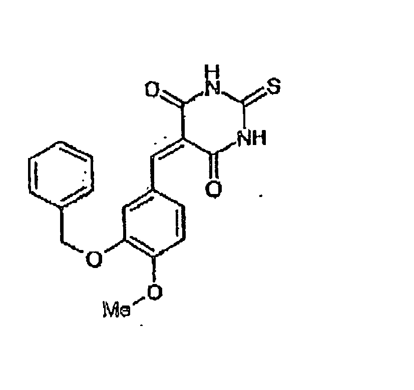 | **PX105990** |
| **(61)** | 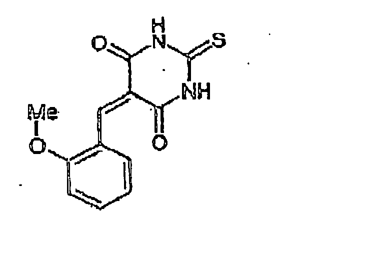 | **PX105993** |
| **(62)** | 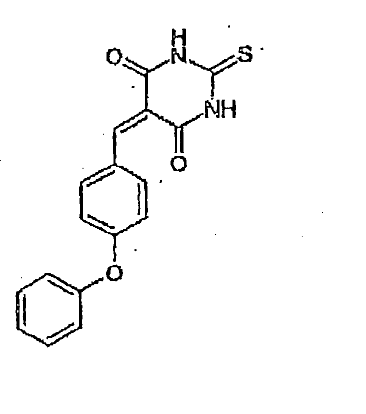 | **PX106021** |
| **(63)** | 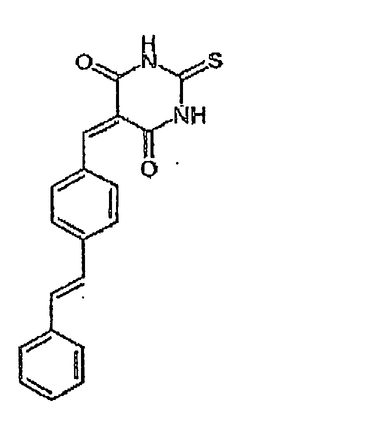 | **PX106027** |
| **(64)** | 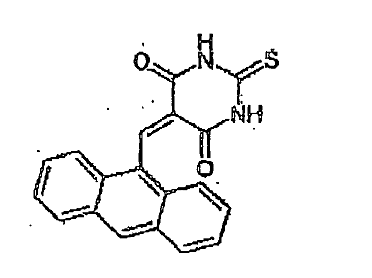 | **PX106031** |
| **(65)** | 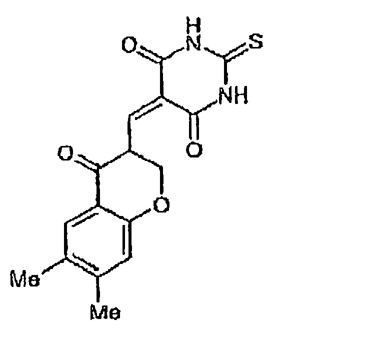 | **PX106036** |
| **(66)** | 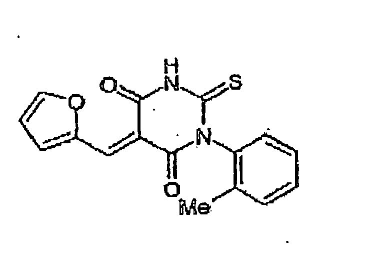 | **PX106130** |
| **(67)** | 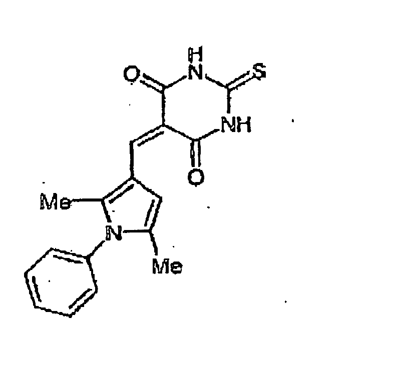 | **PX106151** |
| **(68)** | 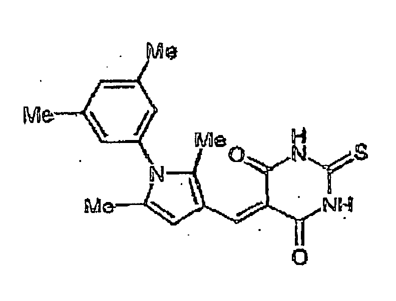 | **PX106155** |
| **(69)** | 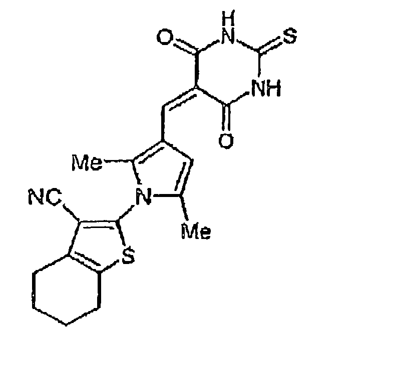 | **PX106174** |
| **(70)** | 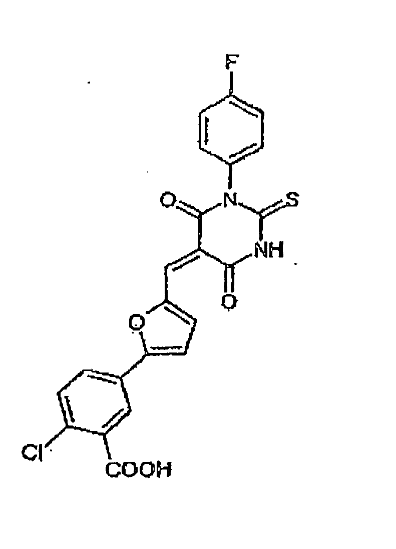 | **PX106244** |
| **(71)** | 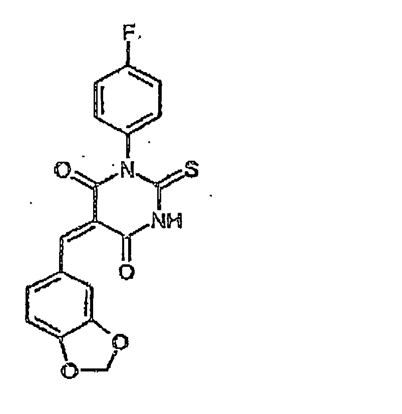 | **PX106255** |
| **(72)** | 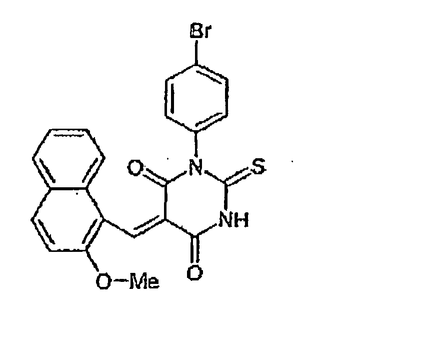 | **PX106265** |
| **(73)** | 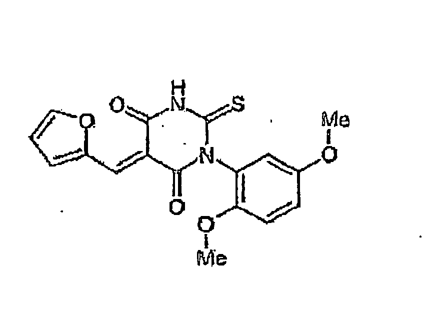 | **PX106274** |
| **(74)** | 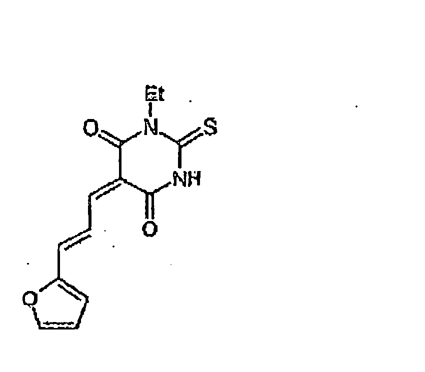 | **PX106281** |
| **(75)** | 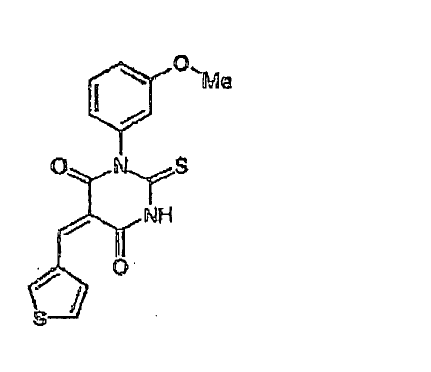 | **PX106287** |
| **(76)** | 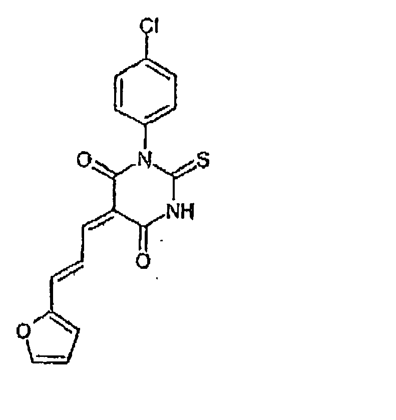 | **PX106291** |
| **(77)** | 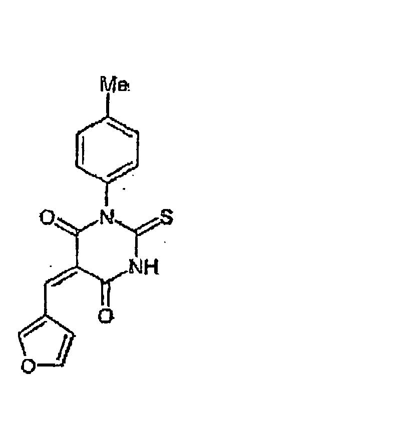 | **PX106297** |
| **(78)** | 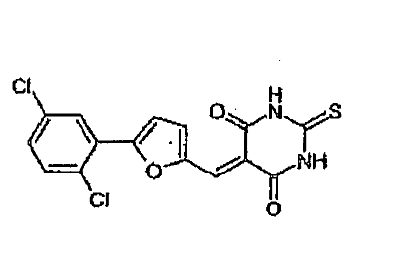 | **PX106326** |
| **(79)** | 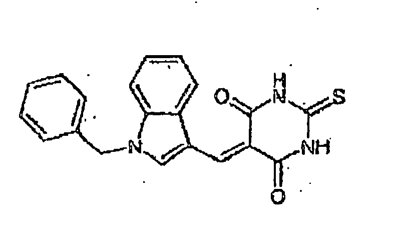 | **PX106341** |
| **(80)** | 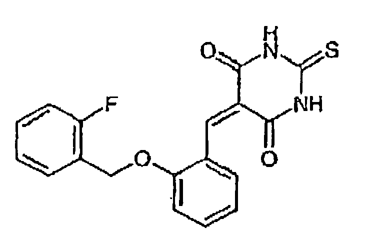 | **PX106343** |

25. Verwendung einer Verbindung ausgewählt aus
| | | |
|---|---|---|
| **(44)** | 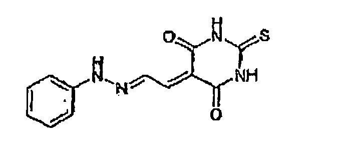 | **PX089619** |
und
| | | |
|---|---|---|
| **(45)** | 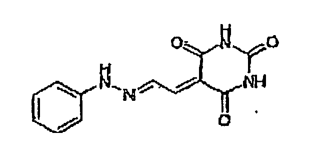 | **PX089620** |
zur Herstellung eines Medikaments zur Inhibierung von Angiogenese.

## Revendications

1. Utilisation d'un composé de formule 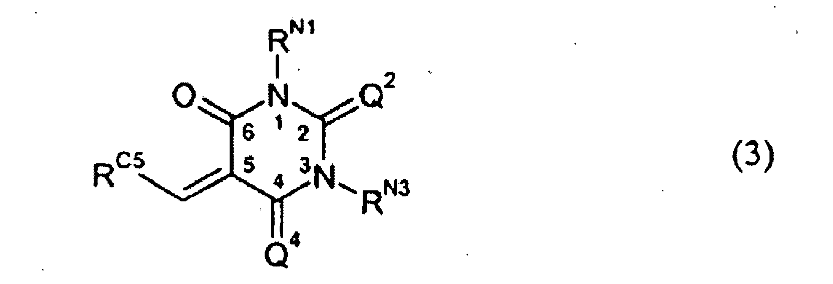 dans laquelle :
Q² est =O, =S, ou =NR^{N2} ;
Q⁴ est =O, =S, ou =NR^{N4} ;
R^{C5} est un substituant du carbone, et est indépendamment un alkyle en C₁-C₇ aliphatique ou alicyclique facultativement substitué, qui peut être saturé, partiellement saturé ou totalement insaturé ; un hétérocyclyle en C₃-C₂₀ facultativement substitué, ou un aryle en C₅-C₂₀ facultativement substitué et peut être cis- ou trans- ;
chacun de R^{N1}, R^{N2}, R^{N3}, et R^{N4} est un substituant de l'azote, et est indépendamment un hydrogène, un alkyle en C₁-C₇ facultativement substitué, un hétérocyclyle en C₃-C₂₀ facultativement substitué, ou un aryle en C₅-C₂₀ facultativement substitué ;
dans lesquels les substituants sont choisis parmi un hydrogène, un halo, un hydroxy, un alkoxy en C₁-C₇, un aryloxy en C₅-C₂₀, un OXO, un formyle, un acyle, un carboxy, un carboxylate, un acyloxy, un amido, un acylamido, un amino, un cyano, un nitro, un sulfhydryle, un thioéther, un sulfonamino, un sulfinamino, un sulfamyle, un sulfonamido, un alkyle en C₁-C₇, un haloalkyle en C₁-C₇, un hydroxyalkyle en C₁-C₇, un carboxyalkyle en C₁-C₇, un aminoalkyle en C₁-C₇, un aryle en C₅-C₂₀-alkyle en C₁-C₇, un hétérocyclyle en C₃-C₂₀ facultativement substitué, un aryle en C₅-C₂₀ facultativement substitué,
ou un sel ou un solvate pharmaceutiquement acceptable de celui-ci
pour la fabrication d'un médicament pour inhiber l'angiogenèse.

2. Utilisation selon la revendication 1, dans laquelle dans le composé (3) Q² est =O ou =S ; et Q⁴ est =O ou =S.

3. Utilisation selon la revendication 1, dans laquelle dans le composé (3) Q² est =O et Q⁴ est =O ; ou Q² est =S et Q⁴ est =O.

4. Utilisation selon la revendication 1, dans laquelle dans le composé (3) Q² est =O et Q⁴ est =O et le composé a la formule suivante :

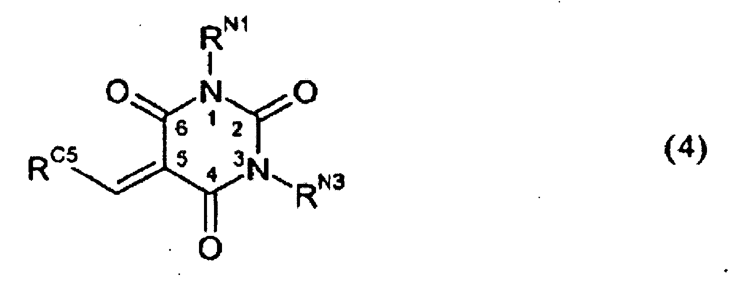

5. Utilisation selon la revendication 1, dans laquelle dans le composé (3) Q² est =S et Q⁴ est =O et le composé a la formule suivante :

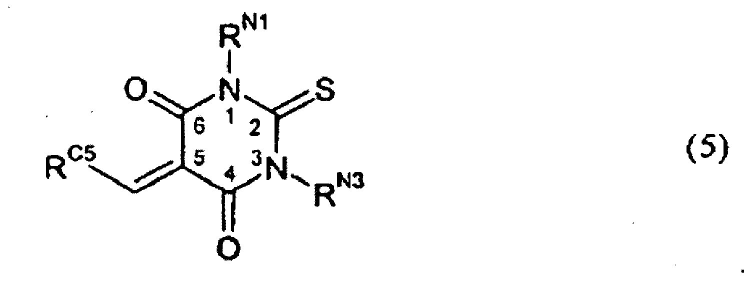

6. Utilisation selon l'une quelconque des revendications 1 à 5, dans laquelle dans les composés de formule (3) à (5), chacun de R^{N1}, R^{N2}, R^{N3}, et R^{N4} est indépendamment un hydrogène, un alkyle en C₁-C₇ aliphatique saturé, un haloalkyle en C₁-C₇ aliphatique saturé, un hydroxyalkyle en C₁-C₇ aliphatique saturé, un aminoalkyle en C₁-C₇ aliphatique saturé, un carboxyalkyle en C₁-C₇ aliphatique saturé, un aryle en C₅-C₂₀-alkyle en C₁-C₇, un carboaryle en C₅-C₂₀, ou un haloaryle en C₅-C₂₀.

7. Utilisation selon l'une quelconque des revendications 1 à 5, dans laquelle dans les composés de formule (3) à (5), chacun de R^{N1}, R^{N2}, R^{N3}, et R^{N4} est indépendamment -H, -Me, -Et, -CH₂COOH, -Ph, -C₆H₄F, -C₆H₄Cl, -C₆H₄Br, -C₆H₄-OCH₃, ou -C₆H₄-CH₃.

8. Utilisation selon l'une quelconque des revendications 1 à 7, dans laquelle dans les composés de formule (3) à (5), R^{N1} est -H ou autre que -H ; et chacun de R^{N2}, R^{N3}, et R^{N4} est -H.

9. Utilisation selon l'une quelconque des revendications 1 à 7, dans laquelle dans les composés de formule (3) à (5), R^{N3} est -H ou autre que -H ; et chacun de R^{N1}, R^{N2}, et R^{N4} est -H.

10. Utilisation selon l'une quelconque des revendications 1 à 7, dans laquelle dans les composés de formule (3) à (5), chacun de RN¹, R^{N2}, R^{N3}, et R^{N4} est -H_{.}

11. Utilisation selon l'une quelconque des revendications 1 à 10, dans laquelle dans les composés de formule (3) à (5), R^{C5} est un alkyle en C₁-C₇, facultativement substitué ainsi qu'il est indiqué dans la revendication 1.

12. Utilisation selon l'une quelconque des revendications 1 à 10, dans laquelle dans les composés de formule (3) à (5), R^{C5} est un cycloalkyle en C₃-C₆, facultativement substitué ainsi qu'il est indiqué dans la revendication 1.

13. Utilisation selon l'une quelconque des revendications 1 à 10, dans laquelle dans les composés de formule (3) à (5), R^{C5} est un cycloalkyle en C₃-C₆ partiellement insaturé, facultativement substitué ainsi qu'il est indiqué dans la revendication 1.

14. Utilisation selon l'une quelconque des revendications 1 à 10, dans laquelle dans les composés de formule (3) à (5), R^{C5} est un aryle en C₅-C₂₀-alkyle en C₁-C₇, facultativement substitué ainsi qu'il est indiqué dans la revendication 1.

15. Utilisation selon l'une quelconque des revendications 1 à 10, dans laquelle dans les composés de formule (3) à (5), R^{C5} est un hétérocyclyle en C₃-C₂₀, facultativement substitué ainsi qu'il est indiqué dans la revendication 1.

16. Utilisation selon l'une quelconque des revendications 1 à 10, dans laquelle dans les composés de formule (3) à (5) , R^{C5} est un aryle en C₅-C₂₀, facultativement substitué ainsi qu'il est indiqué dans la revendication 1.

17. Utilisation selon l'une quelconque des revendications 1 à 10, dans laquelle dans les composés de formule (3) à (5), R^{C5} est un carboaryle en C₅-C₂₀, facultativement substitué ainsi qu'il est indiqué dans la revendication 1.

18. Utilisation selon l'une quelconque des revendications 1 à 10, dans laquelle R^{C5} est un hétéroaryle en C₅-C₂₀, facultativement substitué ainsi qu'il est indiqué dans la revendication 1.

19. Utilisation selon l'une quelconque des revendications 1 à 10, dans laquelle dans les composés de formule (3) à (5), R^{C5} est un cyclohexényle, un phényle, un furanyle, un thiophényle, un pyrrolyle, un indolyle, ou un benzopyronyle, facultativement substitué ainsi qu'il est indiqué dans la revendication 1.

20. Utilisation selon l'une quelconque des revendications 1 à 10, dans laquelle dans les composés de formule (3) à (5), R^{C5} est un groupe phényle, facultativement substitué ainsi qu'il est indiqué dans la revendication 1.

21. Utilisation selon l'une quelconque des revendications 1 à 10, dans laquelle le composé a la formule suivante : 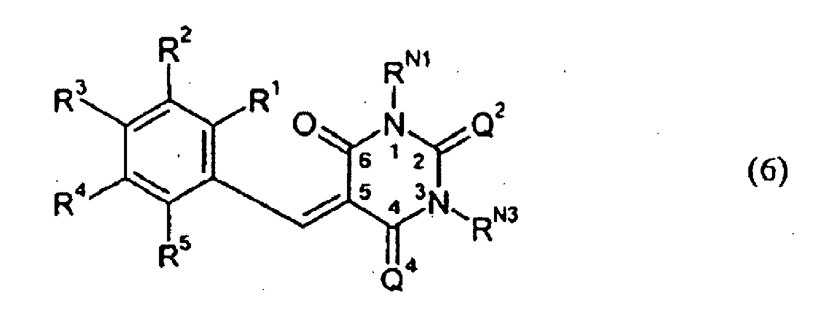 dans laquelle :
chacun de R¹ à R⁵ est un substituant du phényle, et est indépendamment un hydrogène, un halo, un hydroxy, un éther, un formyle, un acyle, un carboxy, un carboxylate, un amido, un acylamido, un amino, un nitro, ou un alkyle en C₁-C₇, un hétérocyclyle en C₃-C₂₀, un aryle en C₅-C₂₀ facultativement substitué ainsi qu'il est indiqué dans la revendication 1 ; et
deux de R¹ à R⁵ peuvent ensemble former une structure bidentate qui, avec les deux atomes de carbone auxquels elle est attachée, forme une structure cyclique avec cinq ou six atomes de cycle.

22. Utilisation selon la revendication 21, dans laquelle dans le composé (6) :
chacun de R¹ à R⁵ est indépendamment un hydrogène, un halo, un hydroxy, un alkoxy en C₁-C₇, un aryloxy en C₅-C₂₀, un aryle en C₅-C₂₀-alkoxy en C₁-C₇, un acyle, un amino ou un alkyle en C₁-C₇, les groupes aryloxy en C₅-C₂₀, aryle en C₅-C₂₀-alkoxy en C₁-C₇, et alkyle en C₁-C₇ étant facultativement substitués ainsi qu'il est indiqué dans la revendication 1
et
deux de R¹ à R⁵ peuvent former une structure bidentate choisie parmi -(CH₂)₃-, -(CH₂)₄-, -O-CH₂-O-, et -O-CH₂-CH₂-O- .

23. Utilisation selon la revendication 21, dans laquelle dans le composé (6), chacun de R¹ à R⁵ est indépendamment choisi parmi : -H, -F, -Cl, -Br, -I, -NMe₂, -Net₂, -OH, -OCH₃, -OCH₂CH₃, -OPh, -OCH₂Ph, -C (=O) CH₃, -CONH₂, -CONHCH₃, -NO₂ -CH₃, -CH₂CH₃, -CF₃, -OCF₃, -CH₂OH, -Ph, et -CH₂Ph.

24. Utilisation selon la revendication 1, dans laquelle le composé est choisi parmi :
| | | |
|---|---|---|
| **(1)** | 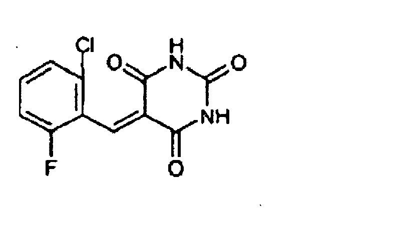 | **PX069119** |
| **(2)** | 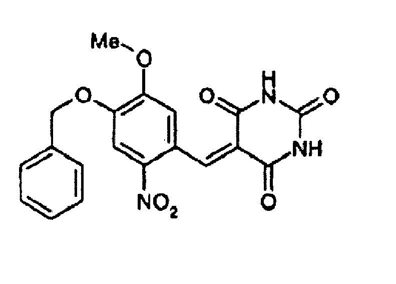 | **PX069153** |
| **(3)** | 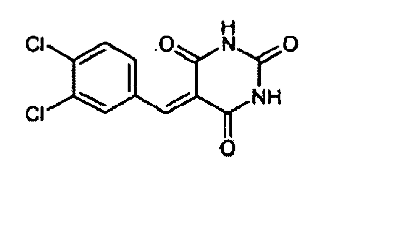 | **PX072002** |
| **(4)** | 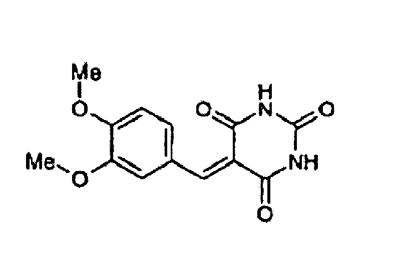 | **PX072004** |
| **(5)** | 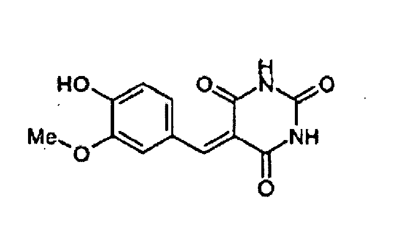 | **PX072008** |
| **(6)** | 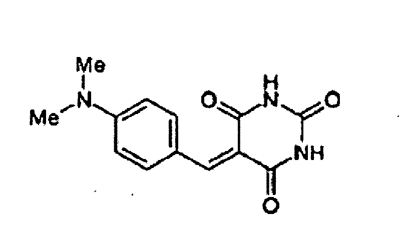 | **PX072009** |
| **(7)** | 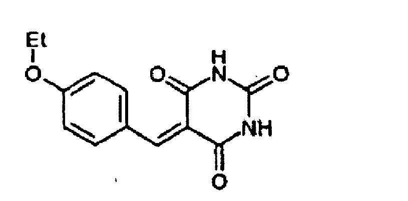 | **PX072012** |
| **(8)** | 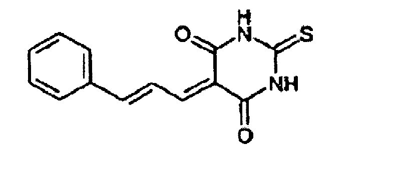 | **PX072015** |
| **(9)** | 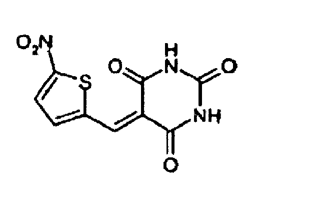 | **PX074037** |
| **(10)** | 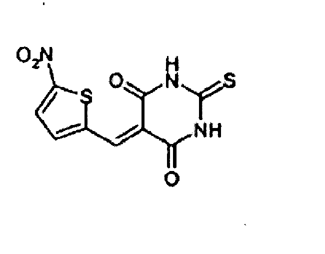 | **PX074038** |
| **(11)** | 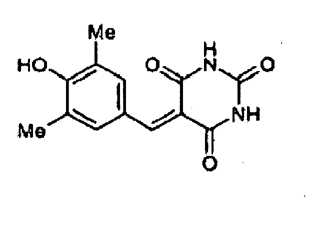 | **PX074100** |
| **(12)** | 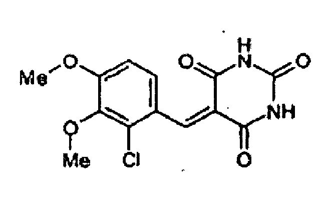 | **PX074728** |
| **(13)** | 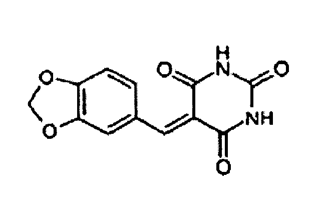 | **PX075240** |
| **(14)** | 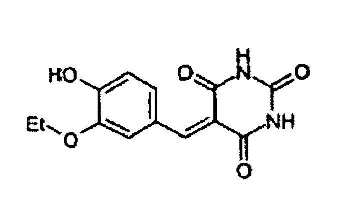 | **PX075244** |
| **(15)** | 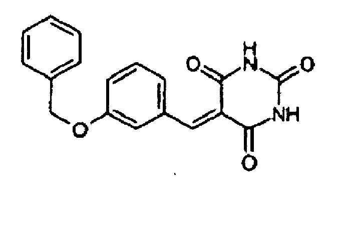 | **PX075245** |
| **(16)** | 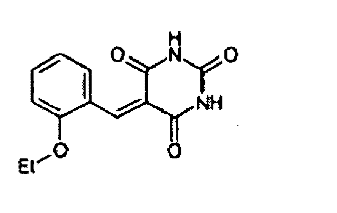 | **PX075248** |
| **(17)** | 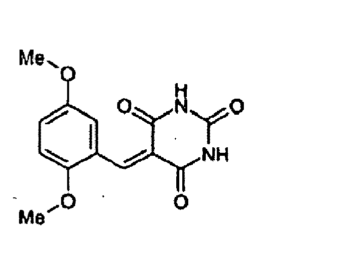 | **PX075257** |
| **(18)** | 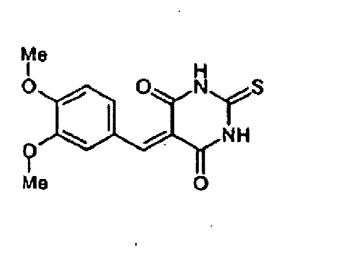 | **PX075262** |
| **(19)** | 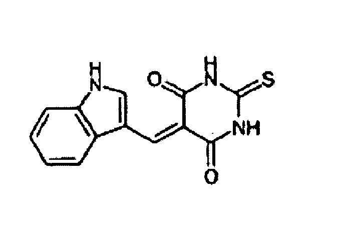 | **PX075276** |
| **(20)** | 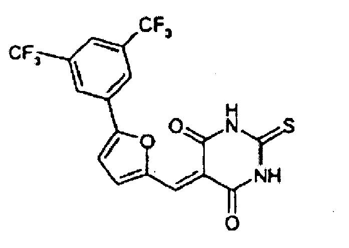 | **PX075367** |
| **(28)** | 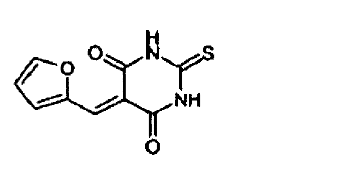 | **PX083634** |
| **(29)** | 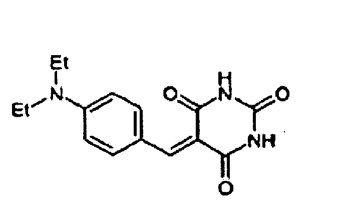 | **PX083675** |
| **(30)** | 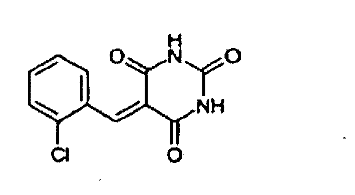 | **PX083677** |
| **(33)** | 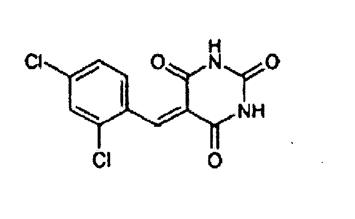 | **PX089367** |
| **(34)** | 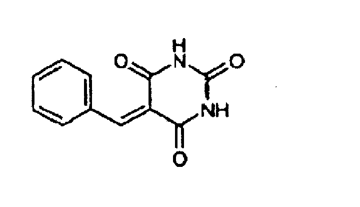 | **PX089368** |
| **(35)** | 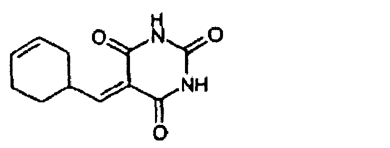 | **PX089369** |
| **(36)** | 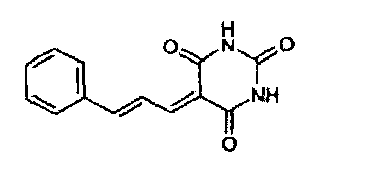 | **PX089370** |
| **(37)** | 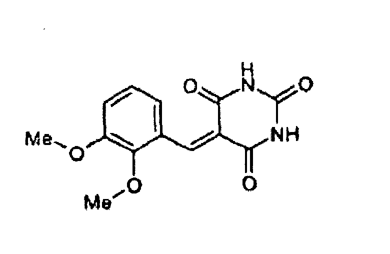 | **PX089371** |
| **(38)** | 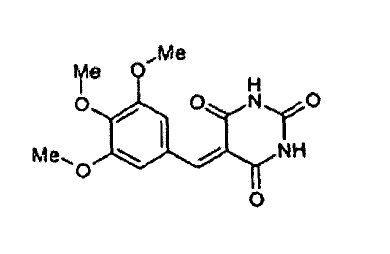 | **PX089372** |
| **(39)** | 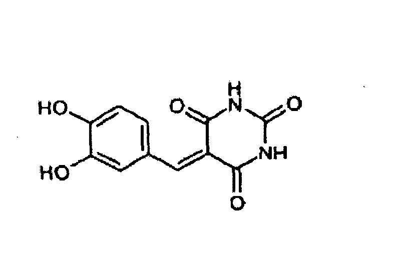 | **PX089374** |
| **(40)** | 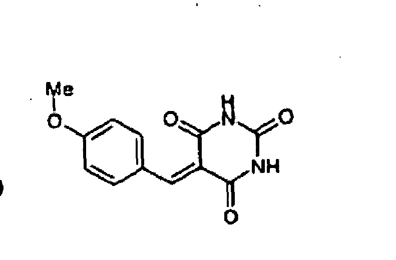 | **PX089375** |
| **(41)** | 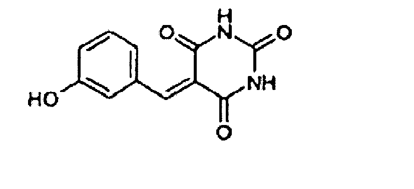 | **PX089376** |
| **(42)** | 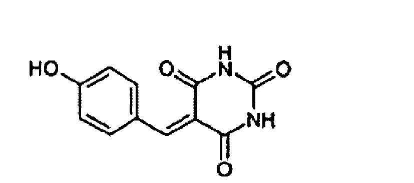 | **PX089377** |
| **(43)** | 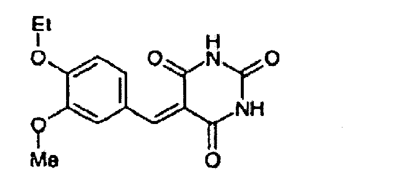 | **PX089378** |
| **(46)** | 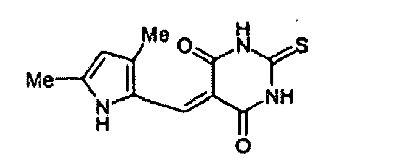 | **PX089624** |
| **(47)** | 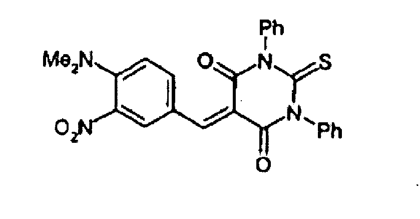 | **PX089626** |
| **(48)** | 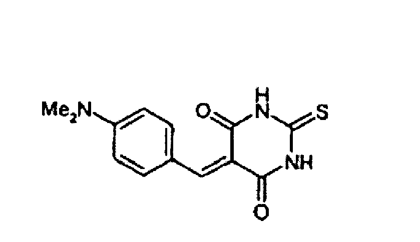 | **PX089631** |
| **(49)** | 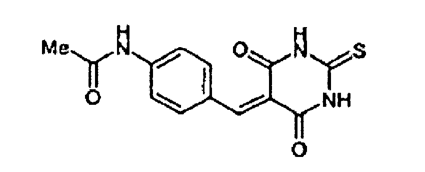 | **PX089632** |
| **(50)** | 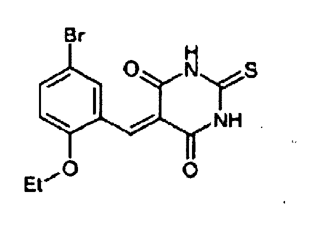 | **PX089633** |
| **(51)** | 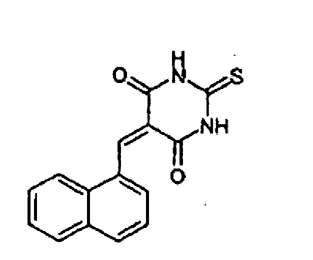 | **PX089635** |
| **(52)** | 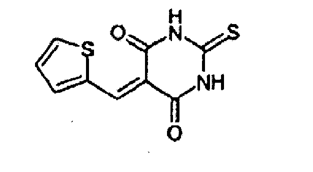 | **PX089638** |
| **(53)** | 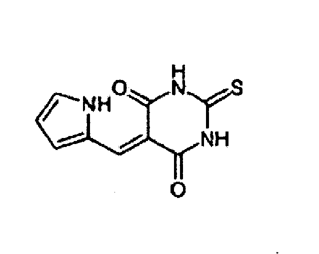 | **PX089639** |
| **(54)** | 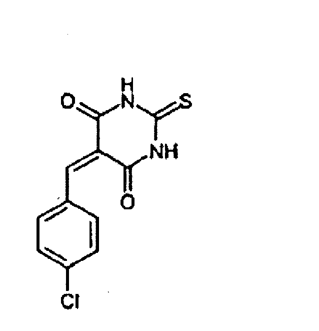 | **PX089640** |
| **(55)** | 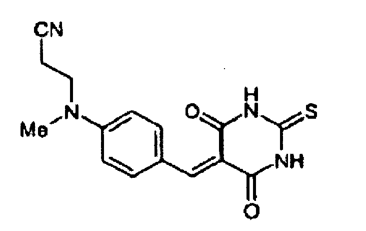 | **PX089643** |
| **(56)** | 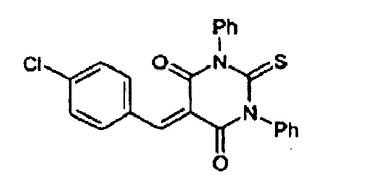 | **PX089645** |
| **(57)** | 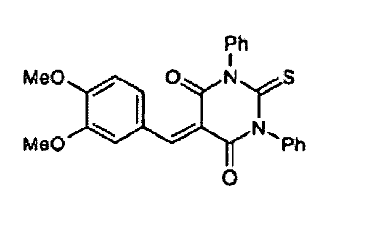 | **PX089646** |
| **(58)** | 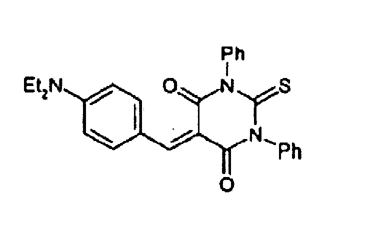 | **PX089647** |
| **(59)** | 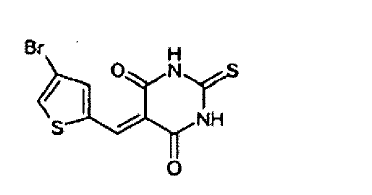 | **PX089648** |
| **(60)** | 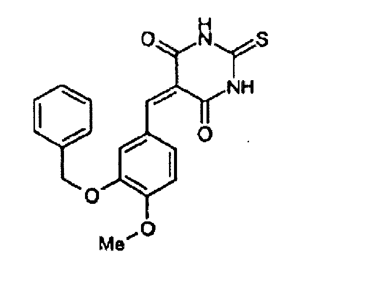 | **PX105990** |
| **(61)** | 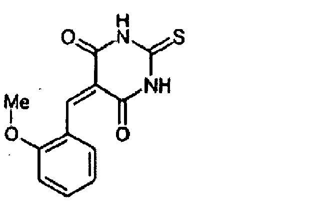 | **PX105993** |
| **(62)** | 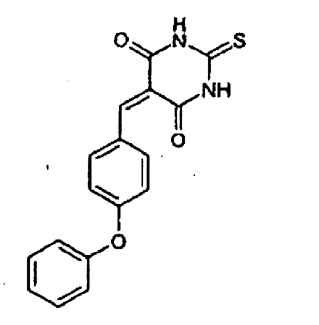 | **PX106021** |
| **(63)** | 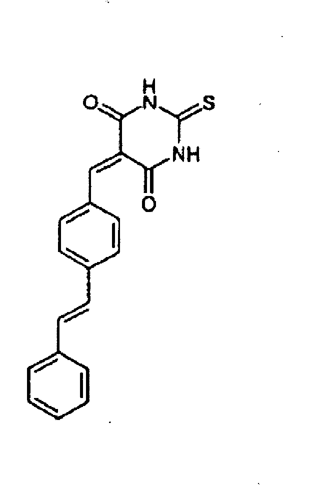 | **PX106027** |
| **(64)** | 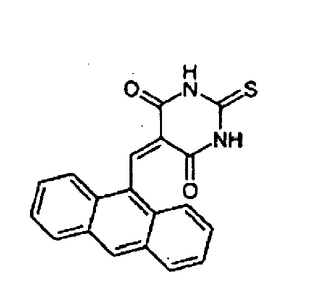 | **PX106031** |
| **(65)** | 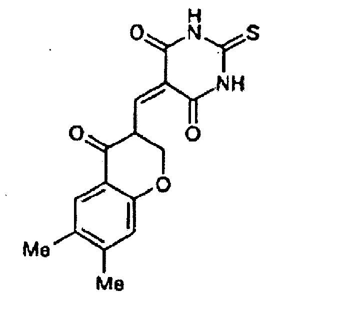 | **PX106036** |
| **(66)** | 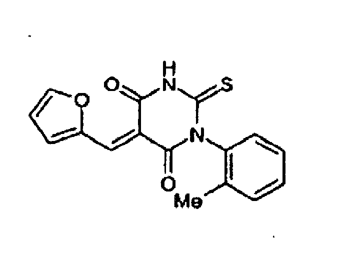 | **PX106130** |
| **(67)** | 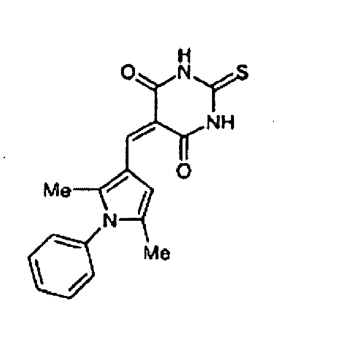 | **PX106151** |
| **(68)** | 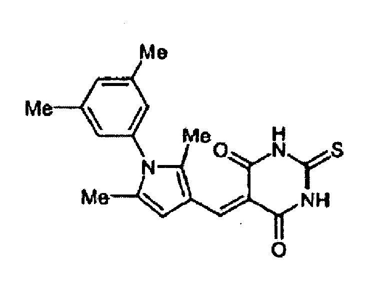 | **PX106155** |
| **(69)** | 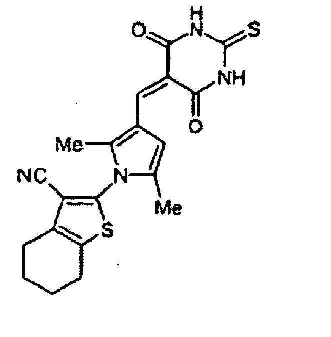 | **PX106174** |
| **(70)** | 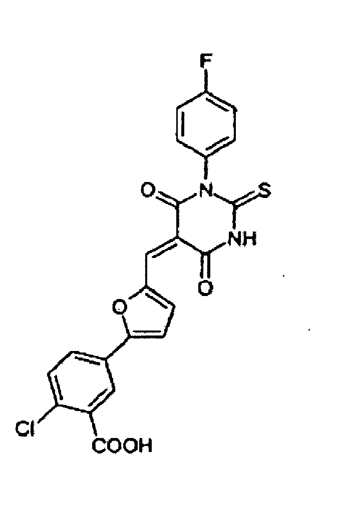 | **PX106244** |
| **(71)** | 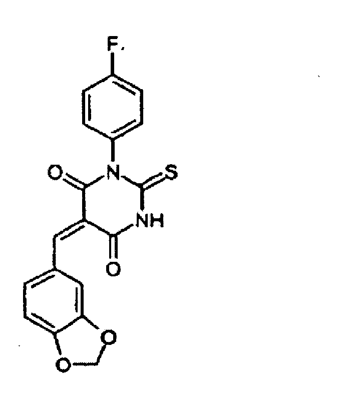 | **PX106255** |
| **(72)** | 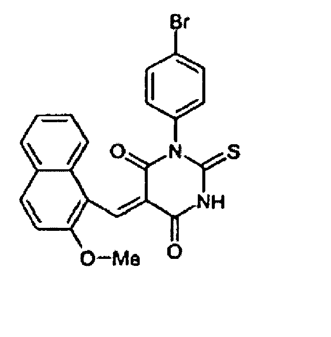 | **PX106265** |
| **(73)** | 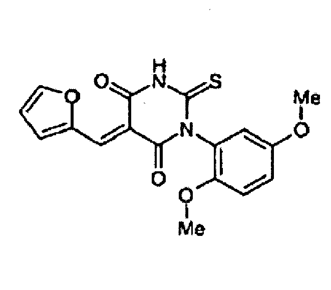 | **PX106274** |
| **(74)** | 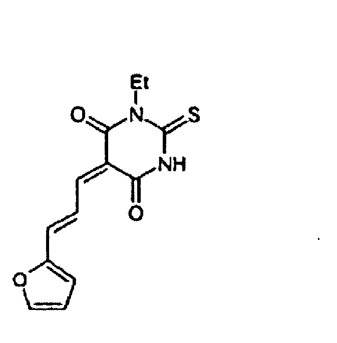 | **PX106281** |
| **(75)** | 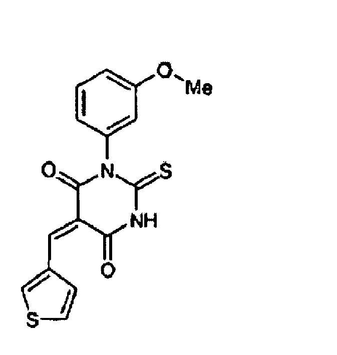 | **PX106287** |
| **(76)** | 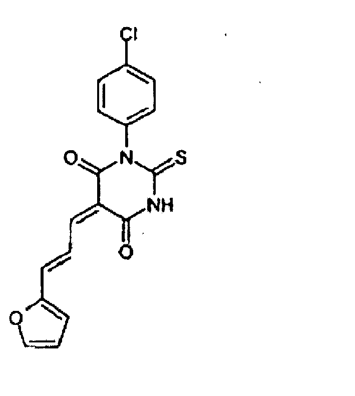 | **PX106291** |
| **(77)** | 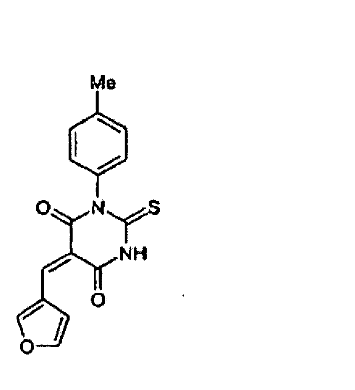 | **PX106297** |
| **(78)** | 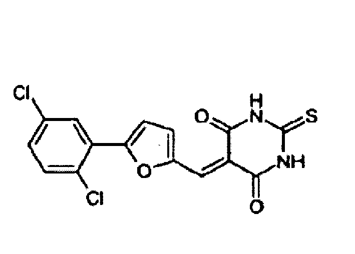 | **PX106326** |
| **(79)** | 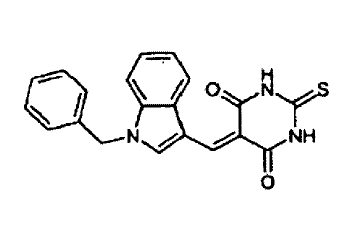 | **PX106341** |
| **(80)** | 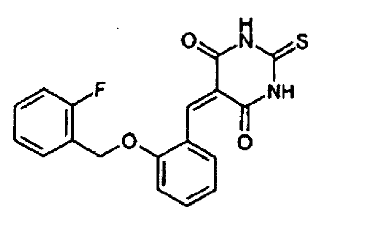 | **PX106343** |

25. Utilisation d'un composé choisi parmi :
| | | |
|---|---|---|
| **(44)** | 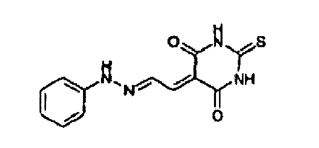 | **PX089619** |
et
| | | |
|---|---|---|
| **(45)** | 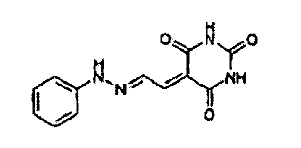 | **PX089620** |
pour la fabrication d'un médicament pour l'inhibition de l'angiogenèse.
